(19) Europäisches Patentamt
European Patent Office
Office européen des brevets

(11) Numéro de publication: **0 236 646 B1**

(12) # FASCICULE DE BREVET EUROPEEN

(45) Date de publication du fascicule du brevet: **10.10.90**

(21) Numéro de dépôt: **86402796.6**

(22) Date de dépôt: **15.12.86**

(51) Int. Cl.⁵: **C07D 233/50**, C07D 403/12, C07D 263/28, C07D 413/12, C07D 277/18, C07D 417/12, A61K 31/415, A61K 31/425, A61K 31/40, A61K 31/42, C07C 237/34

(54) **Nouveaux ortho-anisamides, leur procédé d'obtention et leurs applications thérapeutiques.**

(30) Priorité: **19.12.85 FR 8518829**

(43) Date de publication de la demande: **16.09.87 Bulletin 87/38**

(45) Mention de la délivrance du brevet: **10.10.90 Bulletin 90/41**

(84) Etats contractants désignés: **AT BE CH DE ES FR GB GR IT LI LU NL SE**

(56) Documents cités:
**EP-A- 0 001 947**
**EP-A- 0 081 923**
**EP-A- 0 081 924**
**DE-A- 2 905 883**

**CHEMICAL ABSTRACTS, vol. 84, no. 7, 16 février 1976, page 454, no. 43661t, Columbus, Ohio, US; & JP-A-75 14 651**

(73) Titulaire: **Laboratoires DELAGRANGE, Société Anonyme, 1, Avenue Pierre-Brossolette, F-91380 Chilly-Mazarin(FR)**

(72) Inventeur: **Acher, Jacques, 32, route de Saint Vrain, F-91760 Itteville(FR)**
Inventeur: **Monier, Jean-Claude, 6, rue de la Sorbonne, F-91510 Lardy(FR)**
Inventeur: **Schmitt, Jean-Paul, 13, rue Victor Hugo, F-91290 Arpajon(FR)**
Inventeur: **Gardaix-Luthereau, Renée, 9, rue Guichard, F-94230 Cachan(FR)**
Inventeur: **Costall, Brenda, Dr., The Old Rectory, Addingham Ilkey West Yorkshire(GB)**
Inventeur: **Naylor, Robert, Dr., The Old Rectory, Addingham Ilkey West Yorkshire(GB)**

EP 0 236 646 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de 2-alcoxy (ou 2-hydroxy ou 2-allyloxy) 4-amino (substitué) 5-(ou 3,5-) halo benzamide, utiles comme activants du système nerveux central.

On connaît, par exemple d'après le brevets BSM 5916 M et FR-A 2 118 960, des dérivés de 2-alcoxy benzamide, tels que le N-(1-éthyl 2-pyrrolidinyl méthyl) 2-méthoxy 5-sulfamoyl benzamide (sulpiride), le N-(éthyl 2-pyrrolidinylméthyl) 2-méthoxy 4-amino 5-chloro benzamide ou le N-(diéthylaminoéthyl) 2-méthoxy 4-amino 5-chloro benzamide (métoclopramide), caractérisés par leur activité antidopaminergique qui a conduit à leur utilisation comme anti-émétiques ou comme neuroleptiques.

Des essais pharmacologiques et biochimiques réalisés avec les composés de la présente invention, ont montré que ceux-ci, malgré leur structure chimique proche de celle des composés connus, ne possédaient pas l'activité antidopaminergique habituelle à la série des 2-alcoxy benzamides, mais qu'ils étaient doués, au contraire, d'une activité activante du système nerveux central.

La présente invention concerne ces nouveaux dérivés de benzamide, leurs procédés de préparation et les médicaments qui les contiennent.

Les composés selon l'invention sont représentés par la formule générale (I):

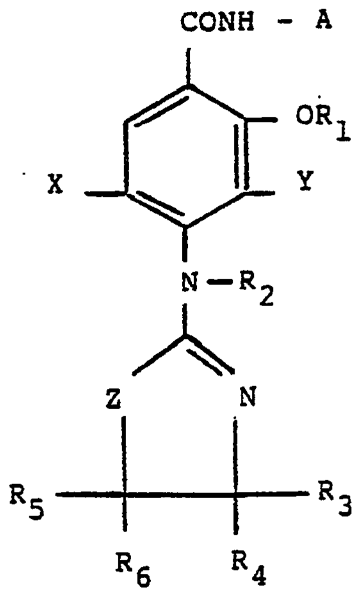

(I)

dans laquelle:

– A représente un groupe alkyle, alcényle, diéthylaminoéthyle, ou un groupe de formule:

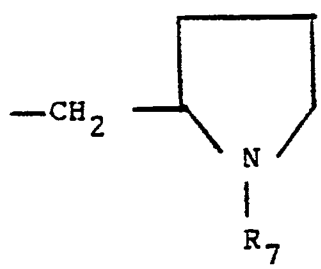

$R_7$ étant un atome d'hydrogène, un groupe alkyle, alcényle, aryle, aralkyle, cycloalkyle, cycloalkylalkyle, cycloalcényle ou cycloalcénylalkyle,

– $R_1$ et $R_2$ représentent des atomes d'hydrogène, des groupes alkyle ou alcényle,

– $R_3$, $R_4$, $R_5$ et $R_6$ représentent des atomes d'hydrogène ou des groupes alkyle;

– X représente un atome de chlore ou de brome,

– Y représente un atome d'hydrogène, de chlore ou de brome,

– Z représente un groupe NH, un atome d'oxygène ou de soufre, les valeurs alkyle, cycloalkyle, alcényle et cycloalcényle contenant un maximum de six atomes de carbone.

L'invention comprend également les sels physiologiquement acceptables des composés de formule (I).

Lorsque la présence d'un atome de carbone asymétrique dans la formule permet l'existence d'isomères optiques, ceux-ci font également partie de l'invention.

Pour préparer les composés de l'invention, on peut faire réagir un acide de formule générale (II):

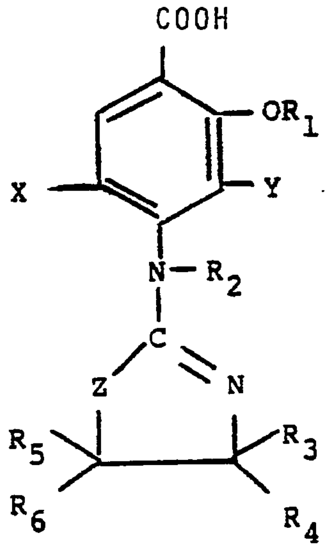

(II)

dans laquelle $R_1$, $R_2$, $R_3$, $R_4$, $R_5$, $R_6$, X, Y et Z ont les mêmes valeurs que dans la formule (I), ou l'un des ses dérivés réactifs, avec une amine de formule générale (III) :

$A-NH_2$

dans laquelle A possède les mêmes valeurs que dans la formula (I),
ou l'un des ses dérivés réactifs, pour obtenir le benzamide attendu. On peut aussi procéder à l'amidification de la fonction acide, avant de fixer les substituants X, Y, ou $R_2$ sur la molécule.

Parmi les dérivés réactifs des acides de formule (II) utilisables dans la synthèse des composés selon l'invention, on peut citer des dérivés tels que halogénure d'acide, ester d'alkyle, ester réactif comme par exemple ester méthoxyméthylique ou ester cyanométhylique, ester aromatique, N-hydroxy imide ester, anhydride symétrique ou anhydride mixte formé par exemple à partir d'un ester d'acide carbonique ou d'un ester haloformique, azide, hydrazide, azolide.

Parmi les dérivés réactifs des amines de formule (III) utilisables pour ces synthèses de benzamides, on peut citer des dérivés tels que ceux obtenus par réaction préalable de l'amine avec un chlorure de phosphore, l'oxychlorure de phosphore, un dialkyl, diaryl-ou orthophénylène-chlorophosphite ou un alkyl-ou aryl-dichlorophosphite, ainsi que l'isothiocyanate de l'amine.

L'invention n'est pas limitée aux dérivés de l'acide et de l'amine cités ci-dessus.

La réaction d'amidification peut être réalisée in situ ou après isolement des dérivés réactifs intermédiaires.

Il est également possible d'effectuer la réaction de l'acide libre et de l'amine libre en présence d'un agent condensant tel que le tétrachlorure de silicium, l'anhydride phosphorique, un carbodiimide ou un alcoxyacétylène.

La réaction d'amidification peut être effectuée en présence ou en l'absence de solvant.

Les systèmes utilisés comme solvants, inertes vis-à-vis de la réaction d'amidification, sont par exemple des alcools, polyols, cétones, benzène, toluène, dioxanne, chloroforme, diméthyl-éther du diéthylèneglycol. Il est aussi possible d'utiliser comme solvant un excès de l'amine employée comme matière première. Il peut être préférable de chauffer le mélange réactionnel pendant l'amidification, par exemple jusqu'au point d'ébullition des solvants cités ci-dessus.

Les composés obtenus selon le procédé de l'invention peuvent réagir si nécessaire avec des acides minéraux ou organiques pharmaceutiquement acceptables tels que l'acide chlorhydrique, l'acide bromhydrique, l'acide sulfurique, l'acide phosphorique, l'acide oxalique, l'acide acétique, l'acide tartrique, l'acide citrique, l'acide méthanesulfonique, pour donner des sels d'addition d'acide.

Ils peuvent également réagir, si nécessaire, avec des halogénures ou des sulfates d'alkyle pour donner des sels d'ammonium quaternaire.

Selon l'une des applications préférentielles de l'invention, on prépare les composés de formule (I) suivant deux variantes à partir d'une même matière première :

1) Le 4-amino 5-halogéno benzoate d'alkyle est traité par le thiophosgène pour donner le 4-isothiocyanato 5-halogéno benzoate d'alkyle (IV) qui est transformé en 4-[N'-amino (ou hydroxy) alkyl] thiouréido 5-halogéno benzoate d'alkyle (V), puis en 4-[4,5-dihydro 2-imidazolyl (ou 2-thiazolyl ou 2-oxazolyl)amino] 5-halogéno benzoate d'alkyle (VI) et enfin en 4-[4,5-dihydro 2-imidazolyl (ou 2-thiazolyl ou 2-oxazolyl) amino] 5-halogéno benzamide (I).

2) Le 4-amino 5-halogèno benzoate d'alkyle est traité par l'acide formique pour donner le 4-formylamino 5-halogèno benzoate d'alkyle (VII) qu'on transforme en 4-dichloroformylimino 5-halogèno benzoate d'alkyle (VIII) puis en 4-(1-H 4,5-dihydro 2-imidazolyl) amino benzoate d'alkyle (VI) et enfin en benzamide de formule (I), dans laquelle Z représente un groupe NH.

Dans ces synthèses les intermédiaires de formules (IV), (V), (VI) et (VIII) sont nouveaux, y compris dans le cas où ils sont 3,5-dihalogènés.

Afin d'illustrer les caractéristiques techniques de la présente invention, quelques exemples de réalisation vont être décrits, étant bien entendu que ceux-ci ne sont pas limitatifs quant à leur mode de mise en oeuvre et aux applications que l'on peut en faire.

Exemple I: N-[2-(DIETHYL AMINO) ETHYL]2-METHOXY 4-[(1-H 4,5-DIHYDRO 2-IMIDAZOLYL) AMINO] 5 CHLORO BENZAMIDE.

Stade I : 2-METHOXY 4-ISOTHIOCYANATO 5-CHLORO BENZOATE DE METHYLE

Dans un tricol de quatre litre équipé d'une agitation, d'un thermomètre et d'un réfrigérant à reflux, on charge 450 cc d'eau, 318 g de 2-méthoxy 4-amino 5-chloro benzoate de méthyle, 155 g de carbonate de calcium, 900 cc de dichloroéthane et 195,5 g de thiophosgène.
On chauffe doucement sous agitation. La réaction commence vers 30-35°. On arrête le chauffage à 40°. La température augmente jusqu'à 48-50°. On refroidit avec un bain d'eau et de glace jusqu'à 40° et maintient cette température pendant 15 minutes environ. Le dégagement gazeux diminue. On chauffe lentement jusqu'au reflux à 75° et laisse deux heures. Le dégagement de $CO_2$ cesse après environ 1 heure de reflux.
On refroidit vers 30° décante et lave la phase aqueuse par 100 cc de dichloroéthane.

Les phases organiques sont réunies, on ajoute un litre d'eau et distille l'azéotrope sous vide, jusqu'à épuisement du dichloroéthane. La suspension aqueuse est essorée, lavée à l'eau puis remise en suspension dans un litre d'éther de pétrole, agitée 15 minutes, essorée, lavée à l'éther de pétrole et séchée à 50°.

On obtient : 364 g. de produit (rendement = 95,8 %)
P F = 90°
RMN = Compatible, pas d'impureté décelée.

Stade II : 2-METHOXY 4-(N'2-AMINO ETHYL)-THIOUREIDO 5-CHLORO BENZOATE DE METHYLE

Dans un tricol de 1 litre équipé d'une agitation, d'un thermomètre et d'une ampoule d'introduction, on charge 13,4 ml (0,2mole) d'éthylène diamine et 300ml d'éther isopropylique. On refroidit à 0°,-5° et coule goutte à goutte, à cette température, en 30 minutes une solution de 25,75g (0,1 mole) de 2-méthoxy 4-isothiocyanato 5-chloro benzoate de méthyle dans 150ml de toluène. On agite encore 2 heures à 5° et essore le précipité, que l'on recharge humide dans le tricol avec 250 cc d'eau. On agite 15 minutes à 5°, essore, lave à l'eau et sèche à 50°.

On obtient : 29,7 g de produit (93,5 %)
P F = 80-90°
TITRE = 91,1 %
S = 9,35 % (calculée 10,08 %)
Rendement en produit pur = 85,2 %

Stade III : 2-METHOXY 4-[(1-H 4,5-DIHYDRO 2-IMIDAZOLYL) AMINO]5 CHLORO BENZOATE DE METHYLE

Dans un tricol de quatre litres muni d'une agitation, d'un thermomètre, d'un réfrigérant à reflux et d'une ampoule d'introduction, on charge 322,2 g (environ 0,92 mole) de 2-méthoxy 4-(N'2-aminoéthyl) -thiouréido 5-chloro benzoate de méthyle et 645 ml d'eau. On chauffe la suspension à 70-75° et coule en une heure 461,6 g (1,218 mole) de solution d'acétate de plomb dans 1,8 litre d'eau distillée.
On maintient deux heures à 70-75° et refroidit à 20°. Le sulfure de plomb est essoré,lavé à l'eau, et séché (258,1 g).
Le filtrat est rechargé dans un tricol de six litres. On coule sous agitation une solution, préalablement passée au noir et filtrée, de 132,5 g de carbonate de sodium (1,25 mole) dans un litre d'eau. On agite une heure (pH 7-8), filtre lave et sèche le précipité. On recueille 79,5 g .
Les eaux-mères sont traitées par à nouveau 132,5 g de carbonate de sodium et on obtient un précipité qu'on essore, lave et sèche. On recueille ainsi 210,4 g de cristaux fondant à 216-217°C.
Rendement global : 80,4 %.

STADE IV : N-[2-(DIETHYL AMINO) ETHYL] 2-METHOXY 4- [(1-H 4,5-DIHYDRO 2-IMIDAZOLYL) AMINO]5-CHLORO BENZAMIDE

Dans un tricol de 250 ml on charge 28,35 g (0,1 mole) de 2-méthoxy 4-[(1-H 4,5-dihydro 2-imidazolyl) amino] 5-chloro benzoate de méthyle, 28 ml d'éthylène glycol, et 12,76 g (0,11mole) de diéthyl amino éthylamine et on agite 48 heures à 70-75°. On ajoute 50 cc d'eau, refroidit à 10°, essore, lave à l'eau et sèche. On obtient 30,7 g d'un produit qui fond à 185-190° (83,5 %).
22,3 g du produit sont chauffés à ébullition dans 100 cc d'eau et 3cc de soude, pendant 15 minutes. On filtre à 60°, lave le précipité à l'eau et sèche.
On obtient 19,8 g (soit 88,8 %) (PF = 198°)
RMN = conforme, ester non décelé.
17,8 g. de produit sont dissous à chaud dans 240 cc de dichloroéthane, traités au noir acticarbone 3 S, filtrés, cristallisés à froid, essorés, lavés au dichororéthane et séchés à 50°.
Le produit, contenant encore du dichloroéthane a été reséché deux fois 48 heures à 70°. Il restait encore du dichloroéthane.
Poids obtenu : 12,1 g (rendement 64,2 %)

Le produit a été empâté dans l'acétone à 60°, refroidi, filtré et lavé à l'acétone,séché 48 heures à 70°. On a 9,7 g de produit.
PF Büchi : 190,5-191,5° PF Kofler : 194-195°
RMN : Compatible; contient encore un peu de dichloroéthane
Titre : 98,4 %
CCM : 3 taches secondaires - Rf. 0,22 < 0,1 %
0,37 0,1 %
0,75 < 0,1 %

Exemple II : N-[2-(DIETHYL AMINO) ETHYL]2-METHOXY 4-[N-ETHYL, N-(1-H 4,5-DIHYDRO 2-IMIDAZOLYL) AMINO]5-CHLORO BENZAMIDE

Dans un autoclave de 250 ml, on charge 42 g (0,114 mole) de N-[2-(diéthyl amino) éthyl] 2-méthoxy 4-[( 1-H 4,5-dihydro 2-imidazolyl) amino] 5-chloro benzamide, 100 ml de méthanol, et 24,8 g (0,228 mole) de bromure d'éthyle. On chauffe à 100° sous agitation. La pression monte à 10 kg. puis redescend à 6 kg. La température se stabilise à 90°. On laisse agiter 6 heures, refroidit et chasse le méthanol sous vide. Le résidu est dissous dans 100 ml d'eau et alcalinisé en refroidissant par 25 g de potasse en pastilles.
On ajoute 150 ml de chlorure de méthylène et agite. Il se forme un précipité qui est essoré et lavé par 50 ml de chlorure de méthylène. Le filtrat est décanté, la phase aqueuse est lavée par 50 ml de chlorure de méthylène.
Le solvant est chassé à sec sous vide et le résidu huileux, agité avec 100 ml d'éther isopropylique, cristallise.
Le précipité est essoré, lavé par 50 ml d'éther isopropylique et séché.
On obtient : 22 g de produit (rendement = 48,8 %)
P F = 135°
R M N : Compatible . Contient 2 à 3 % du produit de départ.

Le produit est dissous à chaud dans 90 cc d'acétate d'éthyle, traité au noir acti carbone 3 S, filtré, cristallisé à froid (- 10°) essoré, lavé à l'éther isopropylique et séché.

On obtient : 16,2 g de produit ( rendement = 73,6 % )
PF (Kofler) = 136°C
Perte de poids (100°) = 0,12 %
Titre = 99,1 %
C1 = 8,93 % - Calculé = 8,95 %
N = 17,76 % - Calculé = 17,69 %

Les spectres R M N et I R sont compatibles.

Exemple III : N- [2-(DIETHYLAMINO) ETHYL] 2-METHOXY 3,5-DIBROMO 4- [(1-H 4,5-DIHYDRO 2-IMIDAZOLYL) AMINO] BENZAMIDE

Stade I : 2-METHOXY 4-ISOTHIOCYANATO 5-BROMO BENZOATE DE METHYLE

Dans un tricol de 2 litres équipé d'une agitation, d'un thermomètre, d'un réfrigérant à reflux et d'un piège, on charge 260 g (1 mole) de 2-methoxy 4-amino 5-bromo benzoate de méthyle 110 g (1,1 mole) de carbonate de calcium, 300 cc d'eau, 600 cc de 1-2 dichloréthane et 88 cc (132 g, soit 1,15 mole) de thiophosgène. On chauffe doucement : le dégagement de gaz carbonique commence vers 30-35°. On arrête le chauffage à 40° et maintient cette température pendant environ 1 h. avec un bain d'eau. Le dégagement gazeux diminue.
On chauffe lentement, en 1h. jusqu'au reflux ( 70-75°) et laisse 3 h. à cette température.

On refroidit à 45-50°, filtre éventuellement un léger insoluble, décante et lave la phase aqueuse par 100 ml de dichloroéthane.

On ajoute 300 cc d'eau à la phase organique et chasse le solvant sous vide. La suspension aqueuse est essorée, lavée et remise en suspension sous agitation dans 600cc d'éther de pétrole, puis essorée à nouveau, lavée à l'éther de pétrole et séchée à 50°.

On obtient 290,09 g de produit (Rendement = 96 %).
PF° = 99°
RMN compatible. Pas d'impureté décelée.
S % = 10,16 (calculée = 10,59).

Stade II : 2-METHOXY 4-(N' 2-AMINO ETHYL) THIOUREIDO 5-BROMO BENZOATE DE METHYLE

Dans un tricol de 2 l. muni d'une agitation, d'un thermomètre et d'une ampoule d'introduction, on charge 1,1litre d'éther isopropylique et 63,5 g soit 1,06 mole d'éthylène diamine et refroidit à 0°.
On coule goutte à goutte, en 30 minutes, sous agitation, une solution filtrée de 106,6 g (0,351 mole) de 2-méthoxy 4-isothiocyanato 5-bromo benzoate de méthyle dans 350 ml de toluène en maintenant la température à 0°. On agite 1 h. à cette température, puis sépare les solvants. Le produit gommeux reste sur les parois. On ajoute 1 l. d'eau dans le tricol et agite une demi-heure. le produit cristallise. Il est essoré et lavé à l'eau, puis séché à 50°.
On obtient 85,7 g de produit. (Rendement = 67,4 %)
PF° = 80°-90° (pâteux)
Titre = 90,4 %
S % = 8,10 (calculé = 8,84).

Stade III : 2-METHOXY 4-[(1-H 4,5-DIHYDRO 2-IMIDAZOLYL)AMINO]5-BROMO BENZOATE DE METHYLE

Dans un tricol de 1 l. muni d'une agitation, d'un thermomètre et d'une ampoule d'introduction, on charge 65 g (0,167 mole) de 2-méthoxy 4-(N'2-aminoéthyl) thioureïdo 5-bromobenzoate de méthyle (titre 98,8 %), et 60 ml d'eau.
On chauffe sous agitation à 70°, et coule en 30 minutes, à cette température 75 g (0,198 mole) d'acétate de plomb dissous dans 30 cc d'eau distillée.
On laisse agiter deux heures à 70-80°, refroidit à 20°, filtre le sulfure de plomb, et le lave à l'eau (46,74 g de sulfure séché). Le filtrat est traité par 15 g de carbonate de sodium dans 150 cc d'eau (pH 6-7). Le précipité est essoré, lavé, séché (11,16 g de sels). Le filtrat est alcalinisé par 27,4 g de carbonate de sodium, agité 10 minutes; le précipité est essoré, lavé et séché.

On obtient 47,92 g de produit. (Rendement = 87,7 %)
PF° = 214°C
Titre = 98 %
RMN compatible.

Stade IV : 2-METHOXY 3,5-DIBROMO 4-[(1-H 4,5-DIHYDRO 2-IMIDAZOLYL)-AMINO] BENZOATE DE METHYLE

Dans un tricol, on charge sous agitation 362 g (1,1 mole) de 2-methoxy 4- [(1-H 4,5-dihydro 2-imidazolyl) amino] 5-bromo benzoate de méthyle, 550 ml d'acide acétique et 256 g (1,43 mole) de N-bromo succinimide. On chauffe à 55° et on agite à cette température pendant 5 heures, puis on laisse reposer à 55° pendant 70 heures.
La masse réactionnelle est refroidie, versée dans 5 litres d'eau sous agitation. L'insoluble est essoré et lavé.
Le filtrat est alcalinisé par 750 ml de lessive de soude (pH 6) en refroidissant avec de la glace, puis avec 230 g de carbonate de sodium, jusqu'à pH 9-10. Le précipité formé est essoré, lavé à l'eau et séché.

On obtient 416 g (rendement = 92,44 %)
Br = 39,63 % (calculé = 39,31 %)
PF Kofler = 193°C(légère fusion partielle à 170°)
R M N = Compatible.

Stade V : N-[2-(DIETHYL AMINO) ETHYL] 2-METHOXY 3,5-DIBROMO 4-[(1-H 4,5-DIHYDRO 2-IMIDAZOLYL) AMINO] BENZAMIDE

Dans un tricol de 250 cc, on charge 30,5 g (0,075 mole) de 2-méthoxy 3,5-dibromo 4-[(1-H 4,5-dihydro 2-imidazolyl) amino]benzoate de méthyle, 50 cc d'éthylène glycol et 17,4 g (0,15 mole) de diéthyl amino éthylamine. On maintient 72 heures à 70-75°, sous agitation.
On ajoute 150 cc d'eau, refroidit à 10°, essore, lave à l'eau, puis à l'acétone et sèche.
On obtient : 18,7 g (rendement = 50,8 %)

R M N : compatible Titre = 99,7 %

18 g. sont dissous à chaud dans 15 volumes (270 cc) de méthyl éthyl cétone, traités au noir, filtrés, cristallisés à froid, essorés lavés à l'acétone et séchés.

On obtient : 14,5 g (rendement de purification = 80,7 %)
PF (Kofler) = 192°C
Perte de poids à 100°C = 0,07 %
Titre = 99,7 %
Br = 32,49 % - (calculé = 32,49 %)
N = 14,17 % - (calculé = 14,26 %)

Les spectres R M N et I R sont compatibles.

Exemple IV : N-[2-(DIETHYL AMINO) ETHYL]2-METHOXY 3,5-DICHLORO 4 [(1-H 4,5-DIHYDRO 2-IMIDAZOLYL) AMINO] BENZAMIDE

Stade I : 2-METHOXY 4-FORMYLAMINO 5-CHLORO BENZOATE DE METHYLE

Dans un ballon de trois litres muni d'un agitateur, d'un thermomètre, d'un réfrigérant et d'une ampoule à brome, on introduit 829 g (768 ml soit 8,13 moles) d'anhydride acétique (d=1,08), et en filet, 1.444 g (1.184 ml 31,4 moles) d'acide formique (d = 1,22).
La réaction est exothermique (40°). On chauffe ensuite une heure à 50° et on refroidit entre 5 et 10°.
On ajoute ensuite par portions 345 g (1,6 mole) de 2-méthoxy 4-amino 5-chloro benzoate de méthyle.
On chauffe ensuite 2,5 heures à 50°C, refroidit à 20°C et verse la solution dans 10 litres d'eau. Le précipité est essoré, lavé à l'eau et séché à l'étuve à 50°C.
Rendement = 366 g. (94 %)
P F = 150°C (Kofler)

Stade II : 2-METHOXY 3,5-DICHLORO 4-DICHLORO FORMYLIMINO BENZOATE DE METHYLE

Dans un ballon de un litre muni d'un agitateur, d'un thermomètre et d'un réfrigérant relié à un barboteur à soude, on introduit 443 g (270 ml soit 3,72 moles) de chlorure de thionyle, et 161,3 g (96 ml soit 1,68 mole) de chlorure de sulfuryle. On refroidit à 0°C et ajoute par portions, en 10 mn environ, 121,7 g (0,50 mole) de 2-méthoxy 4-formylamino 5-chloro benzoate de méthyle qui se dissout. On agite 1 h 30, en ôtant le bain froid, et on laisse reposer une nuit à 15°C. On évapore les solvants sous vide, en ne dépassant pas 30°C dans la masse. On obtient un résidu pulvérulent, qu'on utilise tel que, sans purification (poids=163 g).

Stade III : 2-METHOXY 3,5-DICHLORO 4-[(1-H- 4,5-DIHYDRO 2-IMIDAZOLYL) AMINO] BENZOATE DE METHYLE

Dans un ballon de deux litres muni d'un agitateur, d'un thermomètre, d'un réfrigérant et d'une ampoule à brome, on a introduit 89,9 g (1,50 mole) d'éthylène diamine, et 1000 ml d'acétate d'éthyle. On refroidit à -20°C et on ajoute en filet, en 15 minutes environ le produit obtenu au stade II dans 500 ml d'acétate d'éthyle.
Au début de l'addition un précipité orange se forme. En fin d'introduction, il y a prise en masse autour de l'agitateur qui se bloque. On laisse en contact 15 mn à - 20°C puis ajoute, en laissant remonter à 0°C, une solution de 300 ml d'acide chlorhydrique d( = 1,18) dans 500 ml d'eau.
La phase aqueuse est décantée, extraite 2 fois avec 250 ml de chlorure de méthylène, puis additionée de 200 g de glace et 50 ml de chlorure de méthylène. On alcalinise par 300 ml d'ammoniaque (d = 0,90), sans dépasser 10°.
On laisse agiter 45 mn entre 5 et 10°C et essore le précipité qu'on lave cinq fois avec 40 ml d'eau et sèche à l'étuve à 50°C.
Rendement = 21 g (13 %)
P F = 207°C
C1 % Trouvé = 22,90 Calculé = 22,33
Titre % Trouvé = 93,4 Calculé = 100

Stade IV : N-[2-(DIETHYLAMINO) ETHYL] 2-METHOXY 3,5-DICHLORO 4-(1-H 4,5-DIHYDRO 2-IMIDAZOLYL) AMINO] BENZAMIDE

Dans un ballon de 250 ml muni d'un agitateur, d'un thermomètre et d'un réfrigérant, on introduit 23 g (0,072 mole) de 2-méthoxy 3,5- dichloro 4- [(1-H 4,5- dihydro 2-imidazolyl) amino] benzoate de méthyle, 40 ml de glycol, 16,7 g (0,144 mole) de NN diéthyl amino éthylamine et 3 gouttes d'acide chlorhydrique (d = 1,18).

On chauffe le mélange 67 heures à 75°C, on refroidit, et on verse la suspension dans 300 ml d'eau.

Les cristaux sont essorés, lavés à l'eau (4 fois 30 ml) et séchés une nuit à l'étuve à 50°C.
Rendement = 10,6 g. (36,5 %)
P F = 187°C (Kofler)

22,6 g du produit sont dissous dans 66 ml d'éthanol à 80 %, à l'ébullition. On filtre en présence de charbon végétal et laisse cristalliser le filtrat une nuit au réfrigérateur. Le solide est essoré, lavé deux fois avec 5 ml du solvant de recristallisation et séché à l'étuve à 50°C.
Rendement = 18,8 g. (83 %)
P F 187°C (Kofler)

| | Calculé | Trouvé |
|---|---|---|
| $H_2O$ % | | 0,1 |
| Titre % | 100 | $\frac{199,2}{2}$ |
| Cl % | 17,62 | 17,62 |

EXEMPLE V : N-[(1-ETHYL 2-PYRROLIDINYL) METHYL]2-METHOXY 3,5-DIBROMO 4-[(1-H 4,5-DIHYDRO 2-IMIDAZOLYL) AMINO] BENZAMIDE

Dans un tricol de 500 ml, on charge 81,4 g (0,2 mole) de 2-méthoxy 3,5-dibromo 4-[(1-H 4,5-dihydro 2-imidazolyl) amino] benzoate de méthyle, 80 ml d'éthylène glycol, et 51,2 g (0,4 mole) de 1-éthyl 2-amino méthyl pyrrolidine et on agite 72 heures à 70-75°.

On refroidit, et la pâte noire est extraite par 3 x 150 ml d'éther éthylique. On évapore sous vide le solvant de cet extrait, et on obtient 20 g d'une pâte qui cristallise dans 100 ml d'éther isopropylique. On essore, lave à l'éther isopropylique et sèche. On obtient 6,8 g de cristaux fondant à 115°C. On reprend la pâte noire qui restait dans l'éthylène glycol, par 150 ml d'eau et on l'extrait par 5 fois 200 ml de chlorure de méthylène. Le solvant, séché sur sulfate de magnésie est évaporé sous vide. Le résidu (78,7 g) est dissous à chaud dans 150 ml d'acétate d'éthyle et cristallisé à froid, filtré, lavé à l'éther isopropylique et séché. On obtient 29,65 g de produit à purifier. Les eaux mères aqueuses ont cristallisé. On essore, lave et sèche, et on obtient 8 g de cristaux.
Les trois jets (43 g.) sont dissous à chaud dans 130 ml d'isopropanol, traités au noir, cristallisés une nuit au réfrigérateur, lavés et séchés. On obtient 22,1 g de produit (rendement = 22 %) fondant à 180°C, que l'on purifie. Pour cela, 26,6 g de produit sont dissous à chaud dans 185 ml de méthyl éthyl cétone, traités au noir, cristallisés à froid, essorés, lavés à la méthyl éthyl cétone et séchés. on récupère 18,6 g. de cristaux beige qui sont à nouveau recristallisés dans la méthyl éthyl cétone, avec un traitement au noir. On obtient 13,1 g (rendement de purification = 49,3 %) de cristaux.
P F = 180°C (Kofler) , Titre corrigé = 98,5 %
Br = 32,47 % corrigé = 32,50 % - Calculé = 31,75 %
N = 13,68 % corrigé = 13,69 % - Calculé = 13,92 %

Les spectres R M N et I R sont compatibles.

EXEMPLE VI : N- [2 (DIETHYL AMINO) ETHYL] 2-METHOXY 4-[N-ALLYL, N-(1-H 4,5-DIHYDRO 2-IMIDAZOLYL) AMINO] 5-CHLORO BENZAMIDE

Stade I : 2-METHOXY 4-[N-ALLYL, N-(1- H 4,5-DIHYDRO 2-IMIDAZOLYL) AMINO]5-CHLORO BENZOATE DE METHYLE (BROMHYDRATE)

Dans un autoclave de 1 litre on charge 113,4 g. (0,4 mole) de 2-méthoxy 4-[(1-H 4,5-dihydro 2-imidazolyl) amino] 5-chloro benzoate de méthyle, 400 ml de méthanol et 96,8 g. (0,8 mole) de bromure d'allyle, et on chauffe à 100° sous agitation pendant 18 h. On refroidit et évapore le solvant sous vide.
Le résidu pâteux est repris par 200 ml d'acétone, agité, essoré, lavé par 200 ml d'acétone et séché. On obtient 103,2 g ( rendement = 63,8 %) de produit ayant les caractéristiques suivantes :
P F = 226° instantané puis 220°

Titre = 99 %
H Br = 19,53 % (calculé 20,02 %)
R M N = compatible

Stade II : N-[2-(DIETHYL AMINO) ETHYL] 2-METHOXY 4-[N-ALLYL, N-(1-H 4,5-DIHYDRO 2-IMIDAZOLYL) AMINO] 5-CHLORO BENZAMIDE

Dans un tricol de 500 ml, on charge 40,45 g (0,1 mole) de bromhydrate de 2-méthoxy 4-[N-allyl, N-(1-H 4,5-dihydro 2-imidazolyl) amino]5-chloro benzoate de méthyle, 40 ml d'éthylène glycol et 34,8 g (0,3 mole) de diéthylamino éthylamine, et on agite 72 heures à 70-75°C. On refroidit et ajoute 110 ml de lessive de soude, refroidit encore à 5°, ajoute 150 ml d'éther éthylique et filtre l'émulsion formée. Le gâteau est lavé par 300 ml d'eau et séché.

On obtient = 12,6 g (rendement = 30,9 %)
P F = 142°C
R M N = compatible (impureté aromatique en faible quantité)

On évapore l'éther éthylique du filtrat, et extrait celui-ci par 200 ml, puis par 2 x 100 ml de chlorure de méthylène.
La phase organique est séchée sur sulfate de magnésie , et évaporée sous vide. Le résidu est repris dans 100 ml d'éther isopropylique, filtré, lavé par 2 x 50 ml d'éther isopropylique et séché à 50°C.
On obtient 12,6 g de produit (rendement 30,9 %)
PF = 140°C
R M N = compatible (impureté non décelée)

34,4 g. de produit sont dissous à chaud dans 200 ml d'acétate d'isopropyle, traités au noir, cristallisés une nuit au réfrigérateur, essorés, lavés à l'acétate d'isopropyle et séchés.

On obtient = 27,6 g de produit purifié.
Ce produit est à nouveau recristallisé et traité au noir deux fois de suite, et séché à 60°.

On obtient 16,5 g de produit (rendement de purification 48 %) fondant à 141°C
Titre 98,9 %
R M N = compatible

Exemple VII : N-[(1-ETHYL 2- PYRROLIDINYL) METHYL]2-METHOXY 4-[N-ALLYL, N-(1-H, 4,5-DIHYDRO 2-IMIDAZOLYL) AMINO]5-CHLORO BENZAMIDE

Dans un tricol de 500 ml on charge 60,68 g (0,15 mole) de bromhydrate de 2-méthoxy 4-[(N- allyl, N-(1-H 4,5-dihydro 2-imidazolyl) amino]5-chloro benzoate de méthyle, 60 ml d'éthylène glycol et 38,4 g (0,3 mole) de 1-éthyl 2-aminométhyl pyrrolidine et on agite 72 heures à 70-75°C. On refroidit et on ajoute 15 ml de lessive de soude. La masse noirâtre est extraite par 200 ml puis 2 x 100 ml d'éther éthylique. Le solvant concentré à sec sous vide donne 19,6 g. d'huile (excès d'amine de départ).
La phase aqueuse est étendue avec 150 ml d'eau et extraite par 200 ml puis 2 x100ml de chlorure de méthylène.
Le solvant séché sur sulfate de magnésie est chassé sous vide, et le résidu pâteux est cristallisé dans 300 ml d'éther isopropylique, essoré, (produit gommeux), empâté dans 50 ml d'acétate d'isopropyle, essoré, lavé par 50 ml d'acétate d'isopropyle et séché à 50°.
On obtient 21,6 g (rendement = 34, 3 %) de produit fondant à 123°
R M N = compatible (impureté à la limite de détection)

27,3 g. de produit sont dissous à chaud , dans 60 ml d'acétate d'isopropyle, traités au noir, cristallisés une nuit au réfrigérateur,essorés, lavés à l'éther isopropylique et séchés. Après une seconde cristallisation dans 36 ml d'acétate d'isopropyle, on obtient 7,9 g de produit beige (rendement de purification = 29 %).

P F = 120-122° (pas net)
Titre = 98,2 %
R M N = Compatible (spectre mal résolu)

Exemple VIII : N-[(1-CYCLOPROPYLMETHYL 2-PYRROLIDINYL) METHYL]2-METHOXY 3,5-DICHLORO 4-[(1-H 4,5-DIHYDRO 2-IMIDAZOLYL) AMINO] BENZAMIDE

STADE I : 2-méthoxy 4-acétamino 3,5-dichloro benzoate de méthyle

Dans un ballon d'un litre muni d'un agitateur et d'un thermomètre on introduit 256 g (1 mole) de 2-méthoxy 4- acétamino 5-chloro benzoate de méthyle et 1000 cc d'acide acétique. On obtient une suspension à laquelle on ajoute en une fois 177 g (1mole + 30 % d'excès) de chlorosuccinimide. On chauffe au bain d'eau à 50° - 55° pendant 2 heures jusqu'à ce que tout soit dissous. On supprime alors l'agitation et laisse réagir à l'étuve à 50° - 55° pendant environ 40 heures. Il ne reste alors en solution que des traces de chlorosuccinimide (vérification par dosage).

On refrodit, dilue avec environ 10 litres d'eau : l'ester précipite d'abord liquide puis solide. Il est essoré, lavé à l'eau et séché; Poids obtenu = 237 g (rendement = 81 %), F = 132-133°C.
Après recristallisation dans l'alcool absolu, on obtient 185 g de cristaux fondant à 141-142°C. (rendement 78 %)
rendement total 63 %

STADE II : 2-METHOXY 3,5-DICHLORO 4-AMINO BENZOATE DE METHYLE

Dans un ballon de 6 litres muni d'un agitateur, d'un thermomètre, d'un réfrigérant et d'une ampoule à brome, on introduit 407 g (1,39 mole)de 2-méthoxy 3,5-dichloro 4-acétamino benzoate de méthyle, 2215 ml de méthanol, et en filet, 161 g (88 ml) d'acide sulfurique (d = 1,83). La température augmente jusqu'à environ 40°C. On chauffe à reflux (65°C) 21 heures, refroidit à 20°C et coule le mélange réactionnel dans un réacteur contenant 3500 g d'eau et 1500 g de glace.
Le produit cristallise. On l'essore, le lave à l'eau et le sèche à l'étuve à 50°C. Rendement = 88 % (soit 305 g)
PF = 69 °C
Le spectre R M N est compatible avec la structure attendue.
Cl % : trouvé 28,09 calculé 28,40

Stade III : 2-METHOXY 3,5-DICHLORO 4-FORMYLAMINO BENZOATE DE METHYLE

Dans un ballon de 4 litres muni d'un agitateur, d'un thermomètre, d'un réfrigérant et d'une ampoule à brome, on introduit 630 g (6,2 moles) d'anhydride acétique, et en filet, 1083 g (23,5 moles) d'acide formique.La réaction est exothermique et la température passe de 23 à 47°C en fin d'addition. On chauffe ensuite une heure à 50-55°C, on refoidit à 5°C et on ajoute par portions 305 g (1,22 mole) de 2-méthoxy 3,5-dichloro 4-amino benzoate de méthyle.
On laisse revenir à température ambiant en ôtant le bain froid puis on chauffe deux heures et demie à 50-55°C. On refroidit ensuite à 20°C et verse le mélange réactionnel dans un réacteur contenant 4400 ml d'eau et 1700 g de glace.
Le produit cristallise. On l'essore, le lave à l'eau et le sèche à l'étuve à 50°C. Rendement = 312 g (92 %)
PF = 136-138°C

Stade IV : 2-METHOXY 3,5-DICHLORO 4-[(1-H 4,5-DIHYDRO 2-IMIDAZOLYL) AMINO]BENZOATE DE METHYLE

Dans un ballon de 1 litre muni d'un agitateur, d'un thermomètre, d'un réfrigérant relié à un barboteur contenant de la potasse en pastilles, on introduit 93 g (0,33 mole) de 2-méthoxy 3,5-dichloro 4-formylamino benzoate de méthyle, 386,5 g de chlorure de thionyle et 50,4 g de chlorure de sulfuryle. On agite la suspension 22 heures entre 20 et 25°C (température ambiante). Il y a solubilisation totale après quelques heures de réaction.
On évapore sous vide sans chauffer l'excès des réactifs, on ajoute 135 ml d'acétate d'éthyle et évapore sous vide, à nouveau , sans chauffer le solvant.
On ajoute 135 ml d'acetate d'éthyle à l'huile résiduelle et coule la solution obtenue dans un réacteur maintenu vers - 15°C et contenant 59,1 g d'éthylène diamine et 390 ml d'acétate d'éthyle.
On ôte ensuite le bain froid et lorsque la température de la masse réactionnelle est revenue à 20°C, on verse la suspension lentement dans un mélange de 196,5 ml d'acide chlorhydrique( d = 1,18) et de 132 ml d'eau.
La température augmente jusqu'à 35°C. On refroidit de façon à ne pas dépasser 30°C puis ajoute 475 ml d'eau et décante immédiatement la phase organique.
On lave la phase organique avec 120 ml d'eau puis les phases aqueuses sont réunies et lavées avec 200 ml de chlorure de méthylène.
La solution aqueuse est refroidie et on la neutralise à pH = 9 - 10, sans dépasser 30°C, avec environ 225 ml d'ammoniaque( d = 0,91).
On laisse cristalliser le produit , l'essore puis le lave à l'eau avant de le sécher à l'étuve à 50°C. Poids obtenu = 53 g
rendement = 50 %
PF = 206°C

Stade V : N-[(1-CYCLOPROPYLMETHYL 2-PYRROLIDINYL) METHYL]2-METHOXY 3,5-DICHLORO 4-[(1-H 4,5-DIHYDRO 2-IMIDAZOLYL) AMINO]BENZAMIDE

Dans un ballon de 1 litre muni d'un agitateur, d'un thermomètre, d'un réfrigérant, on introduit 23,3 g (0,073 mole) de 2-méthoxy 3,5-dichloro 4-[(1-H 4,5-dihydro 2-imidazolyl) amino]benzoate de méthyle, 22,5 g de 1-cyclopropylméthyl 2-aminométhyl pyrrolidine et 200 ml de méthanol. On chauffe 10 jours à reflux, et on évapore le méthanol sous vide. On dissout l'huile colorée obtenue dans 150 ml d'acétate de butyle à 60°C. On glace ensuite la solution, et on laisse cristalliser 24 heures à 20°C après amorçage.
On essore le produit, on lave avec un peu d'acétate d'éthyle, et on sèche à l'étuve à 50°. On obtient 22 g de cristaux (rendement = 69 %) qui fondent à 158-160°C.
Le produit peut être recristallisé dans 220 ml d'un mélange préparé à partir de 200 ml d'acétate d'éthyle et 20 ml de méthanol.
On obtient 10 g. de cristaux. PF = 162°C

Exemple IX : N- [[1- (1-CYCLOHEXENYLMETHYL) 2-PYRROLIDINYL] METHYL]2-METHOXY 4-[ (1-H 4,5-DIHYDRO 2-IMIDAZOLYL) AMINO]5-CHLORO BENZAMIDE

Stade I : ACIDE 2-METHOXY 4-[(1H 4,5-DIHYDRO 2-IMIDAZOLYL) AMINO]5 CHLOROBENZOIQUE

On traite 11,8 g de 2-méthoxy 4-[(1-H 4,5-dihydro 2-imidazolyl) amino]5- chloro benzoate de méthyle par 20 ml d'eau et 50 ml de lessive de soude (N) pendant 2 heures à 70°C. Puis on refroidit à 20°C et on neutralise par de l'acide chlorhydrique en solution normale. Le précipité obtenu est lavé et séché. On obtient l'acide sous forme de monohydrate. Les cristaux fondent à 190-200°C, avec décomposition (rendement = 99 %).

Stade II : N[[1-(1-CYCLOHEXENYLMETHYL) 2-PYRROLIDINYL]METHYL]2-METHOXY 4-[(1-H 4,5-DIHYDRO 2-IMIDAZOLYL) AMINO]5-CHLOROBENZAMIDE

Dans un ballon de 250 ml muni d'un agitateur, d'un thermomètre, d'un réfrigérant et d'une ampoule à brome, on introduit 4,85 g (0,018 mole) d'acide 2-méthoxy 4-[(1-H 4,5-dihydro 2-imidazolyl) amino]5-chloro benzoïque, 50 ml d'acétone et 5,45 g (0,054 mole) de triéthylamine. On obtient une suspension, et on ajoute goutte à goutte en maintenant la température à 20°C avec un bain de glace, 5,8 g (0,054 mole) de chloroformiate d'éthyle. On laisse ensuite agiter 45 minutes à 20°C et on ajoute 4,2 g (0,021 mole) de 1-(1cyclohexenylméthyl) 2-aminométhyl pyrrolidine, goutte à goutte, à 20°C (refroidir)
On agite pendant 1 heure la suspension obtenue et on laisse une nuit au repos.
On ajoute 108 ml de soude N, chasse l'acétone sous vide et essore le produit pâteux qui est repris dans un mélange de 2 ml de lessive de soude, 50 ml d'éthanol et 20 ml d'eau.
On chauffe 30 minutes au reflux, ajoute 70 ml d'eau, évapore l'éthanol sous vide et essore les cristaux qui sont lavés à l'eau et séchés à l'étuve à 50°C.
Rendement = 4,9 g. (61 %)
P F = 181°C
Le produit est recristallisé dans 60 ml d'éthanol à 95 %
Rendement = 3,1 g.
P F = 190°C
Spectre R M N compatible avec la structure attendue.

Exemple X : N-[[1-(1-CYCLOHEXENYL METHYL) 2-PYRROLIDINYL]METHYL]2-METHOXY 3,5-DICHLORO 4-[(1-H 4,5-DIHYDRO 2-IMIDAZOLYL) AMINO]BENZAMIDE

Dans un ballon de 250 ml muni d'un agitateur, d'un thermomètre, d'un réfrigérant et d'une ampoule à brome, on a introduit 4,5 g (0,015 mole) d'acide 2-méthoxy 3,5-dichloro 4-[(1-H 4,5-dihydro 2- imidazolyl) amino]benzoïque, 4,5 ml d'acétone, et 4,5 g (0,045 mole) de triethylamine. On obtient une suspension sur laquelle on coule 4,90 g (0,045 mole) de chloroformiate d'éthyle goutte à goutte, en maintenant la température à 20°C par refroidissement.
On laisse agiter 45 minutes à 20°C puis on ajoute goutte à goutte, toujours à 20°C, 3,4 g (0,0175 mole) de 1-(1-cyclohexenylméthyl) 2-amino méthylpyrrolidine. La réaction est exothermique, et il faut refroidir pour rester à 20°C.
On laisse ensuite agiter deux heures à 20°C et reposer une nuit.
On ajoute 87 ml de soude N et évapore l'acétone et une grande partie de l'eau sous vide. On obtient une pâte sur laquelle on ajoute 50 ml d'éthanol et 1,5 ml de soude. On chauffe 30 minutes à reflux, évapore l'éthanol sous vide et ajoute 30 ml d'eau.
Le produit cristallise lentement. On l'essore, le lave à l'eau et sèche à l'étuve à 50°C.
Rendement = 4,65 g (65 %)
PF = 128°C
Ce produit est recristallisé dans 11,5 ml d'éthanol à 95 % et donne 2 g de cristaux fondant à 156°C. Le spectre R M N est compatible avec la structure du produit attendu.

Exemple XI : N-(2-DIETHYLAMINO ETHYL) 2-METHOXY 3-BROMO 4-[(1-H 4,5-DIHYDRO 2-IMIDAZOLYL) AMINO]5-CHLORO BENZAMIDE

Stade I : 2-METHOXY 3-BROMO 4-[(1-H 4,5-DIHYDRO 2-IMIDAZOLYL) AMINO]5-CHLORO BENZOATE DE METHYLE

Dans un erlenmeyer de 500 ml, on charge 56,7g (0,2 mole) de 2-méthoxy 4- [(1-H 4,5-dihydro 2-imidazolyl) amino]5-chloro benzoate de méthyle, 53,5 g (0,3 mole) de N-bromo succiminide, et 100 ml d'acide acétique. Il se produit un échauffement. On agite deux heures à 55-60°C. La dissolution se fait en 15 minutes environ. La fiole est laissée au repos 70 heures dans une étuve à 55-60°, refroidie et versée dans un litre d'eau sous agitation. L'insoluble est essoré et lavé et le filtrat neutralisé à pH 6 par la soude, en refroidissant.
On ajoute alors 40 g de carbonate de sodium par fractions jusqu'à pH 9-10. On agite 1/2 heure, essore lave et sèche le précipité. On obtient 60,5 g (rendement = 83,45 %) de cristaux fondant à 197-198°C.
Spectre R M N : compatible avec la structure attendue.
Br = 21,90 % (calculé = 22,07 %)

Stade II : N-(2-DIETHYLAMINO ETHYL) 2-METHOXY 3-BROMO 4-[(1-H, 4,5-DIHYDRO 2-IMIDAZOLYL) AMINO]5-CHLORO BENZAMIDE

Dans un tricol de 250 ml équipé d'une agitation, d'un thermomètre et d'un réfrigérant à reflux, on charge 54,38 g (0,15 mole) de 2-méthoxy 3-bromo 4-[(1-H 4,5-dihydro 2-imidazolyl) amino]5-chloro benzoate de méthyle, 34,8 g (0,3 mole) de diéthylamino éthylamine et 100 ml de glycol éthylènique. On agite 72 heures à 70° puis on verse dans 400 ml d'eau. On extrait par 3 x 200 ml de chlorure de méthylène, puis encore par 2 x 100 ml de ce solvant.
Le solvant est chassé sous vide, et le résidu est agité dans 100 ml d'éther isopropylique, essoré, lavé par 50 ml d'éther et séché. On obtient 33,8 g de produit (rendement = 50 %) fondant à 164°C (titre = 100,4 %) Le spectre R M N est compatible avec la structure attendue. On purifie néanmoins le produit.
33 g de produit sont dissous dans 15 volumes d'acétate d'éthyle, traités au noir, filtrés et cristallisés. Le produit est essoré, les eaux mères concentrées aux 3/4, cristallisées à froid et essorées. On obtient 25 grammes de produit coloré.
Ces 25 grammes sont dissous dans trois volumes d'isopropanol, traités au noir,filtrés, cristallisés à 0°, essorés, lavés et séchés.
On a 20 g. de produit légèrement crème qui sont à nouveau traités au noir et cristallisés dans trois volumes d'isopropanol.
On obtient : 16,3 g. (rendement de purification = 49,4 %)
PF = 179°C , titre = 99,5 % , spectre R M N = compatible

Exemple XII : N-[(1-BENZYL 2-PYRROLIDINYL) METHYL] 2-METHOXY 4-[(1-H 4,5-DIHYDRO 2-IMIDAZOLYL) AMINO]5-CHLORO BENZAMIDE

Dans un ballon de 250 ml muni d'un agitateur, d'un thermomètre, d'un réfrigérant, on introduit 28,35 g (0,10 mole) de 2-méthoxy 4-[(1-H 4,5-dihydro 2-imidazolyl) amino]5-chloro benzoate de méthyle, 60 ml de méthanol, et 38 g (0,20 mole) de 1-benzyl 2-aminométhyl pyrrolidine.
On chauffe le mélange à reflux environ 100 heures, refroidit dans un bain de glace et essore les cristaux formés. On les lave trois fois avec 30 ml de méthanol et on les sèche à l'étuve une nuit à 50°C. Rendement = 36,4 g (82,5 %) PF = 195°
Le spectre RMN révèle la présence d'environ 30 à 35 % molaire d'ester de départ.
On reprend les cristaux dans 288 ml d'éthanol absolu, on ajoute 38 ml de lessive de soude et on chauffe deux heures à reflux.
On ajoute 750 ml d'eau sur la suspension et essore les cristaux.
On les lave six fois avec 50 ml d'eau et sèche une nuit à l'étuve à 50°C. Rendement = 23 g (52 %) PF = 210°C

Exemple XIII : N-[(1-ETHYL 2-PYRROLIDINYL) METHYL] 2-METHOXY 4-[(1-H 4,5-DIHYDRO 2-IMIDAZOLYL) AMINO]5-CHLORO BENZAMIDE

Dans un tricol de 250 ml muni d'une agitation et d'un thermomètre on charge 28,35 g (0,1 mole) de 2-méthoxy 4-[(1-H, 4,5-dihydro 2-imidazolyl) amino]5-chloro benzoate de méthyle,28 ml d'éthylène glycol,14,08 g (0,11 mole) de 1-éthyl 2-aminométhyl pyrrolidine. On maintient 48 h sous agitation à 70-75°, refroidit, ajoute 50 ml d'eau, essore à 10°lave à l'eau et sèche. On obtient : 31,3 g. de produit - PF 200° (rendement :82,5 %)
29,6 g de ce produit sont dissous dans 100 ml d'eau et 20 ml d'acide chlorhydrique concentré, traités par 2,9 g de noir lavé à l'acide chlorhydrique, filtrés et précipités par 25 ml de lessive de soude. La gomme

est empâtée dans 150 ml d'acétone, agitée, essorée, lavée à l'acétone et séchée.

On obtient : 24,7 g. de produit (rendement : 83,4 %)

P F : 206° R M N : Contient 25 % d'ester de départ

23,8 g de ce produit sont chauffés au reflux dans 200 ml d'eau et 9 ml de lessive de soude, filtrés à 60°, lavés à l'eau et séchés.

On obtient : 18,9 g. (rendement : 79,4 %)

P F : 222°C R M N : Compatible,(ester non décelé.)

PURIFICATION

18 g. sont dissous à froid dans 100 ml d'eau et 12 ml d'acide chlorhydrique, agités une heure avec du noir végétal, filtrés, précipités par la lessive de soude diluée, essorés, lavés à l'acétone et séchés.
Les 16,4 g. obtenus sont dissous à chaud dans 400 ml de dichloroéthane traités au noir acticarbone 3 S, filtrés, cristallisés à froid, essorés lavés au dichloroéthane et séchés à 50°, puis une nuit à 100°.

On obtient : 13 g. (rendement de purification : 72,3 %)

P F (KOFLER) = 222°C

Titre = 98,5 %

C1 = 9,39 % (calculé : 9,33 %)

N = 18,62 % (calculé :18,44 %)

Les spectres RMN ET IR sont compatibles.

Exemple XIV : N-[(1-CYCLOPROPYLMETHYL 2-PYRROLIDINYL) METHYL] 2-METHOXY 4-[(1-H 4,5-DIHYDRO 2-IMIDAZOLYL) AMINO]5-CHLORO BENZAMIDE

Dans un tricol de 250 ml muni d'une agitation et d'un thermomètre on charge 28,35 g (0,1 mole) de 2-méthoxy 4-[(1-H, 4,5-dihydro 2-imidazolyl) amino]5-chloro benzoate de méthyle, 28 ml d'éthylène glycol, et 30,8 g (0,2 mole) de 1-cyclopropylméthyl 2-aminométhyl pyrrolidine. On maintient sous agitation 72 heures à 70-75°C. On refroidit et on ajoute 150 ml d'eau, essore, lave à l'eau et sèche.

On obtient : 33,1 g (rendement : 81,6 %) PF : 201°C

Le produit est dissous à chaud dans 150 ml de propanol, on ajoute deux spatules de noir, filtre et cristallise à froid.

On essore, lave avec du propanol et sèche.

On obtient : 27,4 g. (rendement de cristallisation : 82,8 %)

Spectre RMN et IR : Compatibles

P F : 198°C

Titre : 99,8 %

Perte à 100° : 0,16 %

C1 : Calculé : 8,73 % trouvé : 8,63 %

N : Calculé :17,25 % trouvé :17,56 %

Exemple XV : N-[(1-ALLYL 2-PYRROLIDINYL) METHYL] 2-METHOXY 4-[(1-H,4,5-DIHYDRO 2-IMIDAZOLYL) AMINO]5-CHLORO BENZAMIDE

Dans un tricol de 250 ml muni d'une agitation et d'un thermomètre, on charge 28,35 g (0,1 mole) de 2-méthoxy 4-[(1-H, 4,5-dihydro 2-imidazolyl)amino]5-chloro benzoate de méthyle, 28 ml d'éthylène glycol et 28 g (0,2 mole) de 1-allyl 2-aminométhyl pyrrolidine. On maintient 72 heures sous agitation à 70-75°C.
On refroidit, ajoute 150 ml d'eau, essore à 10° lave à l'eau puis à l'acétone (100 ml) et sèche. On obtient 31,3 g. (PF 207°C).
Le précipité est remis en suspension dans 200 ml d'eau et 2 ml de soude.
On chauffe 15 minutes à 60-70°, filtre à chaud, lave à l'eau et sèche.
On obtient : 31, 2 g. (rendement 79,7 %) - PF 207°C.
31 g sont dissous à chaud dans 200 ml de propanol, traités au noir acti carbone 3 S, filtrés, cristallisés à froid, essorés, lavés au propanol et séchés.

On obtient : 26,7 g. (rendement 86,1 %)

Analyse : Perte de poids à 100° : 0,17 %

P F (KOFLER) : 208°C

C1 : Trouvé : 8,89 %

Corrigé : 8,90 %

Calculé : 9,04 %

N : Trouvé : 18,04 %

Corrigé : 18,07 %

Calculé : 17,87 %

Titre : Trouvé : 99,90 %

Corrigé : 100,1 %

Spectres RMN et IR : Compatibles.

Exemple XVI : N-[(1-METHYL 2-PYRROLIDINYL) METHYL] 2-METHOXY 4-[(1-H, 4,5-DIHYDRO 2-IMIDAZOLYL) AMINO]5-CHLORO BENZAMIDE

Dans un tricol de 250 ml muni d'une agitation et d'un thermomètre, on charge 28,35 g (0,1 mole) de 2-méthoxy 4-[(1-H 4,5-dihydro 2-imidazolyl) amino]5-chloro benzoate de méthyle, 28 ml d'éthylène glycol et 22,4 g (0,2 mole) de 1-méthyl 2-aminométhyl pyrrolidine. On agite pendant 72 heures à 70-75°C (ester non décelé en RMN)
On ajoute 150 ml d'eau, refroidit, essore à 10°, lave à l'eau puis avec 100 ml d'acétone et sèche à 50°.
On obtient : 32,4 g. (rendement : 88,6 %) - PF : 235°C
32,1 g. sont dissous à chaud dans 400 ml de propanol, traités au noir, filtrés, cristallisés à froid, essorés, lavés au propanol et séchés. On obtient 27,5 g de cristaux qui contiennent des traces de propanol (rendement : 85,7 %). 25 g. sont dissous dans 250 ml d'eau et 15 ml d'acide chlorhydrique concentré, précipités par 40 ml d'ammoniaque, essorés, lavés à l'eau et séchés à 60°.

On obtient : 21,9 g. de produit (rendement :87,6 %)
Perte de poids à 100° : 0,3 % PF = 236°C (Kofler)
Titre : Trouvé : 98,5 %
Corrigé : 98,8 %
C1 : Trouvé : 9,67 %
Corrigé : 9,70 %
Calculé : 9,69 %
N : Trouvé : 19,29 %
Corrigé : 19,35 %
calculé : 19,14 %

Les spectres RMN et IR sont compatibles avec la structure proposée.

Exemple XVII : N-[(1-PROPYL 2-PYRROLIDINYL) METHYL] 2-METHOXY 4-[(1-H 4,5-DIHYDRO 2-IMIDAZOLYL) AMINO]5-CHLORO BENZAMIDE

En suivant exactement le procédé de l'exemple XV, on fait réagir 28,5 g (0,1 mole) de 2-méthoxy 4-[(1-H 4,5-dihydro 2-imidazolyl) amino]5-chlorobenzoate de méthyle et 28,4 g (0,2 mole) de 1-propyl 2-aminométhyl pyrrolidine, dans 28 ml d'éthylène glycol.

On obtient : 34,2 g. (rendement : 86,9 %) - PF : 207°.

34 g. de produit sont dissous à chaud dans 300 ml de propanol, traités au noir acti carbone 3 S, filtrés, recristallisés à froid, essorés, lavés au propanol et séchés.

On obtient : 29,6 g. de produit (rendement : 87 %)
Perte de poids à 100° : 0,12 %
P F (Kofler) : 207°C
C1 : Trouvé = 8,88 %
Corrigé = 8,89 %
Calculé = 9,00 %
N : Trouvé = 18,01 %
Corrigé = 18,03 %
Calculé = 17,78 %
Titre : Trouvé = 99,7 %
Corrigé = 99,8 %
Spectre RMN et IR: Compatibles avec la structure attendue.

Exemple XVIII : N-(2-DIETHYAMINO ETHYL) 2-METHOXY 4- [(1-H 4,5-DIHYDRO 2-IMIDAZOLYL) AMINO]5-BROMO BENZAMIDE

Dans un tricol de 250 ml muni d'une agitation et d'un thermomètre, on charge 24,6 g (0,075 mole) de 2-méthoxy 4-[(1-H 4,5-dihydro 2-imidazolyl) amino]5-bromo benzoate de méthyle, 40 ml d'éthylène glycol et 17,4 g.(0,15mole) de diéthylamino éthylamine. On agite 72 heures à 70-75°C. On ajoute 80 ml d'eau, refroidit, essore à 10° et lave à l'eau. Le précipité est repris dans 100 ml d'eau avec 0,8 g de soude, agité 15 minutes à 70-80°, filtré chaud, lavé à l'eau puis à l'acétone et séché à 50°.
On obtient 26,4 g de produit (rendement 85,4 %), fondant à 216°C (Titre = 99,5 %, RMN compatible avec la structure attendue)
25,8 g sont dissous dans 390 ml (15 vol.) d'isopropanol, traités au noir acticarbone 3 S, filtrés, cristallisés à froid, essorés, lavés à l'acétone et séchés.

On obtient 23,3 g (rendement de purification 86,7 %)

Analyse : PF° (Kofler) 217°C
Perte de poids à 100°C 0,01 %
Titre 98,8 %
Br 19,61 % (calculé 19,40 %)
N 17,04 % (calculé 16,98 %)
Spectres RMN et IR compatibles.

Exemple XIX : N-[(1-ETHYL 2-PYRROLIDINYL) METHYL] 2-METHOXY 4-[(1-H 4,5-DIHYDRO 2-IMIDAZOLYL) AMINO]5-BROMO BENZAMIDE

En suivant exactement le procédé décrit à l'exemple XVII, on fait réagir 24,6 g (0,075 mole) de 2-méthoxy 4-[(1-H 4,5-dihydro 2-imidazolyl) amino]5-bromo benzoate de méthyle et 19 g (0,15 mole) de 1-éthyl 2-aminométhyl pyrrolidine, dans 40 ml d'éthylène glycol. On obtient 28,72 g (rendement 90,5 %) de cristaux fondant à 235°C. (Titre 98,8 %).
RMN compatible avec la structure attendue.
27,65 g. de produit sont dissous à chaud dans 10 volumes de propanol, traités au noir acti carbon 3 S, filtrés, cristallisés à froid, essorés lavés à l'acétone et séchés.
On obtient : 24 g. (rendement : 87 %)
Analyse : RMN compatible
Perte à 100° 0,33 %
P F (Kofler) 237°C
Br Trouvé : 18,61 % Corrigé : 18,67 % Calculé : 18,84 %
N Trouvé : 16,48 % Corrigé : 16,53 % Calculé : 16,50 %
Titre Trouvé : 98,1 % Corrigé : 98,4 %

Exemple XX : N-[(1-ALLYL 2-PYRROLIDINYL) METHYL] 2-METHOXY 4-[(1-H 4,5-DIHYDRO 2-IMIDAZOLYL) AMINO]5-BROMO BENZAMIDE

En suivant exactement le procédé décrit à l'exmple XVII, on fait réagir 24,6 g (0,075 mole) de 2-méthoxy 4-[(1-H 4,5-dihydro 2-imidazolyl) amino]5-bromobenzoate de méthyle et 21 g (0,15 mole) de 1-allyl 2-amino méthyl pyrrolidine dans 40 ml d'éthylène glycol. On obtient 28,46 g (rendement = 87 %) de produit fondant à 214°C (titre 99,1 %)
RMN : compatible avec la structure attendue.
27,45 g. sont dissous à chaud dans 11 volumes de propanol, traités au noir acti carbone 3 S, filtrés, cristallisés à froid, essorés, lavés au propanol et séchés.
On obtient : 24,85 g. (rendement de purification : 90,5 %)
Analyse
P F (Kofler) = 215°C
Perte de poids (100°) = 0,13 %
Titre Trouvé = 98,6 % - Corrigé = 98,7 %
Br Trouvé = 18,47 % - Corrigé = 18,49 % - Calculé = 18,33 %
N Trouvé = 16,11 % - Corrigé = 16,13 % - Calculé = 16,05 %
Les spectres RMN et IR sont compatibles.

Exemple XXI : N-(2-DIETHYLAMINOETHYL) 2-METHOXY 4- [(1-H 4,5-DIHYDRO 4-METHYL 2-IMIDAZOLYL) AMINO]5-CHLORO BENZAMIDE

Stade I : 2 METHOXY 4-(N' 2-AMINO 1( ou 2)-ISOPROPYL) THIOUREIDO 5-CHLORO BENZOATE DE METHYLE

Dans un tricol d'un litre, muni d'une agitation, d'un thermomètre et d'une ampoule de coulée, on introduit 51 ml (0,6 mole) de 1,2-diaminopropane et 200 ml d'éther isopropylique . Le milieu est refroidi à 0, -5° et on coule en 1/2 heure une solution de 39,9 g (0,12 mole) de 2-méthoxy 4-isothiocyanato 5-chloro benzoate de méthyle dans le toluène. On agite encore 10 minutes et soutire les solvants. On agite avec 850 ml d'eau. La gomme cristallise.
Le précipité est essoré, lavé à l'eau et séché une nuit sous vide.
On obtient : 40,2 g. (Rendement : 60,6 %)
P F = 100-105°
RMN = Compatible avec un mélange 40-60 % des deux isomères.

Stade II : 2-METHOXY 4-[(1-H 4,5-DIHYDRO 4-METHYL 2-IMIDAZOLYL) AMINO]5-CHLORO BENZOATE DE METHYLE

39,9 g (0,12 mole) de 2-méthoxy 4-(N'2-amino 1-(ou2) isopropyl) thioureido 5-chloro benzoate de méthyle sont chargés dans un tricol avec 90 ml d'eau, et chauffés à 70-75°C. En 30 minutes, on coule à cette température une solution de 40,6 g (0,15 mole) d'acétate de plomb dans 160 ml d'eau distillée.
On laisse agiter 3/4 d'heure à 70-75° et refrodit à 20°, essore le sulfure de plomb et le lave.
On prépare une solution de 27,3 g. de carbonate de sodium(0,26 mole) dans 200 ml d'eau et ajoute la moitié de cette solution au filtrat précédent.
Les sels de plomb précipitent, ils sont essorés et lavés.
Le filtrat est alcalinisé avec le restant de la solution de carbonate de sodium. On essore, lave à l'eau et

sèche sous vide à 25° le précipité formé.

On obtient : 26,5 g. (rendement : 66,4 %)

P F : Complète à 140°

Titre : 98,8 %

RMN : Compatible

Stade III : N-(2-DIETHYLAMINOETHYL) 2-METHOXY 4- [(1-H 4,5-DIHYDRO 4-METHYL 2-IMIDAZOLYL) AMINO]5-CHLORO BENZAMIDE

Dans un tricol de 250 ml on charge 29,75 g (0,1 mole) de 2-méthoxy 4-[(1-H 4,5-dihydro 4-méthyl 2-imidazolyl) amino]5-chloro benzoate de méthyle, 30 ml d'éthylène glycol, et 23,2 g (0,2 mole) de diéthylamino éthylamine. On porte 72 heures sous agitation à 70-75°. On ajoute 150 ml d'eau, refroidit à 15°, essore, lave à l'eau et à l'acétone et sèche à 50°.

On obtient 33,4 g. (rendement : 87,5 %)

P F = 179°C

RMN = Ester non décelé - compatible

Le produit est dissous à chaud dans 200 ml d'éthanol, traité au noir, filtré, recristallisé à froid, essoré, lavé par de l'éthanol, séché à 60°.

On obtient 23,5 g. (rendement de purification : 70,4 %)

P F (Kofler) = 185°C

Perte de poids (100°) = 0,09 %

Titre = 99,5 %

C1 = 9,19 % (calculé : 9,28 %)

N = 18,37 % (calculé : 18,34 %)

Les spectres RMN et IR sont compatibles avec la structure proposée.

Exemple XXII N-[(1-ETHYL 2-PYRROLIDINYL) METHYL] 2-METHOXY 4-[(1-H 4,5-DIHYDRO 4-METHYL 2-IMIDAZOLYL) AMINO]5-CHLORO BENZAMIDE

Dans un tricol de 250 ml on charge 29,75 g (0,1 mole) de 2-méthoxy 4- [(1-H 4,5-dihydro 4-méthyl 2-imidazolyl) amino]5-chloro benzoate de méthyle, 30 ml d'éthylène glycol et 25,6 g (0,2mole) de 1-éthyl 2-aminométhyl pyrrolidine. On porte 72 heures à 70-75°C, sous agitation, on ajoute 150 ml d'eau , refroidit, essore, lave à l'eau puis à l'acétone (légère solubilité du produit dans l'acétone) et sèche à 50°.

On obtient 24,6 g (Rendement = 62,5 %), du produit fondant à 180°C.

RMN : compatible avec la structure attendue.

Le produit est solubilisé à chaud dans 150 ml d'isopropanol, traité au noir, filtré à chaud, cristallisé à froid, essoré, lavé et séché à 70°. On obtient 13,2 g de cristaux.

PF (Kofler) 165°C, recristallise.

Fond à nouveau à 180°C.

Perte à 100° 0,43 %

| | Trouvé | Corrigé | Calculé |
|---|---|---|---|
| Titre % | 98,5 | 98,9 | |
| N % | 17,49 | 17,56 | 17,78 |
| C1 % | 8,97 | 9,01 | 9,00 |

Les spectres RMN et IR sont compatibles avec la structure proposée.

Exemple XXIII : N-[(1-ALLYL 2-PYRROLIDINYL) METHYL] 2-METHOXY 4-[(1-H 4,5-DIHYDRO 4-METHYL 2-IMIDAZOLYL) AMINO]5-CHLORO BENZAMIDE

Dans un tricol de 250 ml, on charge 29,75 g (0,1 mole) de 2-méthoxy 4-[(1-H 4,5-dihydro 4-méthyl 2-imidazolyl) amino]5-chloro benzoate de méthyle, 30 ml d'éthylène glycol et 28 g (0,2 mole) de 1-allyl 2-amino méthyl pyrrolidine. On agite à 70-75°C pendant 72 heures et on ajoute 100 ml d'eau. On refroidit à 15°C, on essore, et on lave à l'eau, puis à l'acétone, et on sèche à 50°. On obtient 32,6 g de produit (rendement : 80,4 %)

Le produit est dissous à chaud dans 200 ml d'éthanol absolu, traité au noir acti carbone 3 S, filtré, recristallisé à froid, essoré, lavé à l'éthanol et séché à 60°

On obtient : 21,3 g. (rendement de purification : 65,3 %)

P F (Kofler) : 170°
Perte de poids à 100° : 0, 1 %
Titre : 100,7 %
C1 : 8,52 % (calculé : 8,73 %)
N : 17,48 % (calculé :17,25 %)
Spectres RMN et IR compatibles avec la structure proposée.

Exemple XXIV : N-(2-DIETHYLAMINOETHYL) 2-METHOXY 4- [(1-H 4,5-DIHYDRO 4-METHYL 2-IMIDAZOLYL) AMINO]5-BROMO BENZAMIDE

Stade I : 2-METHOXY 4-(N'2-AMINO 1-(ou 2) ISOPROPYL) THIOUREIDO 5-BROMO BENZOATE DE METHYLE

Dans un tricol de deux litres équipé d'une agitation, d'un thermomètre et d'une ampoule de coulée, on charge 51ml (0,6 mole) de 1-2 diaminopropane et 200 ml d'éther isopropylique. Le milieu est refroidi à 0 °, -5°, et on introduit goutte à goutte en 1 heure, 60,4 g (0,2 mole) d'une solution de 2-méthoxy 4-isothiocyanato 5-bromo benzoate de méthyle dans 350 ml de toluène.
En fin d'introduction, à 0°, -5°, la thiourée adhère aux parois. Le solvant est soutiré, on ajoute 500 ml d'eau et agite 15 minutes. Le produit cristallise. Il est essoré, lavé à l'eau et séché en une nuit à 30° sous vide.
On obtient : 58,8 g. (rendement = 78,2 %)
P F : 117°
Titre : 101,9 %
RMN : Compatible avec un mélange des deux isomères (60 % - 40 %)

Stade II : 2-METHOXY 4-[(1-H 4,5-DIHYDRO 4-METHYL 2-IMIDAZOLYL) AMINO]5-BROMO BENZOATE DE METHYLE

Dans un tricol de deux litres muni d'une agitation, d'un thermomètre et d'un réfrigérant, on charge 310,6 g(0,826 mole) de 2-méthoxy 4-(N'2-amino 1-(ou 2) isopropyl) thiouréido 5-bromo benzoate de méthyle et 1,250 litres de chlorobenzène. On chauffe cinq heures à 100° sous agitation, puis refroidit à 15°, essore et lave le précipité à l'éther isopropylique et sèche à 70°.
On obtient : 220,4 g.
P F : pas net à partir de 145°
219 g. de produit sont dissous à reflux dans 900 ml d'une solution d'isopropanol à 40 % dans l'eau, traités au noir acti carbone 3 S, filtrés et cristallisés dans la place. On filtre, lave avec une solution glacée d'isopropanol dans l'eau et sèche à 70°.
On obtient : 169,5 g. (rendement de purification 77,4 % rendement global : 60,4 %)
P F : 168 °
Titre : 98,2 %
RMN : Compatible, il reste des traces de chlorobenzène et d'isopropanol

Stade III : N-(2-DIETHYLAMINO ETHYL) 2-METHOXY 4-[(1-H 4,5-DIHYDRO 4-METHYL 2-IMIDAZOLYL) AMINO]5-BROMO BENZAMIDE

Dans un tricol de 250 ml, on charge 34,2 g (0,1 mole) de 2-méthoxy 4- [(1-H 4,5-dihydro 4-méthyl 2-imidazolyl) amino] 5-bromo benzoate de méthyle, 60 ml d'éthylène glycol, et 23,2 g (0,2 mole) de diéthylamino éthylamine. On porte à 70-75° pendant 72 heures sous agitation. On ajoute 100 ml d'eau et refroidit à 10°, essore, lave à l'eau, puis à l'éther de pétrole et sèche.
On obtient : 40,4 g. (rendement = 95 %)
P F : 203 °C
Titre 98,5 %
RMN : Compatible avec la structure attendue.
39,7 g. de produit sont dissous à chaud dans 6 volumes (240 ml) d'éthanol absolu, traités au noir, filtrés, cristallisés à froid, essorés, lavés à l'éther isopropylique et séchés à 60°.
On obtient : 34,6 g. (rendement de purification : 87,3 %)
P F Kofler : 198°C
Perte à 100° : 0,04 %
Titre : 98,9 %
N : 16,31 % (calculé : 16,43 %)
Br : 19,17 % (calculé : 18,76 %)
Les spectres RMN et IR sont compatibles avec la structure proposée.

Exemple XXV : N [(1-ETHYL 2-PYRROLIDINYL) METHYL] 2-METHOXY 4-[(1-H 4,5- DIHYDRO 4-METHYL 2-IMIDAZOLYL) AMINO]5-BROMO BENZAMIDE

Dans un tricol de 250 ml, on charge 34,2 g (0,1 mole) de 2-méthoxy 4-[(1-H 4,5-dihydro 4-méthyl 2-imidazolyl) amino]5-bromo benzoate de méthyle, 60 ml d'éthylène glycol et 25,6 g (0,2 mole) de 1-éthyl 2-amino méthyl pyrrolidine et maintient sous agitation 72 h. à 70-75°C. On ajoute 100 ml d'eau, on refroidit à 10°, essore, lave à l'eau puis à l'acétone et sèche.
On obtient : 37,3 g. (rendement : 85,4 %)
P F : 200° (des particules fondent 190°)
Titre : 98 %
R M N : Compatible avec la structure attendue

36,6 g.de produit sont dissous à chaud dans 270 cc d'éthanol absolu, traités au noir, filtrés, cristallisés à froid, essorés, lavés à l'éther isopropylique et séchés.

On obtient : 25,6 g (rendement de purification : 70 %)
P F Kofler : Vers 170°,recristallise et fond à nouveau à 196°C
Perte à 100° : 0,56 %
Titre - Trouvé : 98,6 % - Corrigé : 99,2 %
N Trouvé : 15,70% - Corrigé : 15,79 % - Calculé : 15,98 %
Br Trouvé : 18,01 - Corrigé : 18,11 % - Calculé : 18,25 %

Les spectres RMN et IR sont compatibles avec la structure proposée.

Exemple XXVI : N-[(1-ALLYL 2-PYRROLIDINYL) METHYL] 2-METHOXY 4-[(1-H 4,5-DIHYDRO 4-METHYL 2-IMIDAZOLYL) AMINO]5-BROMO BENZAMIDE

Suivant le procédé de l'exemple 25, on fait réagir 34,2 g (0,1 mole) de 2-méthoxy 4-[(1-H 4,5-dihydro 4-méthyl 2-imidazolyl) amino]5-bromo benzoate de méthyle avec 28 g (0,2 mole) de 1-allyl 2-aminométhyl pyrrolidine dans 60 ml d'éthylène glycol.
On obtient : 40,17 g. (rendement : 89,4 %)
P F : 175°
Titre : 97 %
R M N : Compatible avec la structure attendue

40 g. sont dissous à chaud dans 200 ml d'éthanol, traités au noir acti carbone 3 S, filtrés, cristallisés à froid, essorés, lavés à l'éther isopropylique et séchés.
On obtient : 31,37 g. (rendement de purification : 78,5 %)
P F (Kofler) ; 174°C
Perte à 100° : 0,09 %
Titre : 99,1 %
N : 15,35 % (calculé : 15,55 %)
Br : 18,13 % (calculé : 17,76 %)

Les spectres RMN et IR sont compatibles avec la structure proposée.

Exemple XXVII : N-(2-DIETHYLAMINO ETHYL) 2-METHOXY 4-[(1-H 4,5-DIHYDRO 4-DIMETHYL 2-IMIDAZOLYL) AMINO]5-CHLORO BENZAMIDE

Stade I : 2-METHOXY 4-[N'-(2-METHYL 2-AMINO PROPYL) THIOUREIDO]5-CHLORO BENZOATE DE METHYLE

Dans un tricol de 2 litres muni d'une agitation, d'un thermomètre et d'une ampoule de coulée, on charge 400 ml d'éther isopropylique et 83,6 ml (0,8 mole) de 1,2-diamino 2-méthyl propane.
On refrodit à 0,-5°C et coule en 45 minutes uns solution de 103 g (0,4 mole) de 2-méthoxy 4-isothiocyanato 5-chloro benzoate de méthyle dans 600 ml de toluène, en se maintenant à cette température. On agite encore 15 minutes et soutire les solvants. Le produit gommeux, collé aux parois est cristallisé en agitant dans un litre d'eau.
On laisse reposer une nuit, essore, lave à l'eau et sèche.

On obtient 117,7 g (rendement 85,2 %)
PF° 149-150°
RMN compatible (On ne distingue pas d'isomères).

Stade II : 2-METHOXY 4-[(1-H, 4,5-DIHYDRO 4-DIMETHYL 2-IMIDAZOLYL) amino]-CHLORO BENZOATE DE METHYLE

Dans un tricol de 2 litres, on charge 117,7 g (0,34 mole) de 2-méthoxy 4-[N'-(2-amino 2-méthyl propyl) thioureido]5-chloro benzoate de méthyle dans 470 ml de chlorobenzène et chauffe à 100° sous agitation, pendant 5 heures. La dissolution est complète vers 85-90°. On refroidit à 20° et ajoute 470 ml d'eau et 50

ml d'acide acétique.

On décante, lave la phase organique par 250 ml d'eau et alcalinise les fractions aqueuses par 106 g de carbonate de sodium (pH 10). Il se forme une pâte qui cristallise lentement.

On essore, lave à l'eau et sèche le produit. On obtient 90,1 g (rendement = 85,1 %) de cristaux fondant entre 130 et 180°C. 109,4 g. de ce produit sont dissous dans 440 ml d'éthanol à chaud, traités au noir, filtrés, cristallisés à froid, essorés, lavés à l'éthanol et séchés.

On obtient 88 g. de produit (rendement = 80,4 %)

PF° = 167°C

Titre = 94,3 %

RMN compatible (présence de solvants).

Stade III : N-(2-DIETHYLAMINO ETHYL) 2-METHOXY 4-[(1-H 4,5-DIHYDRO 4-DIMETHYL 2-IMIDAZOLYL) AMINO]5-CHLORO BENZAMIDE

Dans un tricol de 250 ml muni d'une agitation et d'un thermomètre, on charge 31,15 g (0,1 mole) de 2-méthoxy 4-[(1-H 4,5-dihydro 4-diméthyl 2-imidazolyl) amino]5-chloro benzoate de méthyle, 35 ml d'éthylène glycol, et 23,2 g (0,2 mole) de diéthylamino éthylamine. On agite 80 h. à 70-75°C. On ajoute alors 150 ml d'eau, on refroidit, essore, lave à l'eau puis à l'acétone, et sèche à 50°C.

On obtient 33,4 g (Rendement = 84,45 %) PF° = 194°C

33,4 g sont dissous à chaud dans 200 ml d'une solution isopropanol- eau (50-50), traités au noir acticarbone 3 S, filtrés, cristallisés à froid, essorés, lavés par une solution isopropanol-eau (50-50), et séchés une nuit à 60°, puis une nuit à 70°.

On obtient 26 g de produit (rendement de purification 77,8 %)

PF Kofler 189°C

Perte à 100° 0,06 %

Titre 98,1 %

C1 9,02 % calculé 8,95 %

N 17,67 % calculé 17,69 %

Les spectres RMN et IR sont compatibles.

Exemple XXVIII : N-[(1-ALLYL 2-PYRROLIDINYL) METHYL] 2-METHOXY 4-[1-H 4,5-DIHYDRO 4-DIMETHYL 2-IMIDAZOLYL) AMINO]5-CHLORO BENZAMIDE

Dans un tricol de 250 ml muni d'une agitation, d'un thermomètre et d'un réfrigérant, on charge 31,15 g (0,1 mole) de 2-méthoxy 4-[(1-H 4,5-dihydro 4-diméthyl 2-imidazolyl) amino]5-chloro benzoate de méthyle, 35 ml d'éthylène glycol, et 28 g (0,2 mole) de 1-allyl 2-aminométhyl pyrrolidine, et on agite pendant 80 heures à 70-75°. (Il n'est plus décelé d'ester en RMN). On ajoute 150 ml d'eau, refroidit à 5°, essore, lave à l'eau puis à l'acétone , et sèche.

On obtient 26,7 g (rendement 63,6 %) ( PF° = 160°)

26,7 g de produit sont dissous à chaud dans 265 ml d'acétate d'éthyle, traités au noir acticarbone 3 S, filtrés, cristallisés à froid, essorés, lavés à l'acétate d'éthyle et séchés à 50°.

On obtient 18,9 g (Rendement de purification = 70,8 %)

PF Kofler 161°C

Perte à 100° 0 %

Titre 98,4%

Cl 8,41% (calculé 8,44%)

N 16,45% (calculé 16,68%)

Les spectres RMN et IR sont compatibles.

Exemple XXIX: N-(2-DIETHYL AMINO ETHYL) 2-METHOXY 4-[1H-4,5-DIHYDRO 2-IMIDAZOLYL) AMINO] 5-CHLORO BENZAMIDE

Stade I: 2-CHLORO 4-(DIETHYLAMINO 2-ETHYLAMINO CARBONYL) 5-METHOXY PHENYL DITHIOCARBAMATE DE SODIUM

Dans un tricol de 6 litres muni d'une agitation, d'un thermomètre, d'un réfrigérant à reflux et d'une ampoule d'introduction, on charge 600 g de N(diéthylaminoéthyl) 2-méthoxy-4-amino 5-chloro benzamide dans 2,4 l de tétrahydrofuranne, et introduit par fractions, en 20 minutes, 144 g d'hydrure de sodium à 50%, vers 25–30°C.

On introduit en mince filet (20 minutes) sous bonne agitation 400 ml de sulfure de carbone.

On chauffe lentement jusqu'à 50° et maintient un léger reflux pendant 30 minutes, puis on arrête l'agitation et le chauffage et laisse reposer une nuit. On essore et lave par deux fois avec 1,5 l. de chloroforme, sèche à l'air puis à l'étuve à 50°.

On obtient 886 g (rendement = 111%)

RMN compatible, impureté en faible teneur, pas de benzamide de départ.

Stade II: N-(2-DIETHYLAMINOETHYL) 2-METHOXY 4-ISOTHIOCYANATO 5-CHLORO BENZAMIDE

Dans un tricol de 1 litre muni d'une agitation, d'un thermomètre, d'un réfrigérant et d'une ampoule d'introduction, on charge 159 g de 2-chloro 4-(diéthylamino 2-éthylamino-carbonyl)-5-méthoxy phényl dithiocarbamate de sodium dans 470 ml d'éther éthylique.

On refroidit et coule en 30 mn, entre 20 et 22°, 43,4 g de chloroformiate d'éthyle. Il se produit une modification de l'aspect physique des cristaux en suspension. On laisse 2 heures à 20°C sous agitation.

On essore et reprend le précipité en suspension dans 150 ml d'eau, essore à nouveau, lave à l'eau et sèche à l'étuve à 50°C.

On obtient 87 g . de produit (P F 100 à 150°C)

Rendement = 63,7 %

RMN compatible, il n'est pas décelé de N-(diéthylamino éthyl) 2-méthoxy 4-amino 5-chlorobenzamide base.

Stade III : N-(2-DIETHYLAMINO ETHYL) 2-METHOXY 4-[1-4,5-DIHYDRO 2-IMIDAZOLYL) AMINO]5-CHLORO BENZAMIDE.

Dans un tricol de 2 litres muni d'une agitation, d'un thermomètre , d'un réfrigérant à reflux et d'une ampoule d'introduction, on charge 105 g de N-(diéthylaminoéthyl) 2-méthoxy 4-isothiocyanato- 5-chloro benzamide dans 1 litre de xylène. On coule en mince filet, en refroidissant pour maintenir la température < 25°, 30 g d'éthylène diamine (durée 15 minutes).

Il se produit un fort épaissement du milieu.

On laisse agiter 2 heures à température ambiante.

On porte à reflux et maintient celui-ci 10 heures. (La température monte jusqu'à 125-128°C.) On filtre l'insoluble à chaud, et refroidit le filtrat à 10°, essore le précipité et le lave à l'eau (environ 400 cc). On sèche à l'étuve à 50°.

On obtient 75 g. d'un produit contenant environ 30 à 35 % de N-(diéthylamino éthyl) 2-méthoxy 4-amino 5-chloro benzamide base.

P F pâteux à partir de 140°C.

Ce produit est agité 1 heure dans 75 ml de chloroforme, essoré et séché. On obtient 55 g d'un produit( P F = 185°) contenant encore 5 à 10 % de N-(diéthylaminoéthyl) 2-méthoxy 4-amino 5-chloro benzamide base.

Ce produit recristallisé dans 400 ml d'éthanol, après filtration à chaud d'un insoluble donne 36 g de produit.

(P F 197°C - RMN : Compatible - Pas d'impureté décelée).

En concentrant les filtrats à 50 ml, on obtient un deuxième jet (P F 192°C) de 8 g. de faible teneur en impuretés.

On a un rendement global de 40 %.

Analyses : Titre % 98,6

C1 % 8,83 %(théorie 9,64)

$H_2O$ % > 0,1

Exemple XXX : N-[(1-CYCLOPENTYL 2-PYRROLIDINYL) METHYL] 2-METHOXY 4-[(1-H, 4,5-DIHYDRO 2-IMIDAZOLYL) AMINO]5-CHLORO BENZAMIDE

Dans un tricol de 250 ml équipé d'une agitation, d'un thermomètre et d'un réfrigérant, on charge 28,35 g (0,1mole) de 2-méthoxy 4-[(1-H, 4-5 dihydro 2- imidazolyl) amino]5 chloro benzoate de méthyle, 60 ml de glycol éthylènique, et 33,6 g (0,2mole) de 1- cyclopentyl 2- aminométhyl pyrrolidine. On porte 72 heures à 75°C sous agitation.

Le milieu est refroidi; on ajoute 100 ml d'eau, agite 15 minutes, essore, lave quatre fois avec de l'acétone et sèche.

On obtient : 33,7 g. (rendement : 80,3 %)

P F : 166°

RMN : Compatible (il n'est pas décelé d'ester)

33 g du produit sont recristallisés dans 250 ml d'isopropanol et traités au noir. Le précipité est essoré, lavé à l'isopropanol et séché. On obtient 26 g de produit.

La solution du chlorhydrate à 10 % dans l'eau est colorée. 23,8 g du produit sont repris dans 238 ml d'eau et 6,5 ml d'acide acétique, traités au noir, filtrés, précipités par 20 ml d'ammoniaque, essorés, lavés à l'eau et séchés à 70° sous vide sur anhydride phosphorique.

On obtient : 20,5 g de produit ( rendement de purification = 67,8 %) fondant à 206° (Spectres RMN et IR compatibles avec la structure attendue).

Titre : 98,2 % - Corrigé : 99,8 %

C1 : 8,26 % - Corrigé : 8,39 % - Calculé : 8,44 %

$H_2O$ : 1,6 %

Exemple XXXI : N-[(1-(2-CYCLOHEXENYL) 2-PYRROLIDINYL) METHYL]2-METHOXY 4-[(1-H 4,5-DIHYDRO 2-IMIDAZOLYL) AMINO]5-CHLORO BENZAMIDE

Dans un ballon de 250 ml muni d'un agitateur, d'un thermomètre, d'un réfrigérant, on a introduit 36,8 g (0,130 mole) de 2-méthoxy 4[(1-H 4,5-dihydro 2-imidazolyl)amino]5-chlorobenzoate de méthyle, 73 ml de méthanol, 46,8 g (0,260 mole) de 1-(2-cyclohéxèn-1-yl) 2-aminométhyl pyrrolidine et 3 gouttes d'acide chlorhydrique( d = 1,18). On chauffe la suspension à reflux 70 heures. L'ester se solubilise lentement. On refroidit à 20°C, filtre un léger insoluble, évapore le filtrat à sec sous vide et ajoute 200 ml d'eau sur le résidu pâteux. Le produit cristallise. On l'essore, lave à l'eau abondamment (6fois 80 ml d'eau) et sèche à l'étuve à 50°C, deux jours. Dans la filtrat on constate la présence d'une huile insoluble.
Rendement = 45,6 g. (81 %)
P F = 186 °C

Le produit est recristallisé dans 250 ml d'éthanol à 95%. On l'essore, lave deux fois avec un peu d'alcool, puis sèche une nuit àl'étuve à 50°C. (Léger jaunissement en surface).
rendement = 35,4 g. (63 %)
P F = 204°C

Les spectres RMN et IR sont compatibles avec la structure du produit attendu.

Exemple XXXII : N (2-PYRROLIDINYL METHYL) 2-METHOXY 4-[(1-H 4,5-DIHYDRO 2-IMIDAZOLYL) AMINO]5-CHLORO BENZAMIDE

Dans un tricol muni d'une agitation, d'un thermomètre et d'un réfrigérant à reflux, on charge 141 g (0,5 mole) de 2-méthoxy 4-[(1-H 4,5-dihydro 2-imidazolyl) amino]5-chloro benzoate de méthyle, 250 ml de glycol éthylènique et 100 g (1 mole) de 2-aminométhyl pyrrolidine . On porte sous agitation à 70°C pendant 72 heures.
Le milieu est refroidi et versé dans 1,5 l d'eau glacée.

La suspension est agitée 1 h, essorée, lavée à l'eau et séchée.

On obtient : 107,2 g de produit
Rendement : 60,9 %
PF : 195°
RMN compatible mais impur.

Le filtrat est extrait par 2x500 ml de chlorure de méthylène, le solvant chassé à sec sous vide, et le résidu repris par 100 ml d'eau. La suspension est agitée 2 heures, essorée, lavée et séchée.

On obtient : 8,5 g (4,85 %)
PF : 188-190°
RMN compatible mais impur.

120 g de produit sont recristallisés dans 360 ml de propanol, traités au noir, glacés, essorés, lavés par 50 ml d'acétone et séchés.
On obtient 90 g
Le filtrat concentré donne un deuxième jet de 20 g.

Ces 110 g sont mis en suspension dans 0,5 l d'eau et acidifiés par l'acide chlorhydrique jusqu'à dissolution, traités au noir, filtrés, alcalinisés par la soude à 10 %, essorés, lavés et séchés.

On obtient 55 g.
RMN compatible

30,8 g de produit sont repris dans 118 ml d'eau distillée et 189,13 ml d'acide chlorhydrique.
La solution traitée au noir est filtrée et alcalinisée par une solution de 21 ml de soude 10 N et 20 ml d'eau. La suspension est agitée 15 minutes, et le précipité essoré, lavé abondamment à l'eau et séché à 60° sous vide sur $P_2O_5$,4jours.

On obtient 25,5 g

Analyse PF 206°
$H_2O$ 1,3 %
Titre (corrigé): 100,9 %
C1 (corrigé) : 9,92 % (calculé 10,07 %)
Spectres RMN et IR compatibles avec la structure proposée.

Exemple XXXIII : N-(2-DIETHYLAMINO ETHYL) 2-ALLYLOXY 4-[(1-H 4,5 DIHYDRO 2-IMIDAZOLYL) AMINO]5-CHLORO BENZAMIDE.

Stade I : 2-HYDROXY 4-ACETAMINO 5-CHLORO BENZOATE DE METHYLE.

Dans un ballon de quatre litres muni d'un agitateur, d'un thermomètre, et d'un réfrigérant on introduit 430 g de 2-hydroxy 4-acétamino benzoate de méthyle, 2150 g d'acide acétique et chauffe vers 35-40°C avant d'introduire 278 g de N-chloro succinimide. On maintient la température de 40°C jusqu'à dissolution totale puis on porte à 50°C pendant 23 heures.

Des cristaux apparaissent. On refroidit à 20°C et verse l'ensemble du mélange réactionnel dans un réacteur de 20 litres contenant 2 kg de glace.

Le précipité formé est essoré, lavé à l'eau et séché à l'étuve à 50°C.
Rendement = 483,5 g (96 %)
P F = 150-154°C

Stade II : 2-ALLYLOXY 4-ACETAMINO 5-CHLORO BENZOATE DE METHYLE.

Dans un ballon de quatre litres muni d'un agitateur, d'un thermomètre et d'un réfrigérant, on introduit 428 g de 2-hydroxy 4-acétamine 5-chloro benzoate de méthyle, 1990 g de de diméthylformamide, 8,8 g de chlorure de benzyltributylammonium, 266,6 g de carbonate de potassium et 164 g de chlorure d'allyle.

On chauffe une heure à 50°C, une heure à 60°C, une heure à 70°C et une heure à 80°C. On refroidit à 20°C et verse le contenu du ballon dans un réacteur contenant 2 kg de glace et 2385 ml d'eau.

On essore le précipité, le lave à l'eau et le sèche à l'étuve à 50°C.
Rendement = 463 g (93 %)
P F = 104-1106°C

Le produit est purifié par recristallisation dans 1200 ml de toluène.
Rendement = 294 g (59 %)
P F = 111-112°C

Stade III : 2-ALLYLOXY 4-AMINO 5-CHLORO BENZOATE DE METHYLE.

Dans un ballon de trois litres muni d'un agitateur, d'un thermomètre, d'un réfrigérant et d'une ampoule à brome, on introduit 293,5 g de 2-allyloxy 4-acétamino 5-chloro benzoate de méthyle, 1426 ml de méthanol et en filet, en laissant évoluer la température, 108 g d'acide sulfurique (d = 1,83).

On chauffe ensuite 1,5 heure à reflux, refroidit à 20°C et coule le contenu du ballon dans un réacteur contenant 1300 g de glace et 5000 ml d'eau.

On agite une heure, essore le précipité, le lave à l'eau et le sèche à l'étuve à 60°C.
Rendement = 241 g (96 %)
P F = 110°C

Stade IV : 2-ALLYLOXY 4-ISOTHIOCYANATO 5-CHLORO BENZOATE DE METHYLE.

Dans un ballon de deux litres muni d'un agitateur, d'un thermomètre, d'un réfrigérant et d'une ampoule à brome, on introduit 241 g de 2-allyloxy 4-amino 5-chloro benzoate de méthyle, 116 g de carbonate de potassium, 1345 g de 1,2-dichloréthane et, goutte à goutte à 20°C, 148 g de thiophosgène.

On chauffe ensuite deux heures à 50-60°C, puis deux heures à 70-80°C. On refroidit à 20°C, filtre les sels, les lave avec un peu de dichloréthane et évapore le filtrat sous vide à 50°C.

Le résidu pâteux est mis dans un bécher et est repris par 500 ml d'éther de pétrole.

Des cristaux et blocs très durs se forment. On laisse reposer une nuit vers 0-5°C, essore le solide, le lave avec un peu d'éther de pétrole puis le sèche à l'air.
Rendement = 240 g (85 %)
P F = 74-75°C

Stade V : 2-ALLYLOXY 4-[(1-H-4,5-DIHYDRO 2-IMIDAZOLYL)AMINO]5-CHLORO BENZOATE DE METHYLE.

Dans un ballon de quatre litres muni d'un agitateur, d'un thermomètre, d'un réfrigérant et d'une ampoule à brome, on introduit 152 g d'éthylènediamine, 845 ml de chlorure de méthylène et en filet à 20°C, une solution de 240 g de 2-allyloxy 4-isothiocyanato 5-chloro benzoate de méthyle dans 1270 ml de chlorure de méthylène.

On laisse ensuite agiter 30 mn à 20°C, puis on ajoute 845 ml d'eau, agite une heure et décante la phase organique . On la lave deux fois avec 850 ml d'eau et évapore le solvant sous vide.

On obtient une huile jaune très épaisse.

On ajoute 1690 ml d'éthanol, on chauffe vers 30-35°C jusqu'à dissolution totale, puis évapore sous vide 400 ml de solvant.

On chauffe ensuite la solution à 60°C et coule, par l'intermédiaire d'une ampoule à brome, une solution de 338 g d'acétate de plomb trihydraté dans 1350 ml d'eau.

On chauffe deux heures au reflux, refroidit et filtre le précipité noir vers 70°C.

On le lave à l'éthanol et acidifie le filtrat avec 17 ml d'acide sulfurique (d = 1,83) en solution dans 212 ml d'eau, après être revenu à 20°C.

On ajoute 1500 ml d'eau, évapore sous vide 1400 ml d'éthanol (bain à 60°C) et laisse reposer une nuit à 20°C.

On filtre en présence de noir de carbone 3 S puis on alcalinise le filtrat avec 211 ml d'ammoniaque (d = 0,90).

Le produit cristallise, on le filtre, le lave à l'eau et le sèche à l'étuve à 50-60°C.
Rendement = 215 g (82 %)
P F = 174-178°C

Stade VI : N-(2-DIETHYLAMINO ETHYL) 2-ALLYLOXY 4-[(1-H 4,5-DIHYDRO 2-IMIDAZOLYL) AMINO]5-CHLORO BENZAMIDE.

Dans un ballon de 250 ml muni d'un agitateur, d'un thermomètre et d'un réfrigérant, on introduit 37 g de 2-allyloxy 4-[(1-H 4,5-dihydro 2-imidazolyl) amino]5-chloro benzoate de méthyle, 43 g de diéthylaminoéthylamine et 60 ml de méthanol.
On chauffe à reflux (73°C). La suspension épaisse au début, se fluidifie jusqu'à dissolution totale.
Après 96 heures de réaction, on refroidit à 20°C, filtre les cristaux, les lave au méthanol et les sèche à l'étuve à 50°C.
Poids = 37 g 1er jet
P F = 194°C

Le filtrat est évaporé sous vide. On ajoute 200 ml d'eau au résidu huileux qui cristallise. On essore et lave à l'eau ce 2ème jet.
Poids = 8 g 2ème jet.

Ces 8 g sont dissous dans 35 ml d'éthanol bouillant et on laisse recristalliser le produit à température ambiante. On le filtre et le sèche à 50°C.
Poids = 3 g 3ème jet.
Rendement = 37 + 3 = 40 g (85 %)

Ce 3ème jet et le 1er jet sont réunis et recristallisés deux fois de suite dans 200 ml puis 165 ml d'éthanol avec séchage de l'intermédiaire. Rendement = 28 g (60 %)
P F = 194°C

Analyses

- Produit insoluble à 1 % dans l'eau, le chlorhydrate préparé extemporanément est soluble à 20 % dans l'eau.
- Le spectre RMN était compatible avec la structure du produit attendu.

Exemple XXXIV : N-[1-(1-CYCLOHEXENYLMETHYL) 2-PYRROLIDINYLMETHYL]2-ALLYLOXY 4-[(1-H 4,5-DIHYDRO 2-IMIDAZOLYL) AMINO]5-CHLORO BENZAMIDE.

Dans un ballon de 250 ml muni d'un agitateur, d'un thermomètre et d'un réfrigérant, on a introduit 37 g de 2-allyloxy 4-[(1-H 4,5-dihydro 2-imidazolyl amino]5-chloro benzoate de méthyle, 45 g de 1-(1-cyclohexènylméthyl) 2-aminométhyl pyrrolidine et 75 ml de méthanol.

On a chauffé 96 heures à reflux, refroidi à 20°C, filtré, évaporé le filtrat et fait cristalliser le produit en ajoutant 250 ml d'eau. On a essoré le solide, on l'a lavé à l'eau et séché à l'étuve à 50°C.
Rendement = 46 g (81 %)
P F - 194°C

On a recristallisé ce produit dans 89 ml d'acide chlorhydrique N et 120 ml d'eau. On a filtré et alcalinisé le filtrat avec de l'ammoniaque. Les cristaux formés ont été essorés, lavés à l'eau et séchés à l'étuve à 50°C.
Rendement = 18 g (32 %)
P F = 189°C

Exemple XXXV : N-(ALLYL) 2-METHOXY 4-[(1-H 4,5-DIHYDRO 2-IMIDAZOLYL) AMINO]5-CHLORO BENZAMIDE.

Dans un tricol d'un litre équipé d'une agitation, d'un thermomètre et d'un réfrigérant, on charge 85,05 g de 2-méthoxy 4-[(1-H 4,5-dihydro 2-imidazolyl) amino]5-chloro benzoate de méthyle, 200 ml d'éthylèneglycol et 51,3 g d'allylamine, et porte sous agitation à 70-75°C pendant deux heures.
On ajoute alors 200 ml d'eau et 30 ml de lessive de soude à 40 % et agite une heure à 70-75°C.
On refroidi, essore, lave deux fois avec 250 ml d'eau puis deux fois avec 125 ml d'acétone et on sèche à 70°C.

On obtient : 80,7 g (rendement = 87,1 %)
P F : 224°C
RMN : Compatible, présence d'environ 10 % d'ester.

Purification

79 g de produit sont dissous au reflux dans 550 ml de diméthyl formamide contenant 25 % d'eau, traités au noir, filtrés et cristallisés une nuit au réfrigérateur.

Le produit est essoré, lavé à l'acetone et séché.

On a 66 g de produit gris, qui sont solubilisés dans 1320 ml d'eau, et 30 ml d'acide chlorhydrique concentré, traités au noir et précipités par 50 ml d'ammoniaque.

Le précipité est essoré, lavé et séché.

Il contient toujours de l'ester.

Le produit est agité 3 heures (à 80°C) dans 660 ml d'eau et 20 g de soude en pastilles. On filtre chaud et lave deux fois avec 300 ml d'eau. Le gâteau est repris dans trois litres d'eau et 30 ml d'acide chlorhydrique concentré (pH 1), traité au noir et précipité par 50 ml d'ammoniaque. On laisse reposer une nuit au réfrigérateur, essore, lave à l'eau et sèche 5 jours à 60°C sous vide sur $P_2O_5$.

On obtient : 44,6 g (rendement de purification = 56,5 %)

Analyse

P F (Kofler) = 225°C
$H_2O$ < 0,1 %
Titre = 99,6 %
C1 = 11,47 % (calculé = 11,48 %)
les spectres RMN et IR sont compatibles avec la structure proposée.

Exemple XXXVI : N-(ISOPROPYL) 2-METHOXY 4-[(1-H 4,5-DIHYDRO 2-IMIDAZOLYL) AMINO]5-CHLORO BENZAMIDE.

Dans un tricol d'un litre muni d'une agitation, d'un thermomètre et d'un réfrigérant, on charge 85,05 g de 2-méthoxy 4-[(1-H 4,5-dihydro 2-imidazolyl) amino] 5-chloro benzoate de méthyle, 200 ml d'éthylène-glycol et 53,1 g d'isopropylamine, et porte sous agitation à 70-75°C pendant 120 heures.

On ajoute alors 200 ml d'eau et 30 ml de lessive de soude à 40 % et agite encore une heure à 70-75°C.

On refroidit, essore, lave deux fois avec 250 ml d'eau puis deux fois avec 125 ml d'acétone et sèche à 70°C.

On obtient = 65,5 g (rendement = 70,3 %)
P F = 251°C
R M N = Compatible, ester à la limite de détection

Purification

64,2 g de produit sont dissous à chaud ( 110°C) dans 275 ml de de diméthyl formamide, traités au noir, filtrés et cristallisés une nuit au réfrigérateur.

Le produit est essoré, lavé à l'acétone et séché.

On obtient : 47,6 g de produit (P F = 251°C)

Le produit est dissous dans 475 ml d'eau et 15 ml d'acide chlorhydrique concentré, traité au noir, filtré et précipité par 30 ml d'ammoniaque à 25 %. On laisse reposer deux heures au réfrigérateur, essore, lave à l'eau et sèche quatre jours à 60°C sous vide sur $P_2O_5$.

On a 41,8 g de produit (rendement de purification : 65 %)

Analyse

P F Kofler = 249°C
$H_2O$ = 0,1 %
Titre = 100,0 %
C1 = 11,39 % (calculé = 11,41 %)
N = 18,12 % (calculé = 18,03 %)
Les spectres RMN et IR sont compatibles avec la structure proposée.

Exemple XXXVII : N-(2-DIETHYLAMINO ETHYL) 2-METHOXY 4-[(4,5-DIHYDRO 1,3-THIAZOL 2-YL) AMINO]5-CHLORO BENZAMIDE.

Stade I : N-(5-METHOXY 4-METHOXYCARBONYL 2-CHLOROPHENYL) N'-(2-HYDROXY 1-ETHYL) THIOUREE.

Dans un ballon de deux litres muni d'un agitateur, d'un thermomètre, d'un réfrigérant et d'une ampoule à brome, on introduit 71 g d'éthanolamine, 350 ml de chlorure de méthylène et goutte à goutte, en maintenant la température entre 15 et 20°C par refroidissement, une solution filtrée de 181 g de 2-méthoxy 4-isothiocyanato 5-chloro benzoate de méthyle dans 100 ml de chlorure de méthylène. On agite ensuite 1,5 heure en ôtant le bain froid et essore le précipité formé. On le lave avec un peu de chlorure de méthylène et le sèche à l'étuve à 50°C.

Rendement = 188 g (84 %)
P F = 164-170°C

Analyse

Le spectre RMN était compatible avec la structure du produit attendu.

Stade II : 2-METHOXY 4-[(4,5-DIHYDRO 1,3-THIAZOL 2-YL) AMINO]5-CHLORO BENZOATE DE METHYLE.

Dans un ballon d'un litre muni d'un agitateur, d'un thermomètre, d'un réfrigérant et d'une ampoule à brome, on introduit 64 g de N-(5-méthoxy 4-méthoxycarbonyl 2-chlorophényl) N'(2-hydroxy 1-éthyl) thiourée, 375 ml de méthanol et goutte à goutte, en refroidissant à 20°C, 20 ml d'acide sulfurique (d = 1,83).

On chauffe ensuite quatre heures à reflux, refroidit et verse le mélange réactionnel dans 1000 ml d'eau et 300 g de glace. On alcalinise l'ensemble avec de l'ammoniaque (d = 0,90) jusqu'à pH 9-10 et essore le précipité qui est lavé à l'eau et séché à l'étuve à 50°C.
Rendement = 56 g (93 %)
P F = 189°C

Analyse

Le spectre RMN était compatible avec la structure du produit attendu.

Stade III : N-(2-DIETHYLAMINO ETHYL) 2-METHOXY 4-[(4,5-DIHYDRO 1,3-THIAZOL 2-YL) AMINO]5-CHLORO BENZAMIDE.

Dans un ballon de 250 ml muni d'un agitateur, d'un thermomètre et d'un réfrigérant, on a introduit 45 g de 2-méthoxy 4-[(4,5-dihydro 1,3-thiazol 2-yl) amino]5-chloro benzoate de méthyle, 90 ml de méthanol et 41,3 g de diéthylaminoéthylamine. On a chauffé à reflux (70°C) deux jours et deux nuits, refroidi la solution et évaporé le solvant sous vide.
On a ajouté 150 ml d'eau et 450 ml d'acétate d'éthyle sur le résidu. Le produit a cristallisé. On l'a essoré, lavé avec un peu d'éther et séché à l'étuve à 50°C.
Rendement = 41,5 g (72 %)
P F = vers 112°C

Ce produit a été recristallisé deux fois de suite, en filtrant à chaud en présence de noir de carbone, dans 530 ml puis 450 ml d'acétate d'éthyle.
Rendement = 20 g (34 %)
P F = 162°C

Le produit était compatible mais conservait dans sa structure un peu de solvant. On l'a dissous dans 180 ml d'eau et 12 ml d'acide chlorhydrique (d = 1,18). On a filtré la solution et ajouté de l'ammoniaque (d = 0,90) au filtrat jusqu'à pH = 9-10.
On a laissé reposer la suspension deux heures et essoré le produit qui a été lavé à l'eau et séché à l'étuve à 50°C.
Rendement = 15,5 g (27 %)
P F = 166°C

Analyses

Le spectre RMN était compatible avec la structure du produit attendu.

Exemple XXXVIII : N-[(1-ETHYL 2-PYRROLIDINYL) METHYL] 2-METHOXY 4-[(4,5-DIHYDRO 1,3-THIAZOL 2-YL) AMINO]5-CHLORO BENZAMIDE.

Dans un ballon de 250 ml muni d'un agitateur, d'un thermomètre et d'un réfrigérant, on a introduit 45 g de 2-méthoxy 4-[(4,5-dihydro 1,3-thiazol 2-yl) amino] 5-chloro benzoate de méthyle, 50 ml de 1-éthyl 2-aminométhyl pyrrolidine et 90 ml de méthanol.
On a agité et chauffé à reflux (70°C) trois jours. Le produit s'est solubilisé lentement. On a refroidi, évaporé le solvant sous vide et ajouté 300 ml d'eau au résidu pâteux. On a extrait avec 400 ml, puis 100 ml de chlorure de méthylène.
La phase organique a été lavée trois fois avec 200 ml d'eau puis évaporée à sec sous vide.
On fait cristalliser la pâte résiduelle avec 300 ml d'acétate d'éthyle. On a essoré les cristaux qui ont été lavés avec un peu de solvant puis séchés à l'étuve à 50°C.
Rendement = 47,5 g (80 %)
P F = 144-148°C

Ce produit a été recristallisé dans 200 ml de méthanol. Après un repos de quatre heures au réfrigérateur, on a essoré les cristaux, on les a lavés à l'eau puis séchés à l'étuve à 50°C.
Rendement = 31 g (52 %)

On a dissous ce solide dans 285 ml d'eau et 15 ml d'HC1 (d = 1,18), filtré la solution et reprécipité la base avec 25 ml d'ammoniaque (d = 0,90).
De gros blocs se sont formés, on les aessorés puis écrasés et on les a repris humides dans 100 ml de méthanol.
On a chauffé à reflux jusqu'à dissolution complète puis laissé recristalliser le produit au réfrigérateur 1,5 heure.
On a essoré les cristaux puis on les a lavés à l'eau et séchés à l'étuve à 50°C.
Rendement = 22 g (37 %)
P F = 147°C

Analyses

Les spectres IR et RMN étaient compatibles avec la structure du produit attendu.

Exemple XXXIX : N-(2-DIETHYLAMINO ETHYL) 2-METHOXY 4-[(4,5-DIHYDRO 2-OXAZOLYL) AMINO]5-CHLORO BENZAMIDE.

Stade I : 2-METHOXY 4-[(4,5-DIHYDRO 2-OXAZOLYL) AMINO]5-CHLORO BENZOATE DE METHYLE.

Dans un ballon de quatre litres muni d'un agitateur, d'un thermomètre, d'un réfrigérant et d'une ampoule à brome, on a introduit 104 g de N-(2-chloro 4-méthoxy carbonyl 5-méthoxy phényl) N'-(2-hydroxy 1-éthyl) thiourée, 846 ml de méthanol et 80 g d'iodure de méthyle.
On a chauffé une heure à 50-55°C, refroidi à 40°C et coulé en filet une solution de 20 g de sodium dans 320 ml de méthanol.
On a porté ensuite à reflux 3,5 heures et laissé reposer une nuit.
On a ajouté une solution de 40 g de chlorure d'ammonium dans 1000 ml d'eau et 40 ml d'ammoniaque (d = 0,90).
On a évaporé la solution obtenue sous vide de façon à éliminer le méthanol.

On ajouté encore une solution de 40 ml d'ammoniaque (d = 0,90) dans 600 ml d'eau et essoré le précipité.
On a disssous les cristaux dans une solution de 30 ml d'acide chlorhydrique (d = 1,18) dans 300 ml d'eau, sans chauffer. On a filtré la solution et alcalinisé le filtrat avec 40 ml d'ammoniaque. (d = 0,90).
Le produit a précipité sous la forme d'une gomme qui a cristallisé. On a essoré le solide blanc, on l'a lavé à l'eau et séché à l'étuve à 50°C.
Rendement = 68,5 g (74 %)
P F = 154°C

Analyse

Le spectre RMN révélait la présence d'une impureté en faible teneur.

Stade II : N-(2-DIETHYLAMINO ETHYL) 2-METHOXY 4-[(4,5-DIHYDRO 2-OXAZOLYL) AMINO]5-CHLORO BENZAMIDE.

Dans un ballon de 250 ml muni d'un agitateur, d'un thermomètre et d'un réfrigérant, on a introduit 34,2 g de 2-méthoxy 4-[(4,5-dihydro 2-oxazolyl) amino]5-chloro benzamide, 70 ml de méthanol et 33 g de diéthylamino éthyl amine.
On a chauffé à reflux (≃ 70°C) environ 31 heures. La solubilisation complète s'est produite après 2,5 heures de reflux.
On a refroidi, évaporé le solvant sous vide, ajouté 100 ml de chlorure de méthylène pour dissoudre la gomme épaisse et lavé la solution trois fois avec 50 ml d'eau. On a ajouté 150 ml d'acétate de butyle, évaporé le chlorure de méthylène sous vide, ajouté 200 ml d'acétate de butyle, chauffé pour redissoudre et laissé cristalliser le produit une nuit à 20°C.
Les cristaux ont été essorés et séchés à l'étuve à 50°C.
Rendement = 25 g (57 %)

Le solide blanc a été recristallisé dans 100 ml d'isopropanol. Après un repos de trois heures au réfrigérateur, on a essoré les cristaux qui ont été lavés avec un peu de solvant et séchés à l'étuve à 50°C.
Rendement : 22,5 g (51 %)

On a ensuite dissous le produit dans 150 ml d'eau et 173 ml d'acide chlorhydrique N, filtré la solution et alcalinisé le filtrat avec 20 ml d'ammoniaque (d = 0,90).
On a laissé cristalliser deux jours à 20°C, essoré, lavé à l'eau et séché le produit à l'étuve à 50°C.

Rendement = 18 g (41 %)
P F = 137°C

Analyses

Les spectres RMN et IR étaient compatibles avec la structure du produit attendu.

Exemple XL : N-[(1-CYCLOPROPYLMETHYL 2-PYRROLIDINYL) METHYL] 2-METHOXY 4-[(4,5-DIHYDRO 2-OXAZOLYL) AMINO]5-CHLORO BENZAMIDE.

Dans un ballon de 250 ml muni d'un agitateur, d'un thermomètre et d'un réfrigérant, on a introduit 42,7 g de 2-méthoxy 4-[(4,5-dihydro 2-oxazolyl) amino]5-chloro benzoate de méthyle, 65 ml de méthanol et 46,2 g de 1-cyclopropylméthyl 2-aminométhyl pyrrolidine.
On a chauffé la suspension 26 heures à reflux (72°C). Après 2,5 heures de chauffage le solide était totalement dissous.
On a refroidi à 20°C, évaporé le solvant sous vide et fait cristalliser l'huile résiduelle avec 125 ml d'acétate d'éthyle.
On a laissé reposer quatre heures au réfrigérateur, et essoré les cristaux qui ont été lavés trois fois avec 30 ml d'acétate d'éthyle puis séchés à l'étuve à 50°C.
Rendement = 32,3 g (53 %)
P F = 159°C
On a recristallisé le produit dans 80 ml d'éthanol à 95% puis sans séchage dans 1200 ml d'acétate d'éthyle.
On l'a essoré, lavé avec un peu d'acétate d'éthyle puis séché à l'étuve à 50°C.
Rendement = 20 g (33%)
PF = 161°C

Analyses

– Les spectres RMN et IR étaient compatibles avec la structure du produit attendu.
– Le monochlorhydrate préparé extemporanément était soluble à environ 32% dans l'eau.

Des essaqis biochimiques et pharmacologiques ont été réalisés avec les composés de l'invention. On a trouvé que, contrairement aux dérivés de 2-alcoxybenzamide antérieurement décrits et connus pour leur activité antidopaminergique s'exerçant à différents niveaux, les composés selon l'invention:

– ne se liaient plus aux récepteurs dopaminergiques dans les essais "in vitro", quel que soit le type de ces récepteurs D1 ou D2;
– ne déterminaient plus "in vivo" une élévation de la prolactine, effet biochimique typique des antidopaminergiques s'exerçant par blocage de certains récepteurs hypophysaires de la dopamine;
- ne s'opposaient plus à certains effets comportementaux (émesis, verticalisation, hyperactivité, mouvements stéréotypés etc...) provoqués chez le chien, le rat et la souris par les agonistes dopaminergiques, tels que l'apomorphine et l'amphétamine qui sont utilisés couramment par les pharmacologues pour déceler et mesurer les effets antidopaminergiques d'une drogue ;
- ne produisaient pas d'effet dépresseur central, sauf pour certains à haute dose, encore que cet effet puisse relever d'un effet toxique et non pas neuroleptique;
- n'induisaient pas de catalepsie, commme les neuroleptiques.

La démonstration de l'absence de liaison aux récepteurs dopaminergiques a été apportée par des essais "in vitro" sur des préparations de striatum de rat consistant à étudier le déplacement de ligands spécifiques des récepteurs de la dopamine par les composés de l'invention. On a trouvé par exemple pour le composé de l'exemple 1 une concentration inhibitrice 50 supérieure à $10^{-4}$M à l'égard de $^3$H-spiperone, ligand des récepteurs $D_2$ et aucun déplacement du $^3$H-piflutixol, ligand des récepteurs $D_1$.

L'absence d'élévation de la prolactine dans le sang, après administration des composés, a été démontrée chez le rat mâle recevant le composé de l'exemple 1 à la dose de 1mg/kg, par voie sous-cutanée. L'essai étant conduit sur 8 rats ; aucune élévation des taux de prolactine n'a été observée dans le sérum de ces animaux, le sang ayant été prélevé 10 et 30 mn après l'administration du composé, alors que la même dose d'un antidopaminergique de référence (le Sulpiride) étudié dans les mêmes conditions, multiplie par un facteur de 10 environ les taux de la prolactinémie de base.

L'absence d'antagonisme exercé par les composés à l'égard des effets comportementaux de l'apomorphine a été démontrée chez le chien par le test du vomissement (effet non significatif jusqu'à 100 µg/kg, par voie sous-cutanée) et par les tests des stéréotypies chez le rat et de la verticalisation chez la souris. Dans tous ces tests, aucun effet des composés n'a été observé, même à des doses très importantes, par exemple 100 mg/kg.

L'absence d'effet cataleptigène des composés a été observée chez le rat qui, ayant reçu une dose, même importante (100 mg/kg, par voie sous-cutanée), n'a pas été capable de conserver une position imposée.

En conséquence, les composés selon l'invention, ne peuvent en aucune manière être considérés comme des drogues antidopaminergiques, agissant comme les neuroleptiques.

Ils possèdent en revanche la propriété inattendue et remarquable de stimuler l'activité motrice non seulement chez les rongeurs (rats et souris), mais aussi chez un primate : le marmouset. Cette propriété a été mise en évidence chez le rat placé dans des cages individuelles équipées de cellules photoélectriques. L'activité motrice a été mesurée d'après le nombre d'interruptions d'un faisceau lumineux touchant ces cellules. Les mesures ont été faites entre 8h et 10h du soir et les résultats exprimés en pourcentage par rapport à la valeur des témoins. D'autre part, l'aspect des animaux a été noté.

Les graphiques des figures 1 à 15 indiquent la puissance de l'effet activant des composés de différents exemples, aux doses indiquées, administrées par voie sous-cutanée ou intra-accumbens.

D'autre part, il a été démontré que l'effet activant persistait en traitement chronique.

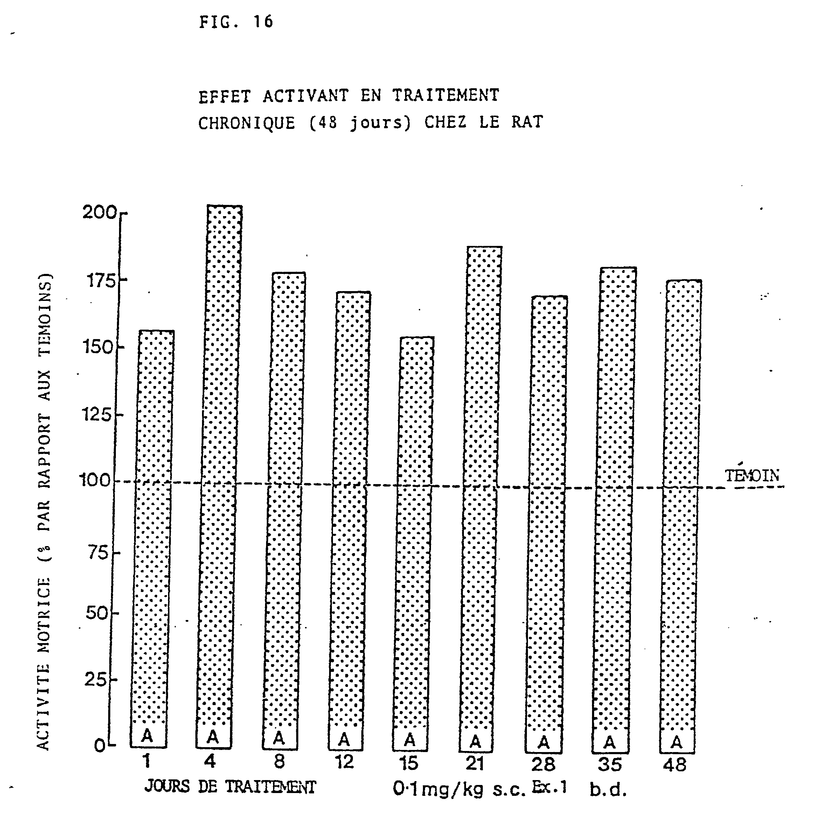

La figure 16 montre par exemple l'effet du composé 1 : 0,1 mg/kg administré chez le rat par voie sous-cutanée, chaque jour à raison de 2 injections par jour pendant 48 jours. L'enregistrement de l'activité motrice a été réalisé par cellules photoélectriques comme dans les essais à dose unique.

L'effet activant des composés a été également démontré chez le marmouset, placé dans une cage individuelle munie de 2 barres. Les mouvements de l'animal entre le plancher et la cage, la barre intermédiaire, et la barre supérieure, ont été enregistrés par un procédé à rayon infrarouge.

Compte tenu de cette propriété activante des composés de l'invention, des modèles animaux de dépression comportementale ont été mis en oeuvre pour rechercher un éventuel effet antidépresseur, par exemple la souris prétraitée par la réserpine(5 mg/kg) par voie intrapéritonéale 18h avant le test, ou le rat recevant de la réserpine dans le nucleus accumbens (1 ug/24h). Le composé de l'exemple 1, à la dose de 0,1 mg/kg par voie sous-cutanée antagonise la dépression motrice et le ptosis, se révélant à cette dose aussi actif que 30 mg/kg d'imipramine par voie intrapéritonéale.

Pendant cette recherche pharmacologique , une étude toxicologique du composé le plus remarquable de cette série à été réalisée. Le tableau suivant indique la toxicité aiguë du composé selon l'exemple 1, en fournissant les valeurs de la dose léthale 50 (DL 50) chez la souris traitée par différentes voies d'administration.

COMPOSE DE L'EXAMPLE

| Voie d'administration | DL 50 (mg/kg) |
| --- | --- |
| i.v. | 12,8 - 14,4 |
| s.c. | 52 - 54 |
| i.p. | 37,7 - 40,8 |
| p.o. | 165 - 180 |

Ce profil pharmacologique des composés de l'invention suggère que les plus actifs pourraient être appliqués en thérapeutique dans certaines formes de dépression et/ou d'inhibition psychomotrice.

**Revendications pour les Etat Contractants BE,CH, DE FR, GB IT, LU, NL, SE**

1. Nouveaux benzamides substitués de formule générale (I):

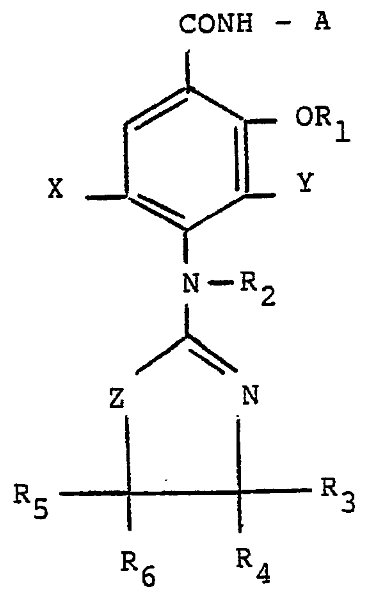

(I)

dans laquelle:

– A représente un groupe alkyle, alcényle, diéthylaminoéthyle ou un groupe de formule:

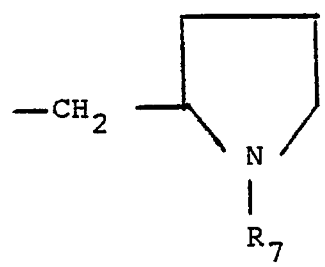

$R_7$ étant un atome d'hydrogène, un groupe alkyle, alcényle, aryle, aralkyle, cycloalkyle, cycloalkylalkyle, cycloalcényle ou cycloalcénylalkyle,

– $R_1$ et $R_2$ représentent des atomes d'hydrogène, des groupes alkyle ou alcényle,

– $R_3$, $R_4$, $R_5$ et $R_6$ représentent des atomes d'hydrogène ou des groupes alkyle,

– X représente un atome de chlore ou de brome,

– Y représente un atome d'hydrogène, de chlore ou de brome,

– Z représente un groupe NH, un atome d'oxygène ou de soufre, les valeurs alkyle, cycloalkyle, alcényle et cycloalcényle contenant un maximum de six atomes de carbone, ainsi que leurs isomères optiques et leurs sels d'addition physiologiquement acceptables.

2. Selon la revendication 1, un composé de formule:

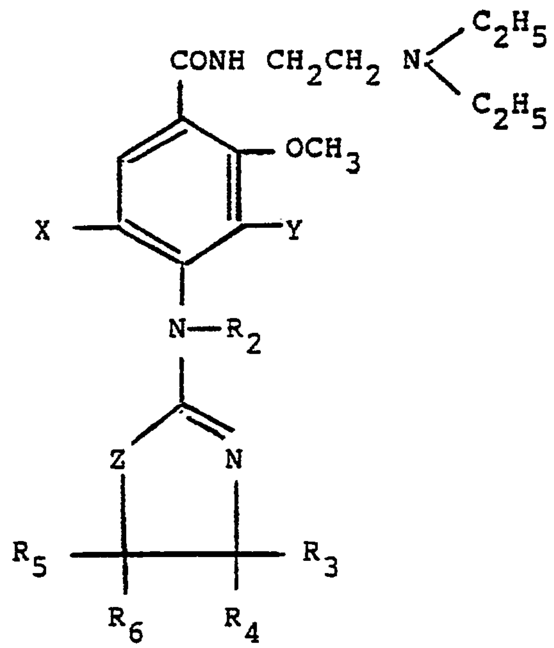

dans laquelle X, Y, Z, $R_2$, $R_3$, $R_4$, $R_5$, $R_6$ ont les mêmes significations que dans la revendication 1.

3. Selon les revendications 1 et 2, le N-[2-(diéthylamino)éthyl] 2-méthoxy 4-[(1-H-4,5-dihydro 2-imidazolyl) amino] 5-chloro benzamide.

4. Selon les revendications 1 et 2, le N-[2-(diéthylamino) ethyl] 2-méthoxy 4-[N-éthyl-N-(1-H-4,5-dihydro 2-imidazolyl) amino] 5-chloro benzamide.

5. Selon les revendications 1 et 2, le N-[2-(diéthylamino)éthyl]2-méthoxy 3,5-dichloro 4-[(1-H 4,5-dihydro 2-imidazolyl) amino]benzamide

6. Selon les revendications 1 et 2, le N-[2-(diéthylamino)éthyl]2-méthoxy 4-[(1-H 4,5-dihydro 4-diméthyl 2-imidazolyl) amino]5-chloro benzamide

7. Selon les revendications 1 et 2, le N-[2-(diéthylamino)éthyl]2-méthoxy 4-[(4,5-dihydro 1,3-thiazol 2-yl) amino]5-chloro benzamide.

8. Selon les revendications 1 et 2, le N-[2-(diéthylamino)éthyl]2-méthoxy 4-[(4,5-dihydro 2-oxazolyl) amino]5-chloro benzamide

9. Selon la revendication 1, un composé répondant à la formule :

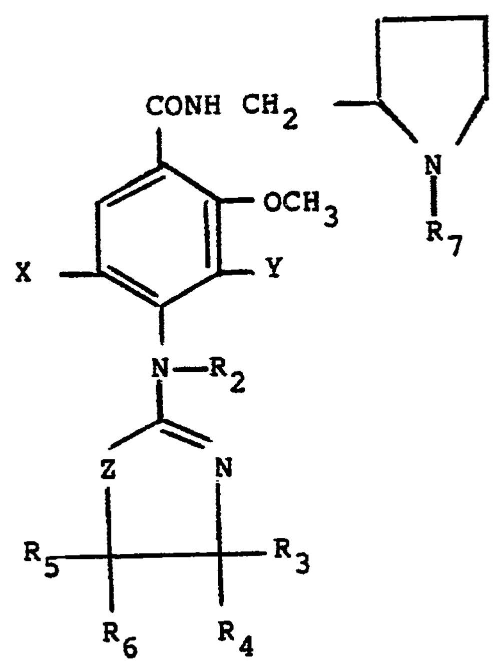

dans laquelle X, Y, Z, $R_2$, $R_3$, $R_4$, $R_5$, $R_6$, $R_7$ ont les mêmes significations que dans la revendication 1

10. Selon les revendications 1 et 9, le N-[(1-éthyl 2-pyrrolidinyl) méthyl]2-méthoxy 3,5-dibromo 4-[(1-H 4,5-dihydro 2-imidazolyl) amino]benzamide

11. Selon les revendications 1 et 9, le N- [(1-cyclohexenylméthyl 2-pyrrolidinyl) méthyl]2-méthoxy 4-[(1-H 4,5-dihydro 2-imidazolyl) amino]5-chlorobenzamide

12. Selon les revendications 1 et 9, le N-[(1-cyclopropylméthyl 2-pyrrolidinyl) méthyl] 2-méthoxy 4-[(1-H 4,5-dihydro 2-imidazolyl) amino]5-chlorobenzamide

13. Selon les revendications 1 et 9, le N-[(1-allyl 2-pyrrolidinyl) méthyl] 2-méthoxy 4-[(1-H 4,5-dihydro 2-imidazolyl) amino]5-chloro benzamide

14. Selon les revendications 1 et 9, le N-[(1-éthyl 2-pyrrolidinyl) méthyl] 2-méthoxy 4-[(4,5-dihydro 1,3-thiazol 2-yl) amino]5-chloro benzamide.

15. Selon les revendications 1 et 9, le N-[(1-cyclopropylméthyl 2-pyrrolidinyl) méthyl] 2-méthoxy 4-[(4,5-dihydro 2-oxazolyl) amino]5- chloro benzamide.

16. Selon la revendication 1, le N-(allyl) 2-méthoxy 4-[(1-H 4,5-dihydro 2-imidazolyl) amino]5-chlorobenzamide

17. Procédé de préparation des composés selon l'une quelconque des revendications 1 à 16 qui consiste à traiter un acide de formule (II) :

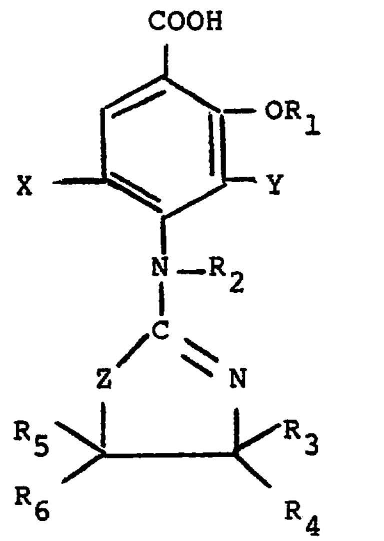

(II)

dans laquelle $R_1$, $R_2$, $R_3$, $R_4$, $R_5$, $R_6$ X, Y et Z ont les mêmes significations que dans la revendications 1, ou l'un de ses dérivés réactifs, par une amine de formule (III) :

A - $NH_2$ (III)

dans laquelle A est défini comme dans la revendication 1, ou l'un de ses dérivés réactifs.

18. Procédé de préparation des composés de formule (I) tels que définis dans l'une quelconque des revendications 1 à 16, qui consite à transformer un 4-amino 5 (ou 3,5)-halogéno benzoate d'alkyle de formule:

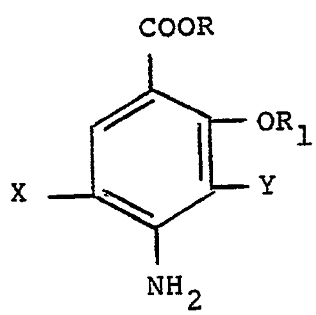

dans laquelle R représente un groupe alkyle et $R_1$, X et Y sont définis comme dans la revendication 1, en 4-isothiocyanato benzoate d'alkyle de formule (IV) :

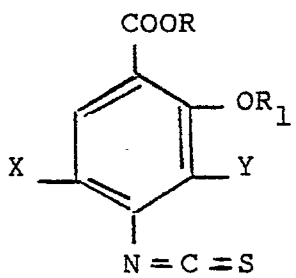

(IV)

puis en dérivé 4-(N'-amino(ou hydroxy) alkyl) thiouréido de formule (V):

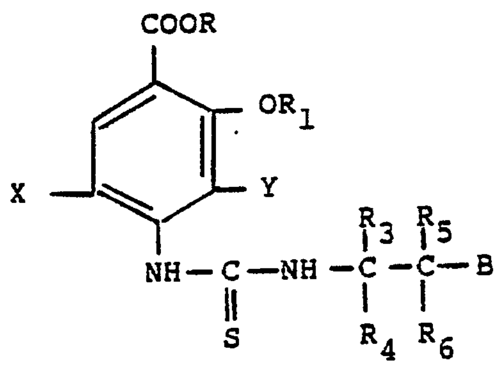

(V)

dans laquelle B représente un groupe amino ou hydroxy,
puis en dérivé 4-[(4,5-dihydro 2-imidazolyl (ou 2-thiazolyl ou 2-oxazolyl) amino] de formule (VI) :

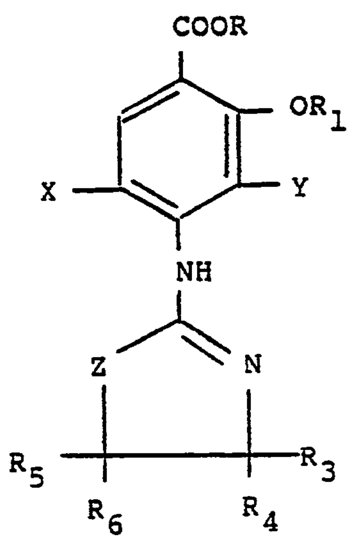

(VI)

et enfin à amidifier le composé de formule (VI) pour obtenir le composé de formule (I) correspondant ou à amidifier le composé de formule (VI) pour obtenir un composé de formule (I) dans laquelle $R_2$ est un atome d'hydrogène et à le transformer en composé de formule (I) dans laquelle $R_2$ est un groupe alkyle ou alcényle.

19. Procédé de préparation des composés de formule (I) où $R_2 \neq H$, dans lequel le dérivé 4-[(4,5-dihydro 2-imidazolyl (ou 2-thiazolyl ou 2-oxazolyl) amino] de formule (VI):

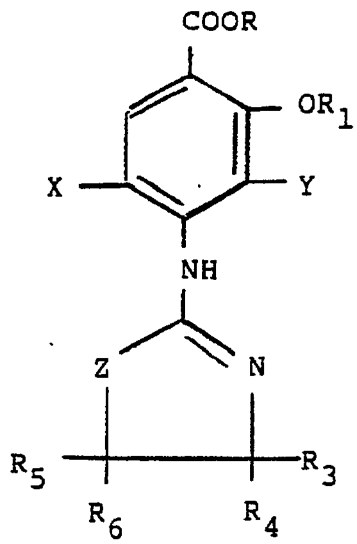

(VI)

est transformé en dérivé de formule (VI bis)

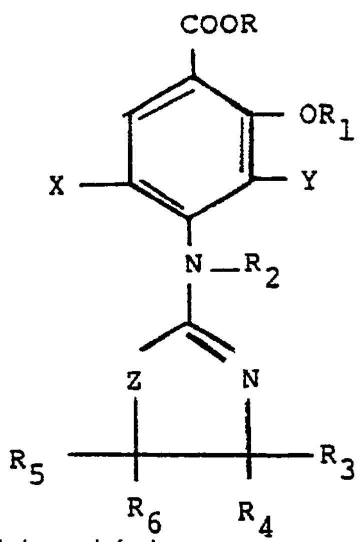

(VI bis)

avec $R_2$ = alkyle ou alcényle
qui est ensuite amidifié pour obtenir le composé de formule (I) correspondant.

20. A titre de nouveaux intermédiaires de synthèse selon la revendication 18, les 4-isothiocyanato benzoates d'alkyle de formule (IV).

21. A titre de nouveaux intermédiaires de synthèse selon la revendication 18, les 4-(N'-amino (ou hydroxy) alkyl) thiouréido benzoates d'alkyle de formule (V).

22. A titre de nouveaux intermédiaires de synthèse selon la revendication 18, les 4-[4,5-dihydro 2-thiazolyl (ou 2-oxazolyl) amino]benzoates d'alkyle de formule (VI).

23. A titre de nouveaux intermédiaires de synthèse selon la revendication 19, les 4-[4,5-dihydro 2-thiazolyl (ou 2-oxazolyl) amino]benzoates d'alkyle de formule (VI bis).

24. Procédé de préparation des composés de formule (I) dans laquelle Z représente un groupe NH, qui consiste à transformer un 4-amino 5 (ou 3,5)-halogénobenzoate d'alkyle de formule:

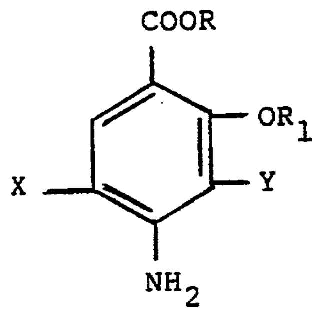

dans laquelle R représente un groupe alkyle et $R_1$, X et Y sont définis comme dans la revendication 1, en 4-formylamino benzoate d'alkyle de formule (VII):

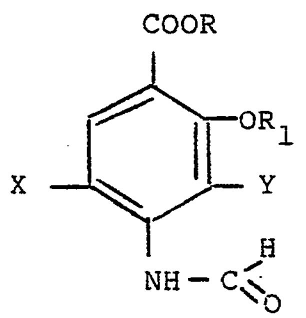

(VII)

puis en 4-dichloroformylamino benzoate d'alkyle de formule (VIII):

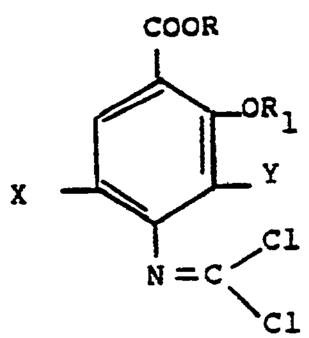

(VIII)

puis en 4-[(1-H 4,5-dihydro 2-imidazolyl) amino] benzoate de formule (VI) dans laquelle Z représente un groupe NH, et enfin à amidifier le composé de formule (VI) pour obtenir le composé de formule (I) correspondant ou à amidifier le composé de formule (VI) pour obtenir un composé de formule (I) dans laquelle $R_2$ est un atome d'hydrogène et à le transformer en composé de formule (I) dans laquelle $R_2$ est un groupe alkyle ou alcényle.

25. Procédé de préparation des composés de formule (I), dans lequel le composé de formule (VI) est transformé en dérivé de formule (VI bis) dans laquelle Z représente un groupe NH et $R_2$ représente un groupe alkyle ou alcényle,qui est ensuite amidifié pour obtenir le composé de formule (I) correspondant.

26. A titre de nouveaux intermédiaires de synthèse selon la revendication 24, les 4-dichloroformylamino benzoates d'alkyle de formule (VII).

27. A titre de nouveaux intermédiaires de synthèse selon les revendications 18, 19, 24 et 25, les 4-[(1-H 4,5-dihydro 2-imidazolyl) amino] benzoates d'alkyle de formule (VI) dans laquelle Z représente un groupe NH.

28. A titre de nouveaux intermédiaires de synthèse selon les revendications 19 et 25, les 4-[(1H-4,5-dihydro 2-imidazolyl) amino] benzoates d'alkyle de formule (VI bis) dans laquelle Z représente un groupe NH.

29. A titre de médicaments nouveaux les composés selon l'une quelconque des revendications 1 à 16.

30. Compositions pharmaceutiques caractérisées en ce qu'elles contiennent comme principe actif au moins l'un des composés selon l'une quelconque des revendications 1 à 16, joints à des excipients pharmaceutiquement acceptables.

Revendications pour les Etats Contractants: AT, GR, ES

1. Procédé de préparation de nouveaux benzamides substitués de formule générale (I):

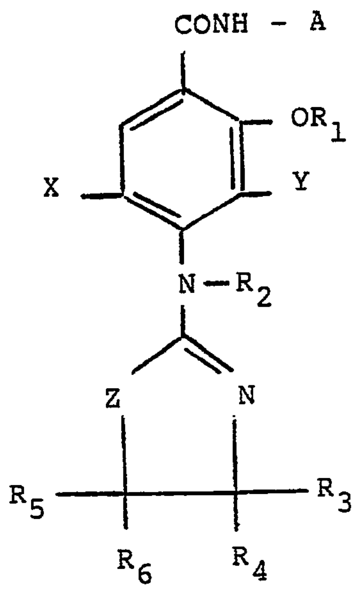

(I)

dans laquelle:

– A représente un groupe alkyle, alcényle, diéthylaminoéthyle ou un groupe de formule:

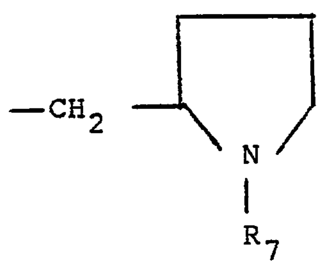

$R_7$ étant un atome d'hydrogène, un groupe alkyle, alcényle, aryle, aralkyle, cycloalkyle, cycloalkylalkyle, cycloalkyle, cycloalcényle ou cycloalcénylalkyle,

– $R_1$ et $R_2$ représentent des atomes d'hydrogène, des groupes alkyle ou alcényle,

– $R_3$, $R_4$, $R_5$ et $R_6$ représentent des atomes d'hydrogène ou des groupes alkyle,

– X représente un atome de chlore ou de brome,

– Y représente un atome d'hydrogène, de chlore ou de brome,

– Z représente un groupe NH, un atome d'oxygène ou de soufre, les valeurs alkyle, cycloalkyle, alcényle et cycloalcényle contenant un maximum de six atomes de carbone,

ainsi que leurs isomères optiques et leurs sels d'addition physiologiquement acceptables,

qui consiste à traiter un acide de formule (II)

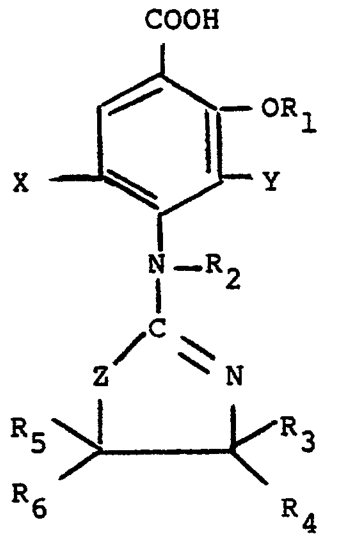

(II)

dans laquelle $R_1$, $R_2$, $R_3$, $R_4$, $R_5$, $R_6$, X, Y et Z ont les mêmes significations que ci-dessus,
ou l'un de ses dérivés réactifs, par une amine de formule (III):
A - $NH_2$ (III)
dans laquelle A est défini comme ci-dessus
ou l'un des dérivés réactifs.

2. Procédé de préparation, selon la revendication 1, d'un composé de formule :

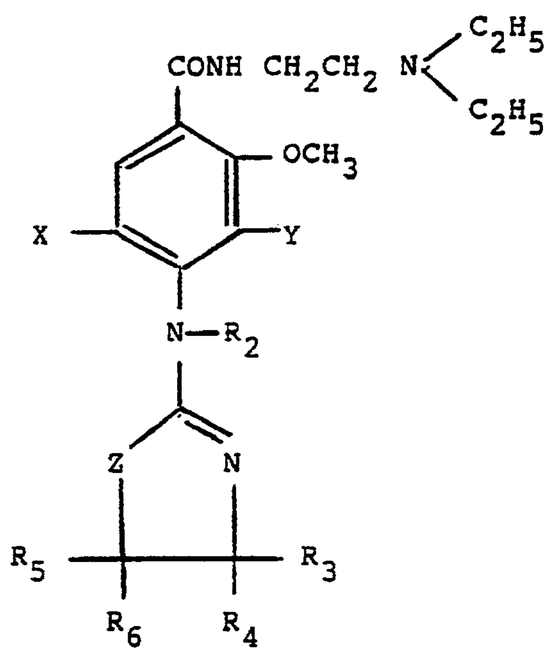

dans laquelle X, Y, Z, $R_2$, $R_3$, $R_4$, $R_5$, $R_6$, ont les mêmes significations que dans la revendication 1.

3. Procédé de préparation selon les revendications 1 et 2, de N-[2-(diéthylamino)éthyl] 2-méthoxy 4-[(1-H 4,5-dihydro 2-imidazolyl)] amino 5-chloro benzamide.

4. Procédé de préparation selon les revendications 1 et 2, de N-[2-(diéthylamino)ethyl]2-méthoxy 4-[N-éthyl-N-(1-H 4,5-dihydro 2-imidazolyl) amino]5-chloro benzamide

5. Procédé de préparation selon les revendications 1 et 2, de N- [2-(diéthylamino)éthyl]2-méthoxy 3,5-dichloro 4-[(1-H 4,5-dihydro 2-imidazolyl) amino] benzamide

6. Procédé de préparation selon les revendications 1 et 2, de N-2-(diéthylamino)éthyl]2-méthoxy 4-[(1-H 4,5-dihydro 4-diméthyl 2-imidazolyl) amino]5-chloro benzamide

7. Procédé de préparation selon les revendications 1 et 2, de N-[2-(diéthylamino)éthyl]2-méthoxy 4-[(4,5-dihydro 1,3-thiazol 2-yl) amino]5-chloro benzamide.

8. Procédé de préparation selon les revendications 1 et 2, de N-[2-(diéthylamino)éthyl]2-méthoxy 4-[(4,5-dihydro 2-oxazolyl) amino]5-chloro benzamide

9. Procédé de préparation selon la revendication 1, d'un composé de formule :

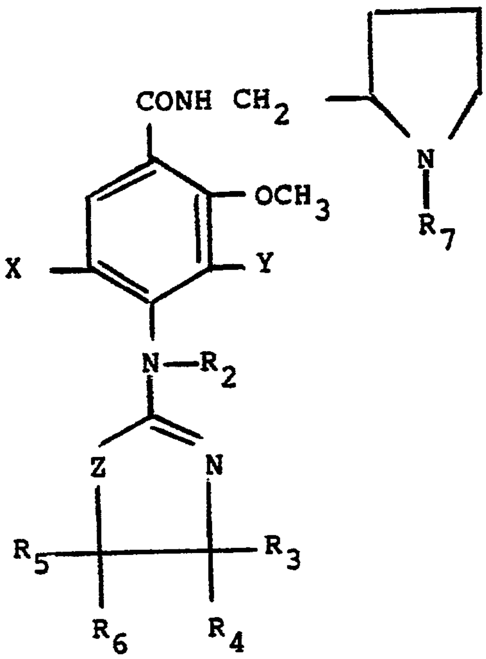

dans laquelle X, Y, Z, $R_2$, $R_3$, $R_4$, $R_5$, $R_6$, $R_7$ ont les mêmes significations que dans la revendication 1.

10. Procédé de préparation selon les revendications 1 et 9, de N-[(1-éthyl 2-pyrrolidinyl) méthyl]2-méthoxy 3,5-dibromo 4[(1-H 4,5-dihydro 2-imidazolyl)] amino benzamide

11. Procédé de préparation selon les revendications 1 et 9, de N-[(1-cyclohexenylméthyl 2-pyrrolidinyl) méthyl]2-méthoxy 4-[(1-H 4,5-dihydro 2-imidazolyl) amino]5-chlorobenzamide

12. Procédé de préparation selon les revendications 1 et 9, de N-[(1-cyclopropylméthyl 2-pyrrolidinyl) méthyl]2-méthoxy 4-[(1-H 4,5-dihydro 2-imidazolyl) amino]5-chlorobenzamide

13. Procédé de préparation selon les revendications 1 et 9, de N-(1-allyl 2-pyrrolidinyl) méthyl] 2-méthoxy 4-[(1-H 4,5-dihydro 2-imidazolyl) amino]5-chloro benzamide

14. Procédé de préparation selon les revendications 1 et 9, de N-[(1-éthyl 2-pyrrolidinyl) méthyl] 2-méthoxy 4-[(4,5-dihydro 1,3-thiazol 2-yl) amino]5-chloro benzamide.

15. Procédé de préparation selon les revendications 1 et 9, de N-[(1-cyclopropylméthyl 2-pyrrolidinyl) méthyl] 2-méthoxy 4-[(4,5-dihydro 2-oxazolyl) amino]5- chloro benzamide.

16. Procédé de préparation selon la revendication 1, de N-(allyl) 2-méthoxy 4-[(1-H 4,5-dihydro 2-imidazolyl) amino]5-chlorobenzamide

17. Procédé de préparation des composés de formule (I), qui consiste à transformer un 4-amino 5 (ou 3,5) - halogéno benzoate d'alkyle de formule:

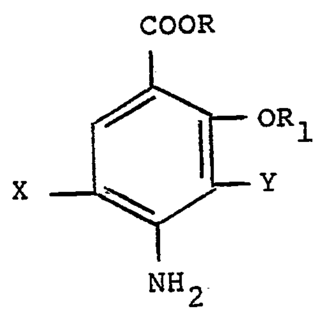

dans laquelle R représente un groupe alkyle et $R_1$, X et Y sont définis comme dans la revendication 1, en 4-isothiocyanato benzoate d'alkyle de formule (IV):

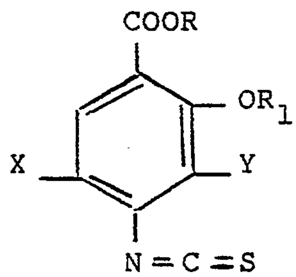

(IV)

puis en dérivé 4-(N'-amino (ou hydroxy)alkyl) thiouréido de formule (V) :

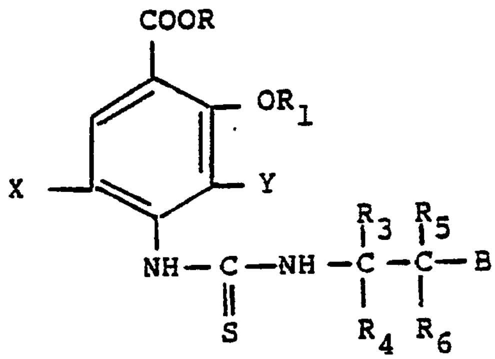

(V)

dans laquelle B représente un groupe amino ou hydroxy,
puis en dérivé 4-[(4,5-dihydro 2-imidazolyl (ou 2-thiazolyl ou 2-oxazolyl) amino] de formule (VI) :

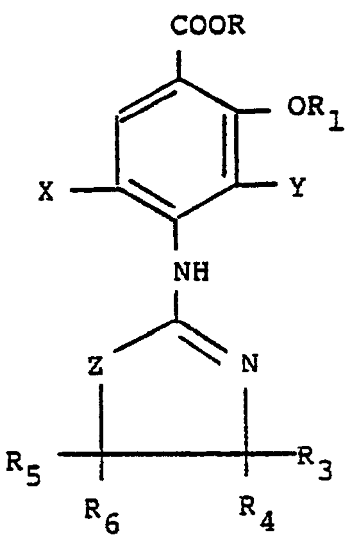

(VI)

et enfin à amidifier le composé de formule (VI) pour obtenir le composé de formule (I) correspondant ou à amidifier le composé de formule (VI) pour obtenir un composé de formule (I) dans laquelle $R_2$ est un atome d'hydrogène et à le transformer en composé de formule (I) dans laquelle $R_2$ est un groupe alkyle ou alcényle.

18. Procédé de préparation des composés de formule (I) où $R_2 \neq H$, dans lequel le dérivé 4-[(4,5-dihydro 2-imidazolyl (ou 2-thiazolyl ou 2-oxazolyl) amino] de formule (VI):

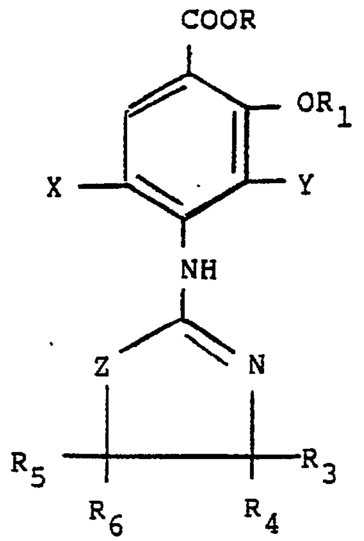

(VI)

est transformé en dérivé de formule (VI bis):

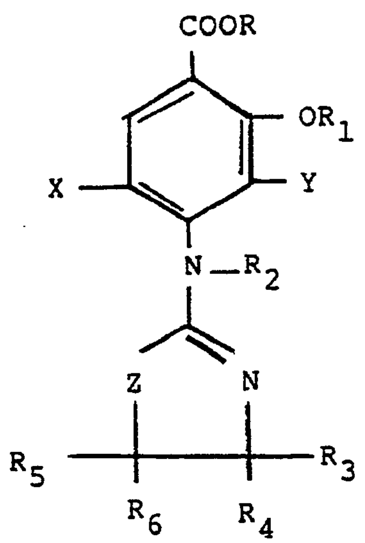

(VI bis)

avec $R_2$ = alkyle ou alcényle
qui est ensuite amidifié pour obtenir le composé de formule (I) correspondant.

19. Procédé de préparation des composés de formule (I) dans laquelle Z représente un groupe NH, qui consiste à transformer un 4-amino 5 (ou 3,5)-halogénobenzoate d'alkyle de formule:

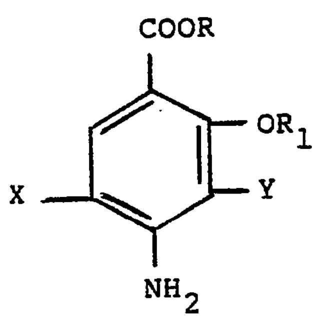

dans laquelle R représente un groupe alkyle et $R_1$, X et Y sont définis comme dans la revendication 1, en 4-formylamino benzoate d'alkyle de formule (VII):

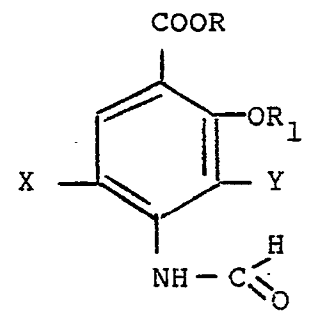

(VII)

puis en 4-dichloroformylimino benzoate d'alkyle de formule (VIII):

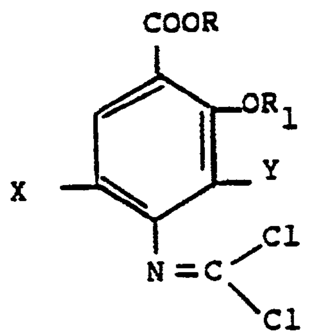

(VIII)

puis en 4-[(1-H 4,5-dihydro 2-imidazolyl) amino] benzoate de formule (VI) dans laquelle Z représente un groupe NH, et enfin à amidifier le composé de formule (VI) pour obtenir le composé de formule (I) correspondant ou à amidifier le composé de formule (VI) pour obtenir un composé de formule (I) dans laquelle $R_2$ est un atome d'hydrogène et à le transformer en composé de formule (I) dans laquelle $R_2$ est un groupe alkyle ou alcényle.

20. Procédé de préparation des composés de formule (I), dans lequel le composé de formule (VI) est transformé en dérivé de formule (VI bis) dans laquelle Z représente un groupe NH et $R_2$ représente un groupe alkyle ou alcényle, qui est ensuite amidifié pour obtenir le composé de formule (I) correspondant.

21. Procédé de préparation d'une composition pharmaceutique qui consiste à incorporer au moins l'un des composés obtenus selon l'une quelconque des revendications 1 à 20, à un excipient pharmaceutiquement acceptable.

**Claims for the contracting states: BE, CH, LI, DE, FR, GB, IT, LU, NL, SE**

1. New substituted benzamides of general formula (I)

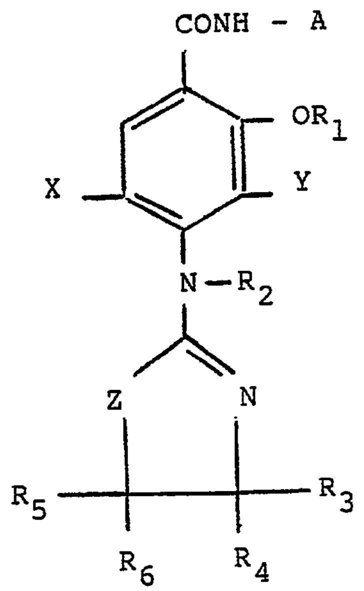

(I)

wherein:
– A represents an alkyl, alkenyl, diethylaminoethyl group or a group of the formula:

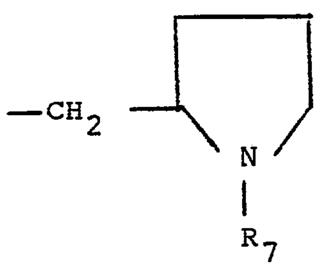

$R_7$ being a hydrogen atom or an alkyl, alkenyl, aryl, aralkyl, cycloalkyl, cycloalkylalkyl, cycloalkenyl or cycloalkenylalkyl group,
– $R_1$ and $R_2$ represent hydrogen atoms or alkyl or alkenyl groups
– $R_3$, $R_4$, $R_5$ and $R_6$ represent hydrogen atoms or alkyl groups
– X represents a chlorine or bromine atom
– Y represents a hydrogen atom or a chlorine or bromine atom
– Z represents an NH group or an oxygen or sulphur atom, and where the alkyl, cycloalkyl, alkenyl and cycloalkenyl residues contain a maximum of six carbon atoms.
2. In accordance with Claim 1, a compound of the formula:

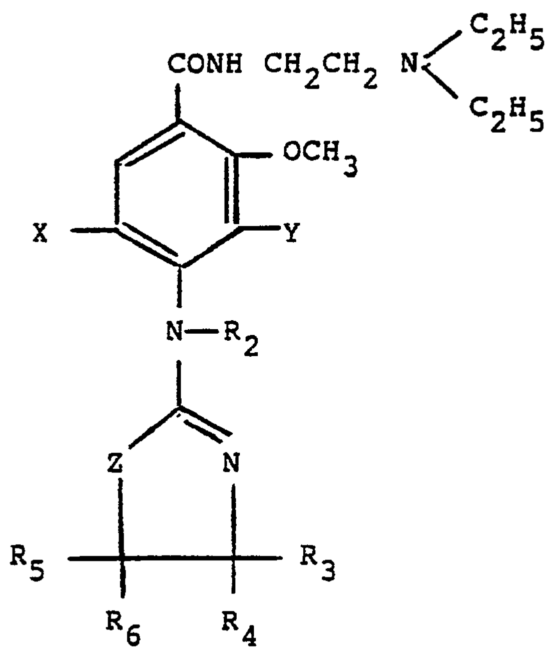

wherein X, Y, Z, $R_2$, $R_3$, $R_4$, $R_5$, $R_6$ hav the same meaning as in Claim 1.

3. In accordance with Claims 1 and 2, N-[2-(diethylamino)ethyl] 2-methoxy 4-[(1-H 4,5-dihydro 2-imidazolyl) amino] 5-chloro benzamide.

4. In accordance with Claims 1 and 2, N-[2-(diethylamino)ethyl] 2-methoxy 4-[N-ethyl-N-(1-H-4,5-dihydro 2-imidazolyl) amino] 5-chloro benzamide.

5. In accordance with Claims 1 and 2, N-[2-(diethylamino)ethyl] 2-methoxy 3,5-dichloro 4-[(1-H-4,5-dihydro 2-imidazolyl) amino] benzamide.

6. In accordance with Claims 1 and 2, N-[2-(diethylamino)ethyl] 2-methoxy 4-[(1-H 4,5-dihydro 4-dimethyl 2-imidazolyl) amino] 5-chloro benzamide.

7. In accordance with Claims 1 and 2, N-[2-(diethylamino)ethyl] 2-methoxy 4-[(4,5-dihydro, 1,3-thiazol 2-yl) amino] 5-chloro benzamide.

8. In accordance with Claims 1 and 2, N-[2-(diethylamino)ethyl] 2-methoxy 4-[(4,5-dihydro 2-oxazolyl) amino] 5-chloro benzamide.

9. In accordance with Claim 1, a compound of the formula:

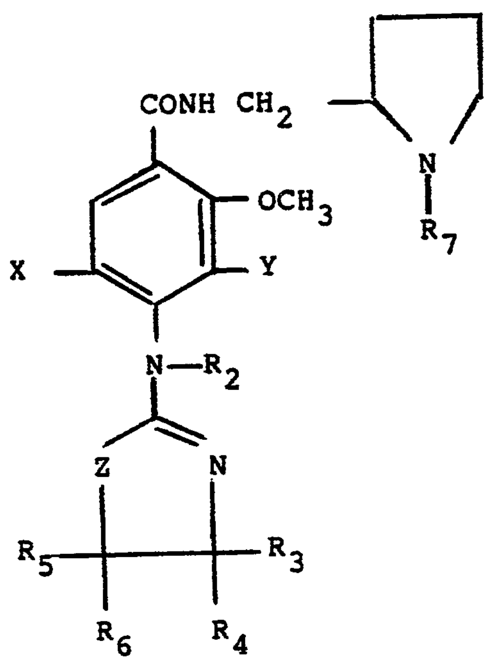

wherein X, Y, Z, $R_2$, $R_3$, $R_4$, $R_5$, $R_6$, $R_7$ have the same meaning as in Claim 1.

10. In accordance with Claims 1 and 9, N-[(1-ethyl 2-pyrrolidinyl)methyl] 2-methoxy 3,5-dibromo 4-[(1-H 4,5-dihydro 2-imidazolyl) amino] benzamide.

11. In accordance with Claims 1 and 9, N-[1-(1-cyclohexenylmethyl 2-pyrrolidinyl)methyl] 2-methoxy 4-[(1-H 4,5-dihydro 2-imidazolyl) amino] 5-chloro benzamide.

12. In accordance with Claims 1 and 9, N-[(1-cyclopropylmethyl 2-pyrrolidinyl)methyl] 2-methoxy 4-[(1-H 4,5-dihydro 2-imidazolyl) amino] 5-chloro benzamide.

13. In accordance with Claims 1 and 9, N-[(1-allyl 2-pyrrolidinyl)methyl] 2-methoxy 4-[(1-H 4,5-dihydro 2-imidazolyl) amino] 5-chloro benzamide.

14. In accordance with Claims 1 and 9, N-[(1-ethyl 2-pyrrolidinyl)methyl] 2-methoxy 4-[4,5-dihydro 1,3-thiazol 2-yl) amino] 5-chloro benzamide.

15. In accordance with Claims 1 and 9, N-[(1-cyclopropylmethyl 2-pyrrolidinyl)methyl] 2-methoxy 4-[(4,5-dihydro 2-oxazolyl) amino] 5-chloro benzamide.

16. In accordance with Claim 1, N-(allyl) 2-methoxy 4-[(1-H 4,5-dihydro 2-imidazolyl) amino] 5-chloro benzamide.

17. A method of preparing compounds in accordance with any of Claims 1 to 16, comprising treating an acid of formula (II):

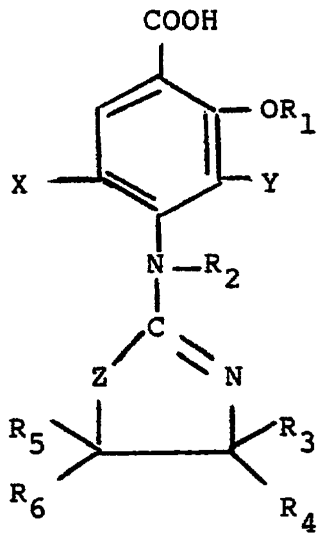

(II)

wherein $R_1$, $R_2$, $R_3$, $R_4$, $R_5$, $R_6$, X, Y and Z have the same meaning as in Claim 1, or one of its reactive derivatives, with an amine of formula (III):

$A{-}NH_2$ (III)

wherein A is defined as in Claim 1, or one of its reactive derivatives.

18. A method of preparing formula (I) compounds as defined in any of Claims 1 to 16, comprising converting an alkyl 4-amino 5 (or 3,5)-halo benzoate of the formula:

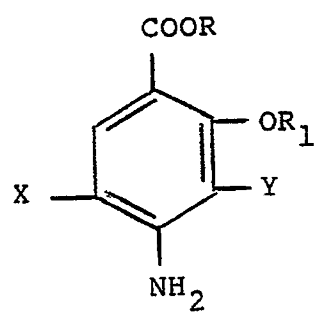

where R represents an alkyl group and $R_1$, X and Y are defined as in Claim 1, to alkyl 4-isothiocyanato benzoate of formula (IV):

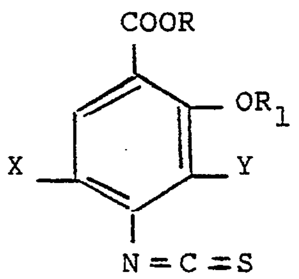

(IV)

then to 4-(N'-amino (or hydroxy) alkyl) thioureido derivative of formula (V):

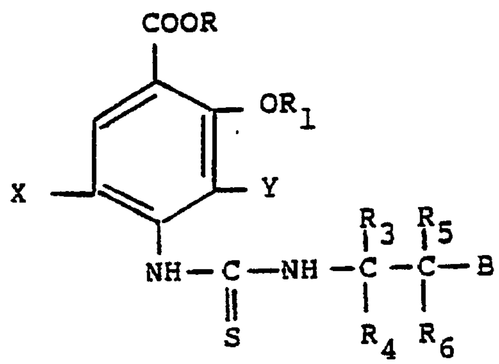

(V)

where B represents an amino or hydroxy group,
then to 4-[4,5-dihydro 2-imidazolyl (or 2-thiazolyl or 2-oxazolyl) amino] derivative of formula (VI):

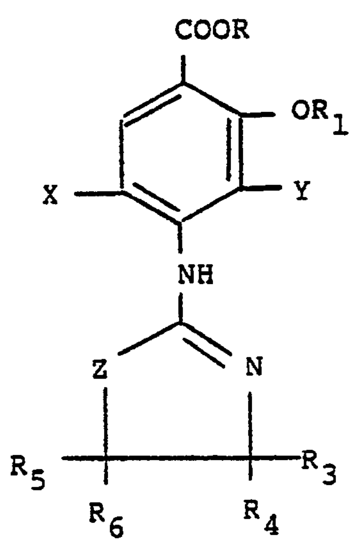

(VI)

and finally amidifying the formula (VI) compound to obtain the corresponding formula (I) compound, or amidifying the formula (VI) compound to obtain a formula (I) compound where $R_2$ is a hydrogen atom, and converting it to formula (I) compound where $R_2$ is an alkyl or alkenyl group.

19. A method of preparing formula (I) compounds in which $R_2 \neq H$, wherein the 4-[(4,5-dihydro 2-imidazolyl (or 2-thiazolyl or 2-oxazolyl) amino] derivative of formula (VI):

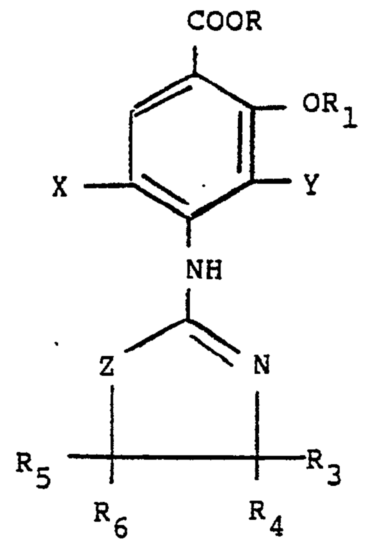

(VI)

is converted to formula (VIa) derivative

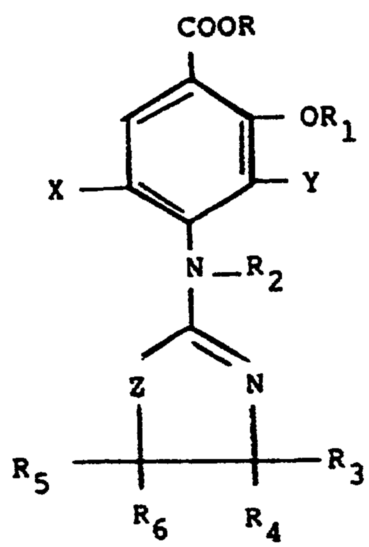

(VIa)

with $R_2$ = alkyl or alkenyl which is then amidified to give the corresponding formula (I) compound.

20. As new intermediate products in synthesis, according to Claim 18, alkyl 4-isothiocyanato benzoates of formula (IV).

21. As new intermediate products in synthesis, according to Claim 18, alkyl 4-N')-amino (or hydroxy) alkyl) thioureid benzoates of formula (V).

22. As new intermediate products in synthesis, according to Claim 18, alkyl 4-[4,5-dihydro 2-thiazolyl (or 2-oxazolyl) amino] benzoates of formula (VI).

23. As new intermediate products in synthesis, according to Claim 19, alkyl 4-[4,5-dihydro 2-thiazolyl (or 2-oxazolyl) amino] benzoates of formula (VIa).

24. A method of preparing formula (I) compounds where Z represents an NH group, comprising converting an alkyl 4-amino 5 (or 3,5)-halo benzoate of the formula:

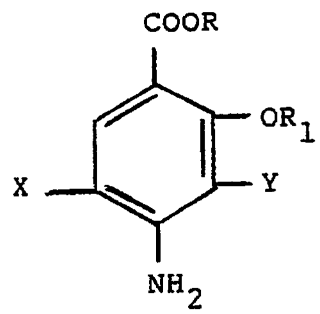

where R represents an alkyl group and $R_1$, X and Y are defined as in Claim 1, to alkyl 4-formylamino benzoate of formula (VII):

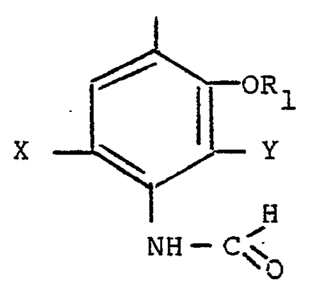

(VII)

then to alkyl 4-dichlorformylimino benzoate of formula (VIII):

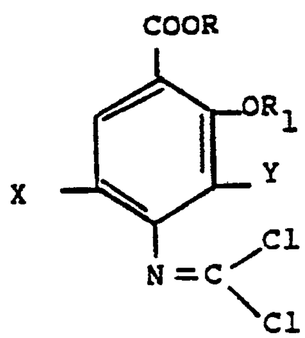

(VIII)

then to alkyl 4-[(1-H 4,5-dihydro 2-imidazolyl amino] benzoate of formula (VI) where Z represents an NH group,
and finally amidifying the formula (VI) compound to obtain the corresponding formula (I) compound or amidifying the formula (VI) compound to obtain a formula (I) compound where $R_2$ is a hydrogen atom, and converting it to formula (I) compound where $R_2$ is an alkyl or alkenyl group.

25. A method of preparing formula (I) compounds, wherein the formula (VI) compound is converted to formula (VIa) derivative in which Z represents an NH group and $R_2$ represents an alkyl or alkenyl group, and finally amidifying the formula (VIa) compound to obtain the formula (I) compound.

26. As new intermediate products in synthesis, according to Claim 24, alkyl 4-dichloroformylimino benzoates of formula (VIII).

27. As new intermediate products in synthesis, according to Claims 18, 19, 24, and 25, alkyl 4-[(1-H 4,5-dihydro 2-imidazolyl) amino] benzoates of formula (VI) where Z represents an NH group.

28. As new intermediate products in synthesis, according to Claims 19 and 25, alkyl 4-[(1H-4,5-dihydro 2-imidazolyl) amino] benzoates of formula (VIa) where Z represents an NH group.

29. As new medicaments, the compounds of any of Claims 1 to 16.

30. Pharmaceutical compositions, characterised in that they contain, as the active principle, at least one of the compounds according to any of Claims 1 to 16, together with pharmaceutically acceptable excipients.

**Claims for the contracting States: AT, ES, GR**

1. A method of preparing new substituted benzamides of general formula (I)

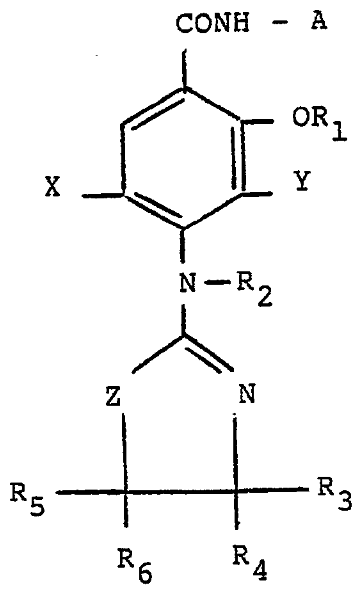

(I)

wherein:
– A represents an alkyl, alkenyl, diethylaminoethyl group or a group of the formula:

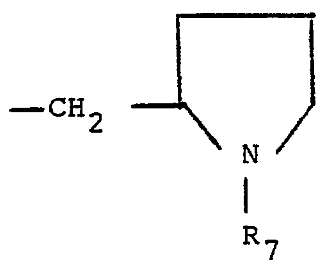

$R_7$ being a hydrogen atom or an alkyl, alkenyl, aryl, aralkyl, cycloalkyl, cycloalkylalkyl, cycloalkenyl or cycloalkenylalkyl group,
– $R_1$ and $R_2$ represent hydrogen atoms or alkyl or alkenyl groups
– $R_3$, $R_4$, $R_5$ and $R_6$ represent hydrogen atoms or alkyl groups
– X represents a chlorine or bromine atom
– Y represents a hydrogen, chlorine or bromine atom
– Z represents an HN group or an oxygen or sulphur atom,
and wherein the alkyl, cycloalkyl, alkenyl and cycloalkenyl residues contain a maximum of six carbon atoms,
and their optical isomers and physiologically acceptable salts of addition,
comprising treating an acid of formula (II)

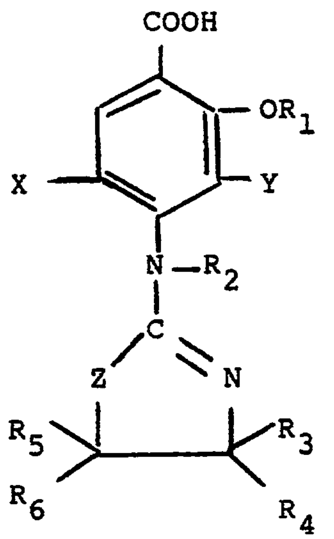

(II)

wherein $R_1$, $R_2$, $R_3$, $R_4$, $R_5$, $R_6$, X, Y and Z have the same meanings as above, or one of its reactive derivatives, with an amine of formula (III):

$A{-}NH_2$ (III)

wherein A is defined as above,

or one of its reactive derivatives.

2. The method of claim 1, for preparing a compound of the formula:

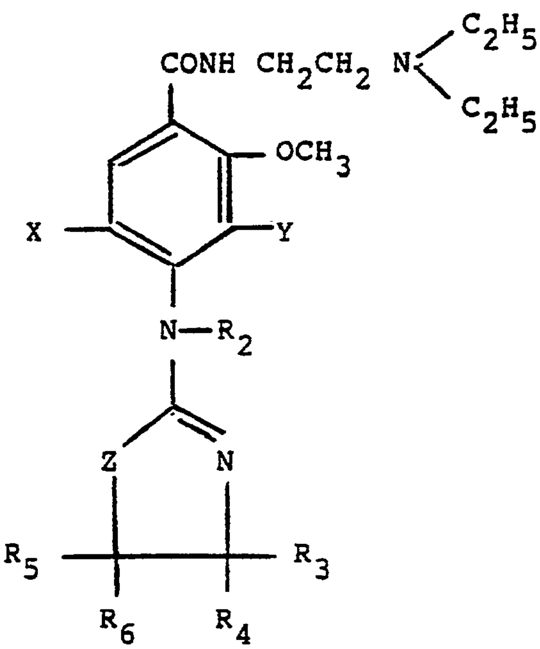

wherein X, Y, Z, $R_2$, $R_3$, $R_4$, $R_5$, $R_6$ have the same meaning as in Claim 1.

3. The method of claims 1 and 2, for preparing N-[2-(diethylamino)ethyl] 2-methoxy 4-[(1-H 4,5-dihydro 2-imidazolyl) amino] 5-chloro benzamide.

4. The method of claims 1 and 2 for preparing N-[2-(diethylamino)ethyl] 2-methoxy 4-[N-ethyl-N-(1-H 4,5-dihydro 2-imidazolyl) amino] 5-chloro benzamide.

5. The method of claims 1 and 2 for preparing N-[2-(diethylamino)ethyl] 2-methoxy 3,5-dichloro 4-[(1-H 4,5-dihydro 2-imidazolyl) amino] benzamide.

6. The method of claims 1 and 2, for preparing N-[2-(diethylamino)ethy] 2-methoxy4-l(1-H 4,5-dihydro 4-dimethyl 2-imidazolyl) amino] 5-chloro benzamide.

7. The method of claims 1 and 2, for preparing N-[2-(diethylamino)ethyl] 2-methoxy 4-[(4,5-dihydro, 1,3-thiazol 2-yl) amino] 5-chloro benzamide.

8. The method of claims 1 and 2, for preparing N-[2-(diethylamino)ethyl] 2-methoxy 4-[(4,5-dihydro 2-oxazolyl) amino] 5-chloro benzamide.

9. The method of claim 1, for preparing a compound of the formula:

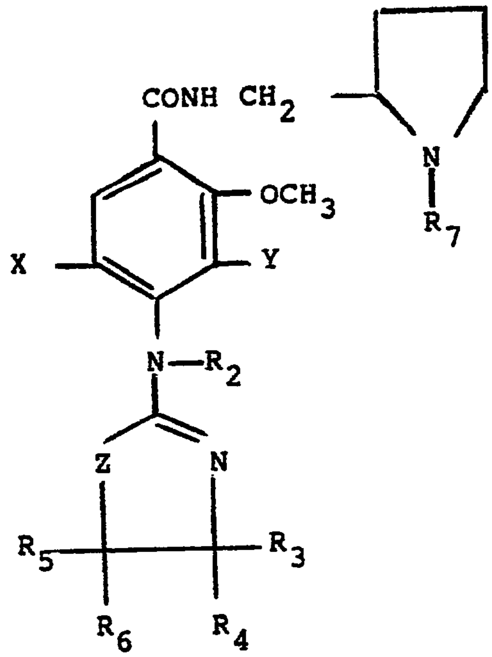

wherein X, Y, Z, $R_2$, $R_3$, $R_4$, $R_5$, $R_6$, $R_7$ have the same meaning as in Claim 1.

10. The method of claims 1 and 9,k for preparing N-[(1-ethyl 2-pyrrolidinyl)methyl] 2-methoxy 3,5-dibromo 4-[(1-H 4,5-dihydro 2-imidazolyl)] amino benzamide.

11. The method of claims 1 and 9, for preparing N-[(1-cyclohexenylmethyl 2-pyrrolidinyl)methyl] 2-methoxy 4-[(1-H 4,5-dihydro 2-imidazolyl) amino] 5-chloro benzamide.

12. The method of claims 1 and 9, for preparing N-[(1-cyclopropylmethyl 2-pyrrolidinyl)methyl] 2-methoxy 4-[(1-H 4,5-dihydro 2-imidazolyl) amino] 5-chloro benzamide.

13. The method of claims 1 and 9, for preparing N-[(1-allyl 2-pyrrolidinyl)methyl] 2-methoxy 4-[(1-H 4,5-dihydro 2-imidazolyl) amino] 5-chlor benzamide.

14. The method of claims 1 and 9, for preparing N-[(1-ethyl 2-pyrrolidinyl)methyl] 2-methoxy 4-[4,5-dihydro 1,3-thiazol 2-yl) amino] 5-chloro benzamide.

15. The method of claims 1 and 9, for preparing N-[(1-cyclopropylmethyl 2-pyrrolidinyl)methyl] 2-methoxy 4-[(4,5-dihydro 2-oxazolyl) amino] 5-chloro benzamide.

16. The method of claim 1, for preparing N-(allyl) 2-methoxy 4-[(1-H 4,5-dihydro 2-imidazolyl) amino] 5-chloro benzamide.

17. A method of preparing formula (I) compounds, converting an alkyl 4-amino 5 (or 3,5)-halo benzoate of the formula:

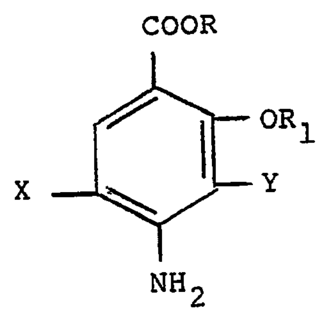

where R represents an alkyl group and $R_1$, X and Y are defined as in claim 1,

to alkyl 4-isothiocyanato benzoate of formula (IV):

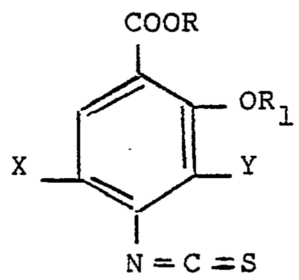

(IV)

then to 4-(N'-amino (or hydroxy) alkyl) thioureido derivative of formula (V):

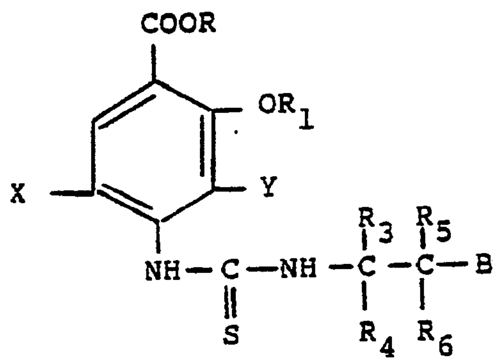

(V)

where B represents an amino or hydroxy group,
then to 4-[4,5-dihydro 2-imidazolyl (or 2-thiazolyl or 2-oxazolyl) amino] derivative of formula (VI):

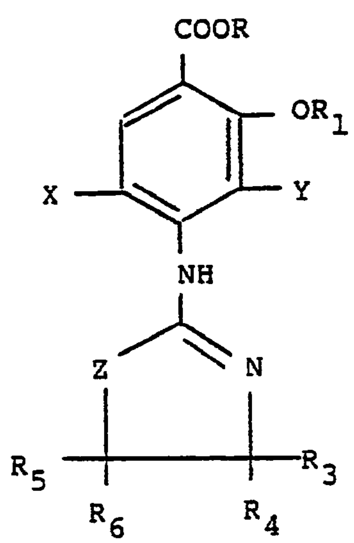

(VI)

and finally amidifying the formula (VI) compound to obtain the corresponding formula (I) compound, or amidifying the formula (VI) compound to obtain a formula (I) compound where $R_2$ is a hydrogen atom, and converting it to formula (I) compound where $R_2$ is an alkyl or alkenyl group.

18. A method of preparing formula (I) compounds in which $R_2 \neq H$, wherein the 4-[(4,5-dihydro 2-imidazolyl (or 2-thiazolyl or 2-oxazolyl) amino] derivative of formula (VI):

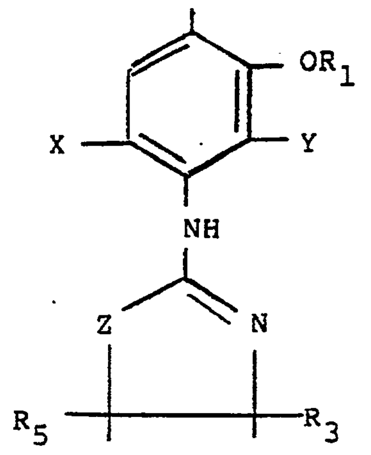

(VI)

is converted to formula (VIa) derivative

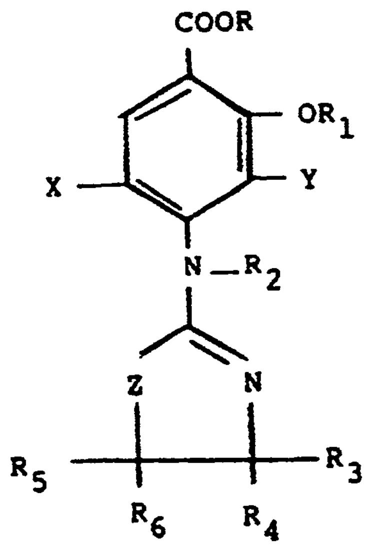

(VIa)

with $R_2$ = alkyl or alkenyl
which is then amidified to give the corresponding formula (I) compound.

19. A method of preparing formula (I) compounds where Z represents an NH group, comprising converting an alkyl 4-amino 5 (or 3,5)-halo benzoate of the formula:

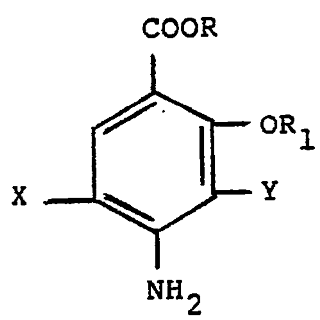

where R represents an alkyl group and $R_1$, X and Y are defined as in claim 1,
to alkyl 4-formylamino benzoate of formula (VII):

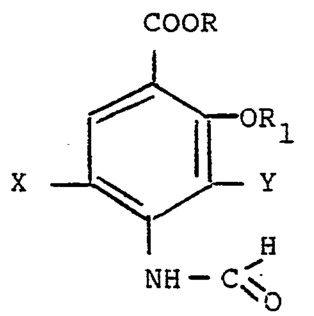

(VII)

then to alkyl 4-dichlorformylimino benzoate of formula (VIII):

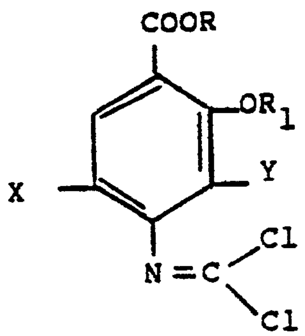

(VIII)

then to alkyl 4-[(1-H 4,5-dihydro 2-imidazolyl) amino] benzoate of formula (VI) where Z represents an NH group,
and finally amidifying the formula (VI) compound to obtain the corresponding formula (I) compound or amidifying the formula (VI) compound to obtain a formula (I) compound where $R_2$ is a hydrogen atom, and converting it to formula (I) compound where $R_2$ is an alkyl or alkenyl group.

20. A method of preparing formula (I) compounds, wherein the formula (VI) compound is converted to formula (VIa) derivative in which Z represents an NH group and $R_2$ represents an alkyl or alkenyl group, and finally amidifying the formula (VIa) compound to obtain the corresponding formula (I) compound.

21. A method of preparing a pharmaceutical composition, comprising incorporating at least one compound obtained according to any of claims 1 to 20, in a pharmaceutically acceptable excipient.

**Patentansprüche für die benannten Vertragsstaaten: BE, CH, LI, DE, FR, GB, IT, LU, NL, SE**

1. Neue substituierte Benzamide der allgemeinen Formel (I)

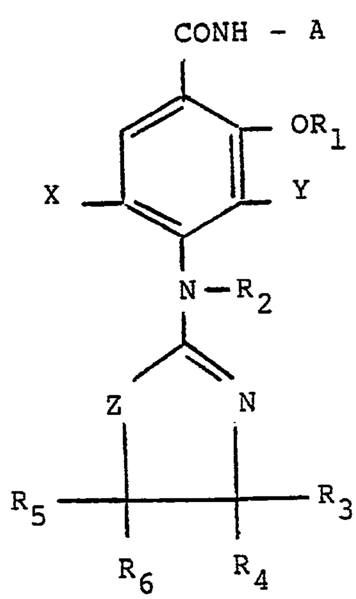

(I)

in der A eine Alkyl-, Alkenyl- oder Diethylaminoethylgruppe oder eine Gruppe der Formel

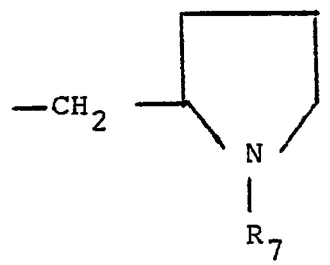

in welcher $R_7$ ein Wasserstoffatom oder eine Alkyl-, Alkenyl-, Aryl-, Aralkyl-, Cycloalkyl-, Cycloalkylalkyl-, Cycloalkenyl- oder Cycloalkenylalkylgruppe ist,
$R_1$ und $R_2$ Wasserstoffatome, Alkyl- oder Alkenylgruppen, $R_3$, $R_4$, $R_5$ und $R_6$ Wasserstoffatome oder Alkylgruppen, X ein Chlor- oder Bromatom, Y ein Wasserstoff-, Chlor- oder Bromatom und Z eine Gruppe NH, ein Sauerstoff- oder ein Schwefelatom bedeuten,
wobei die Werte Alkyl, Cycloalkyl, Alkenyl und Cycloalkenyl maximal bis zu 6 Kohlenstoffatome aufweisen,
sowie ihre optischen Isomere und physiologisch verträglichen Säureadditionssalze.

2. Substituierte Benzamide nach Anspruch 1 gemäß der Formel

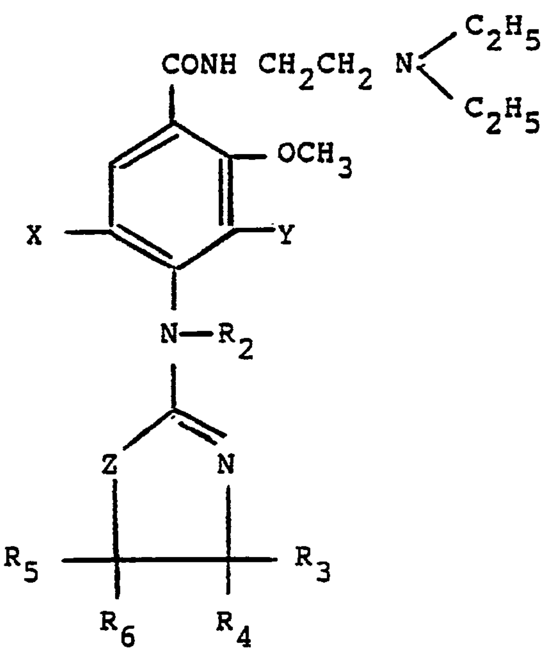

in der X, Y, Z, $R_2$, $R_3$, $R_4$, $R_5$ und $R_6$ wie in Anspruch 1 definiert sind.

3. N-[2-(Diethylamino)ethyl]-2-methoxy-4-[(1-H-4,5-dihydro-2-imidazolyl)amino]-5-chlorbenzamid gemäß Anspruch 1 oder 2.

4. N-[2-(Diethylamino)ethyl]-2-methoxy-4-[N-ethyl-N-(1-H-4,5-dihydro-2-imidazolyl)amino]-5-chlorbenzamid gemäß Anspruch 1 oder 2.

5. N-[2-(Diethylamino)ethyl]-2-methoxy-3,5-dichlor-4-[(1-H-4,5-dihydro-2-imidazolyl)amino]-benzamid gemäß Anspruch 1 oder 2.

6. N-[2-Diethylamino)ethyl]-2-methoxy-4-[(1-H-4,5-dihydro-4-dimethyl-2-imidazolyl)amino]-5-chlorbenzamid gemäß Anspruch 1 oder 2.

7. N-[2-(Diethylamino)ethyl]-2-methoxy-4-[(4,5-dihydro-1,3-thiazol-2-yl)amino]-5-chlorbenzamid gemäß Anspruch 1 oder 2.

8. N-[2-(Diethylamino)ethyl]-2-methoxy-4-[(4,5-dihydro-2-oxazolyl)amino]-5-chlorbenzamid gemäß Anspruch 1 oder 2.

9. Verbindung nach Anspruch 1 gemäß der Formel

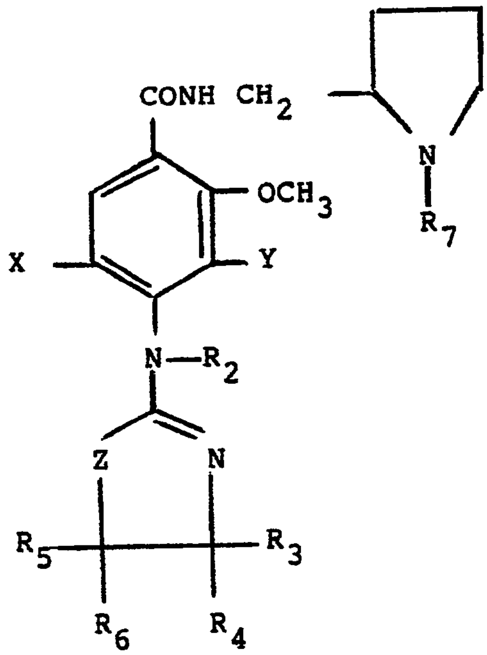

in der X, Y, Z, $R_2$, $R_3$, $R_4$, $R_5$, $R_6$ und $R_7$ wie in Anspruch 1 definiert sind.

10. N-[(1-Ethyl-2-pyrrolidinyl)-methyl]-2-methoxy-3,5-dibrom-4-[(1-H-4,5-dihydro-2-imidazolyl)-amino]-benzamid gemäß Anspruch 1 oder 9.

11. N-[(1-Cyclohexenylmethyl-2-pyrrolidinyl)methyl]-2-methoxy-4-[(1-H-4,5-dihydro-2-imidazolyl)amino]-5-chlorbenzamid gemäß Anspruch 1 oder 9.

12. N-[(1-Cyclopropylmethyl-2-pyrrolidinyl)methyl]-2-methoxy-4-[(1-H-4,5-dihydro-2-imidazolyl)amino]-5-chlorbenzamid gemäß Anspruch 1 oder 9.

13. N-[(1-Allyl-2-pyrrolidinyl)methyl]-2-methoxy-4-[(1-H-4,5-dihydro-2-imidazolyl)amino]-5-chlorbenzamid gemäß Anspruch 1 oder 9.

14. N-[(1-Ethyl-2-pyrrolidinyl)methyl]-2-methoxy-4-[(4,5-dihydro-1,3-thiazol-2-yl)amino]-5-chlorbenzamid gemäß Anspruch 1 oder 9.

15. N-[(1-Cyclopropylmethyl-2-pyrrolidinyl)methyl]-2-methoxy-4-[(4,5-dihydro-2-oxazolyl)amino]-5-chlorbenzamid gemäß Anspruch 1 oder 9.

16. N-(Allyl)-2-methoxy-4-[(1-H-4,5-dihydro-2-imidazolyl)-amino]-5-chlorbenzamid nach Anspruch 1.

17. Verfahren zur Herstellung der substituierten Benzamide gemäß einem der Ansprüche 1 bis 16, dadurch gekennzeichnet, daß man eine Säure der Formel (II)

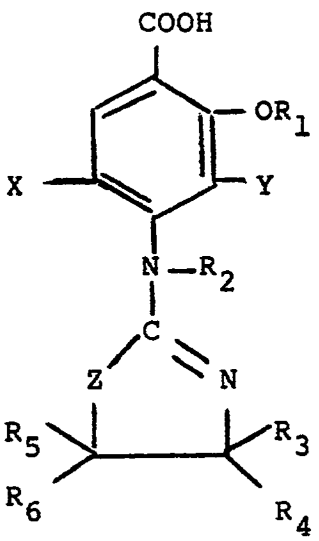

(II)

in der $R_1$, $R_2$, $R_3$, $R_4$, $R_5$, $R_6$, X, Y und Z wie in Anspruch 1 definiert sind, oder ein reaktives Derivat derselben mit einem Amin der Formel (III)

$A-NH_2$ (III)
in der A wie in Anspruch 1 definiert ist, oder ein reaktives Derivat desselben, umsetzt.

18. Verfahren zur Herstellung der Verbindung der Formel (I) gemäß einem der Ansprüche 1 bis 16, dadurch gekennzeichnet, daß man ein 4-Amino-5-(oder 3,5-)-halogenalkylbenzoat der Formel

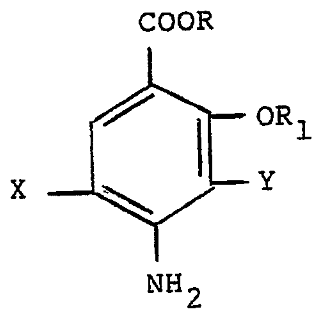

in der R eine Alkylgruppe bedeutet und $R_1$, X und Y wie in Anspruch 1 definiert sind, in ein 4-Isothiocyanatoalkylbenzoat der Formel (IV)

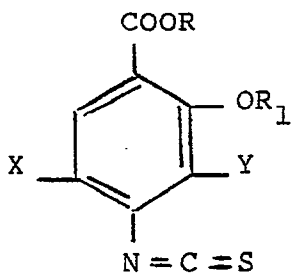

(IV)

dieses in ein 4-N'-Amino(oder Hydroxy)alkyl-thioharnstoffderivat der Formel (V)

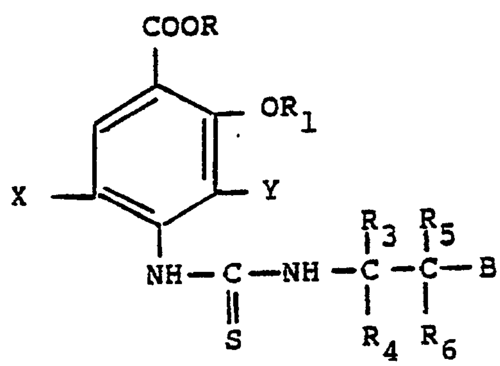

(V)

in der B eine Amino- oder Hydroxygruppe bedeutet, und dieses in ein 4-[(4,5-Dihydro-2-imidazolyl-(oder 2-thiazolyl- oder 2-oxazolyl-)amino]-Derivat der Formel (VI)

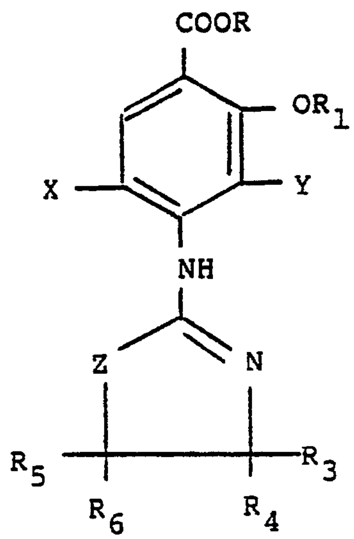

(VI)

umwandelt und schließlich die Verbindung der Formel (VI) für die Herstellung der entsprechende Verbindung der Formel (I) amidiert oder die Verbindung (VI) für die Herstellung einer Verbindung der Formel (I), in der $R_2$ ein Wasserstoffatom ist, amidiert und diese in eine Verbindung der Formel (I) überführt, in der $R_2$ eine Alkyl- oder Alkenylgruppe ist.

19. Verfahren zur Herstellung der Verbindung der Formel (I), in der $R_2$ ungleich H ist, in dem man das 4-[(4,5-Dihydro-2-imidazolyl-(oder 2-thiazolyl- oder 2-oxazolyl-)-amino]-Derivat der Formel (VI)

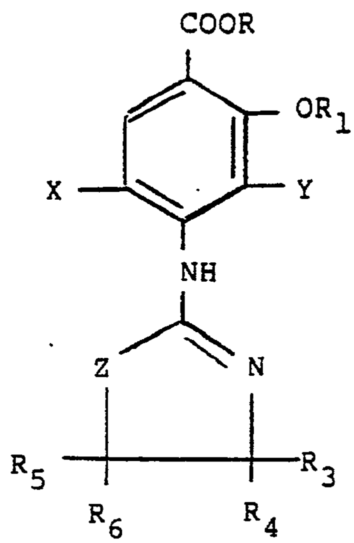

(VI)

in ein Derivat der Formel (VI')

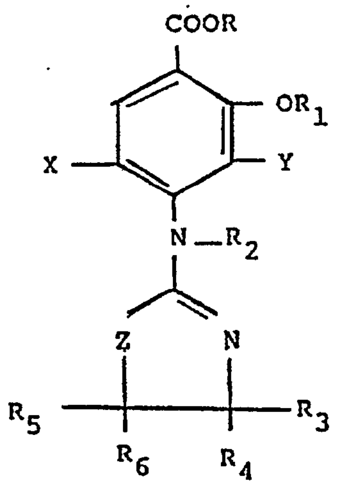

(VI')

mit $R_2$ = Alkyl oder Alkenyl umwandelt, das man anschließend unter Erhalt der entsprechenden Verbindung der Formel (I) amidiert.

20. 4-Isothiocyanatoalkylbenzoate der Formel (IV) als neue Zwischenprodukte für die Synthese gemäß Anspruch 18.

21. 4-[N'-Amino-(oder hydroxy)alkyl]-thioureido-alkylbenzoate der Formel (VI) als neue Zwischenprodukte für die Synthese gemäß Anspruch 18.

22. 4-[4,5-Dihydro-2-thiazolyl-(oder 2-oxazolyl)amino]alkylbenzoate der Formel (VI) als neue Zwischenprodukte für die Synthese nach Anspruch 18.

23. 4-[4,5-Dihydro-2-thiazolyl(oder 2-oxazolyl)amino]-alkylbenzoate der Formel (VI') als neue Zwischenprodukte für die Synthese nach Anspruch 19.

24. Verfahren zur Herstellung der Verbindung der Formel (I), in der Z eine Gruppe NH ist, dadurch gekennzeichnet, daß man ein 4-Amino-5-(oder 3,5)- halogen-alkylbenzoat der Formel

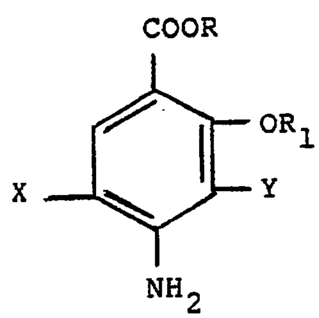

in der R eine Alkylgruppe ist und $R_1$, X und Y wie in Anspruch 1 definiert sind,
in ein 4-Formylamino-alkylbenzoat der Formel (VII)

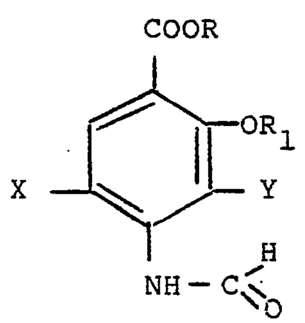

(VII)

dieses in ein 4-Dichlorformylimino-alkylbenzoat der Formel (VIII)

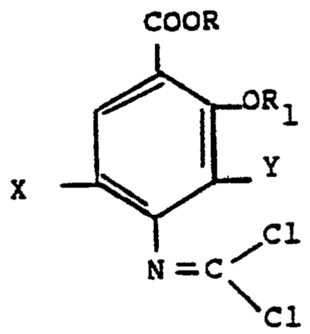

(VIII)

dieses in ein 4-[(1-H-4,5-dihydro-2-imidazolyl)amino]-benzoat der Formel (VI), in der Z eine Gruppe NH bedeutet, überführt und schließlich die Verbindung der Formel (VI) für die Herstellung der entsprechenden Verbindung der Formel (I) amidiert oder die Verbindung der Formel (VI) für die Herstellung einer Verbindung der Formel (I), in der $R_2$ ein Wasserstoffatom ist, amidiert und diese in eine Verbindung der Formel (I) überführt, in der $R_2$ eine Alkyl- oder Alkenylgruppe ist.

25. Verfahren zur Herstellung der Verbindung der Formel (I), in dem man eine Verbindung der Formel (VI) in ein Derivat der Formel (VI'), in der Z eine NH-Gruppe und $R_2$ eine Alkyl- oder Alkenylgruppe ist, überführt und diese zur Herstellung der Verbindung der Formel (I) amidiert.

26. 4-Dichlorformyliminoalkylbenzoate der Formel (VIII) als neue Zwischenprodukte für die Synthese gemäß Anspruch 24.

27. 4-[1-H-4,5-dihydro-2-imidazolyl)amino]-alkylbenzoate der Formel (VI), in der Z eine NH-Gruppe ist, als neue Zwischenprodukte für die Synthese gemäß den Ansprüchen 18, 19, 24 und 25.

28. 4-[(1-H-4,5-Dihydro-2-imidazolyl)amino]-alkylbenzoate der Formel (VI bis), in der Z eine NH-Gruppe bedeutet, als neue Zwischenprodukte für die Synthese gemäß den Ansprüchen 19 und 25.

29. Verbindungen nach einem der Ansprüche 1 bis 16 als neue Medikamente.

30. Pharmazeutische Zusammensetzungen, dadurch gekennzeichnet, daß sie als Wirkstoffe mindestens eine der Verbindungen gemäß einem jeden der Ansprüche 1 bis 16 zusammen mit pharmazeutisch verträglichen Exzipienten enthalten.

**Patentansprüche für die benannten Vertragsstaaten: AT, ES, GR**

1. Verfahren zur Herstellung neuer substituierter Benzamide der allgemeinen Formel (I)

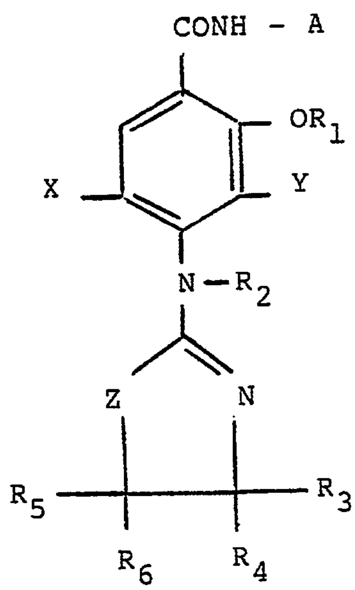

(I)

in der A eine Alkyl-, Alkenyl- oder Diethylaminoethylgruppe oder eine Gruppe der Formel

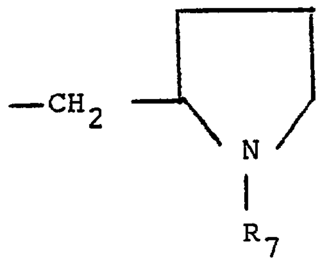

in welcher $R_7$ ein Wasserstoffatom oder eine Alkyl-, Alkenyl-, Aryl-, Aralkyl-, Cycloalkyl-, Cycloalkylalkyl-, Cycloalkenyl- oder Cycloalkenylalkylgruppe ist,
$R_1$ und $R_2$ Wasserstoffatome, Alkyl- oder Alkenylgruppen, $R_3$, $R_4$, $R_5$ und $R_6$ Wasserstoffatome oder Alkylgruppen, X ein Chlor- oder Bromatom, Y ein Wasserstoff-, Chlor- oder Bromatom und Z eine Gruppe NH, ein Sauerstoff- oder ein Schwefelatom bedeuten,
wobei die Werte Alkyl, Cycloalkyl, Alkenyl und Cycloalkenyl maximal bis zu 6 Kohlenstoffatome aufweisen,
sowie ihre optischen Isomere und physiologisch verträglichen Säureadditionssalze, dadurch gekennzeichnet, daß man eine Säure der Formel (II)

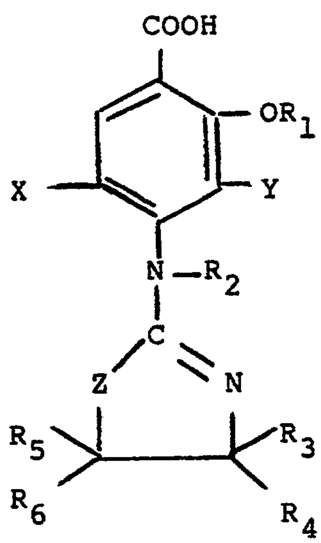

(II)

in der $R_1$, $R_2$, $R_3$, $R_4$, $R_5$, $R_6$, X, Y und Z wie oben definiert sind, oder ein reaktives Derivat derselben mit einem Amin der Formel (III)

A–$NH_2$ (III)

in der A wie oben definiert ist, oder ein reaktives Derivat desselben, umsetzt.

2. Verfahren nach Anspruch 1 zur Herstellung von substituierten Benzamiden gemäß der Formel

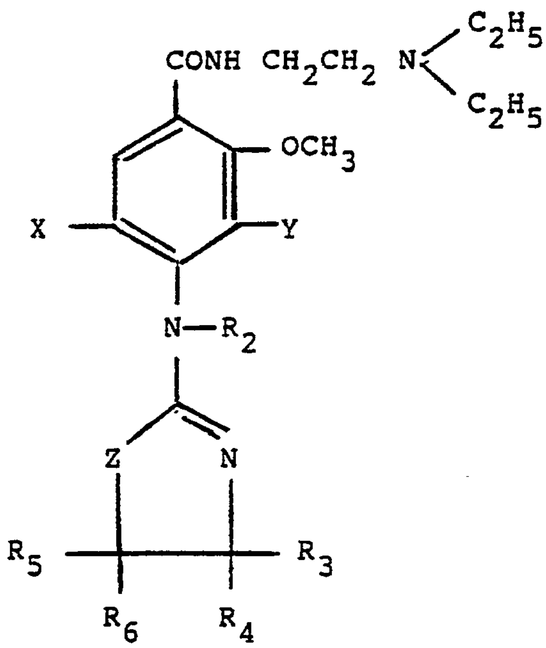

in der X, Y, Z, $R_2$, $R_3$, $R_4$, $R_5$ und $R_6$ wie in Anspruch 1 definiert sind.

3. Verfahren gemäß Anspruch 1 oder 2 zur Herstellung von N-[2-(Diethylamino)ethyl]-2-methoxy-4-[(1-H-4,5-dihydro-2-imidazolyl)amino]-5-chlorbenzamid.

4. Verfahren gemäß Anspruch 1 oder 2 zur Herstellung von N-[2-(Diethylamino)ethyl]-2-methoxy-4-[N-ethyl-N-(1-H-4,5-dihydro-2-imidazolyl)amino]-5-chlorbenzamid.

5. Verfahren gemäß Anspruch 1 oder 2 zur Herstellung von N-[2-(Diethylamino)ethyl]-2-methoxy-3,5-dichlor-4-[(1-H-4,5-dihydro-2-imidazolyl)amino]-benzamid.

6. Verfahren gemäß Anspruch 1 oder 2 zur Herstellung von N-[2-(Diethylamino)ethyl]-2-methoxy-4-[(1-H-4,5-dihydro-4-dimethyl-2-imidazolyl)amino]-5-chlorbenzamid.

7. Verfahren gemäß Anspruch 1 oder 2 zur Herstellung von N-[2-(Diethylamino)ethyl]-2-methoxy-4-[(4,5-dihydro-1,3-thiazol-2-yl)amino]-5-chlorbenzamid.

8. Verfahren gemäß Anspruch 1 oder 2 zur Herstellung von N-[2-(Diethylamino)ethyl]-2-methoxy-4-[(4,5-dihydro-2-oxazolyl)amino]-5-chlorbenzamid.

9. Verfahren nach Anspruch 1 zur Herstellung einer Verbindung gemäß der Formel

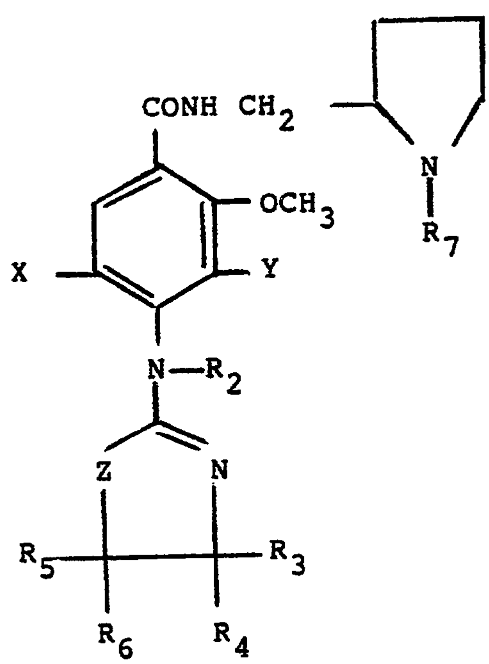

in der X, Y, Z, $R_2$, $R_3$, $R_4$, $R_5$, $R_6$ und $R_7$ wie in Anspruch 1 definiert sind.

10. Verfahren gemäß Anspruch 1 oder 9 zur Herstellung von N-[(1-Ethyl-2-pyrrolidinyl)-methyl]-2-methoxy-3,5-dibrom-4-[(1-H-4,5-dihydro-2-imidazolyl)-amino]-benzamid.

11. Verfahren gemäß Anspruch 1 oder 9 zur Herstellung von N-[(1-Cyclohexenylmethyl-2-pyrrolidinyl)methyl]-2-methoxy-4-[(1-H-4,5-dihydro-2-imidazolyl)amino]-5-chlorbenzamid.

12. Verfahren gemäß Anspruch 1 oder 9 zur Herstellung von N-[(1-Cyclohexenylmethyl-2-pyrrolidinyl)methyl]-2-methoxy-4-[(1-H-4,5-dihydro-2-imidazolyl)amino]-5-chlorbenzamid.

13. Verfahren gemäß Anspruch 1 oder 9 zur Herstellung von N-[(1-Allyl-2-pyrrolidinyl)methyl]-2-methoxy-4-[(1-H-4,5-dihydro-2-imidazolyl)amino]-5-chlorbenzamid.

14. Verfahren gemäß Anspruch 1 oder 9 zur Herstellung von N-[(1-Ethyl-2-pyrrolidinyl)methyl]-2-methoxy-4-[(4,5-dihydro-1,3-thiazol-2-yl)amino]-5-chlorbenzamid.

15. Verfahren gemäß Anspruch 1 oder 9 zur Herstellung von N-[(1-Cyclopropylmethyl-2-pyrrolidinyl)methyl]-2-methoxy-4-[(4,5-dihydro-2-oxazolyl)amino]-5-chlorbenzamid.

16. Verfahren nach Anspruch 1 zur Herstellung von N-(Allyl)-2-methoxy-4-[(1-H-4,5-dihydro-2-imidazolyl)-amino]-5-chlorbenzamid.

17. Verfahren zur Herstellung der Verbindungen der Formel (I), dadurch gekennzeichnet, daß man ein 4-Amino-5-(oder 3,5-)-halogenalkylbenzoat der Formel

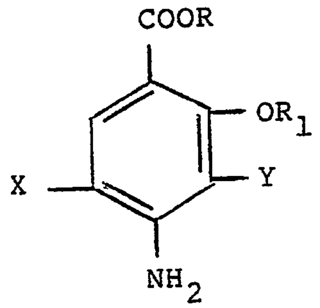

in der R eine Alkylgruppe bedeutet und $R_1$, X und Y wie in Anspruch 1 definiert sind, in ein 4-Isothiocyanatoalkylbenzoat der Formel (IV)

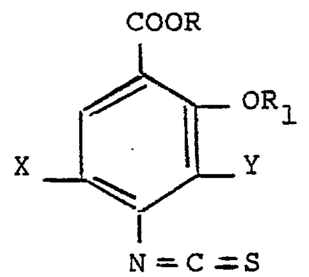

(IV)

dieses in ein 4-(N'-Amino(oder Hydroxy)alkyl-thioharnstoffderivat der Formel (V)

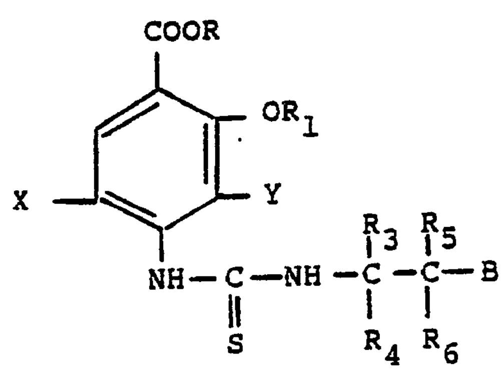

(V)

in der B eine Amino- oder Hydroxygruppe bedeutet, und dieses in ein 4-[(4,5-Dihydro-2-imidazolyl-(oder 2-thiazolyl- oder 2-oxazolyl-)amino]-Derivat der Formel (VI)

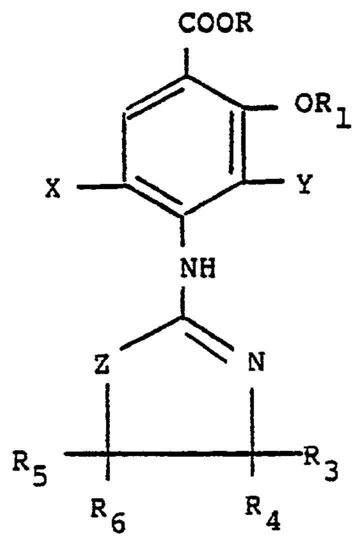

(VI)

umwandelt und schließlich die Verbindung der Formel (VI) für die Herstellung der entsprechenden Verbindung der Formel (I) amidiert oder die Verbindung (VI) für die Herstellung einer Verbindung der Formel (I), in der R2 ein Wasserstoffatom ist, amidiert und diese in einer Verbindung der Formel (I) überführt, in der $R_2$ eine Alkyl- oder Alkenylgruppe ist.

18. Verfahren zur Herstellung der Verbindungen der Formel (I), in der $R_2$ ungleich H ist, in dem man das 4-[(4,5-Dihydro-2-imidazolyl-(oder 2-thiazolyl- oder 2-oxazolyl-)-amino]-Derivat der Formel (VI)

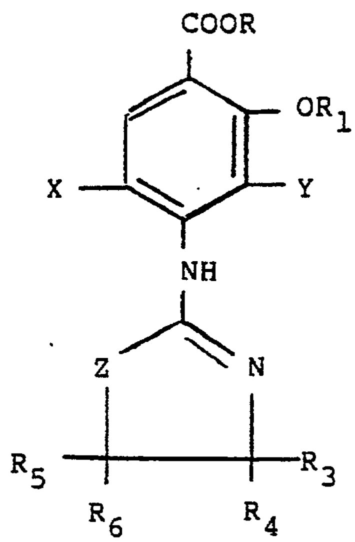

(VI)

in ein Derivat der Formel (VI')

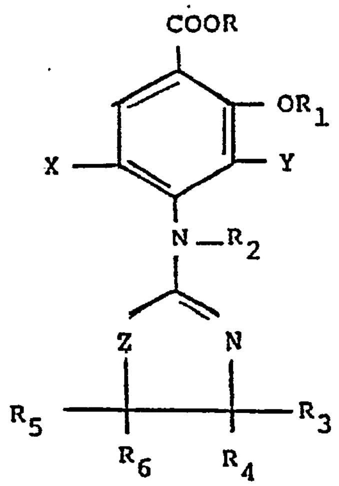

(VI')

mit $R_2$ = Alkyl oder Alkenyl umwandelt, das man anschließend unter Erhalt der entsprechenden Verbindung der Formel (I) amidiert.

19. Verfahren zur Herstellung der Verbindungen der Formel (I), in der Z eine Gruppe NH ist, dadurch gekennzeichnet, daß man ein 4-Amino-5-(oder 3,5)-halogen-alkylbenzoat oder Formel

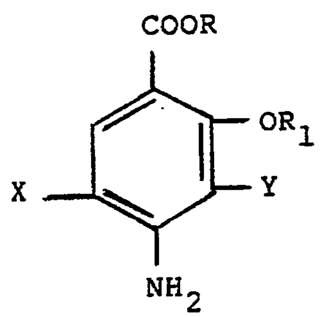

in der R eine Alkylgruppe ist und $R_1$, X und Y wie in Anspruch 1 defineirt sind,
in ein 4-Formylamino-alkylbenzoat der Formel (VII)

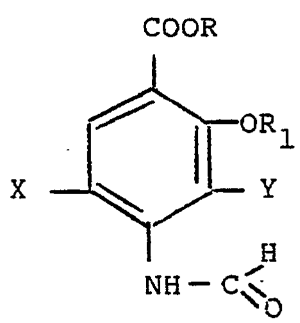

(VII)

dieses in ein 4-Dichlorformylimino-alkylbenzoat der Formel (VIII)

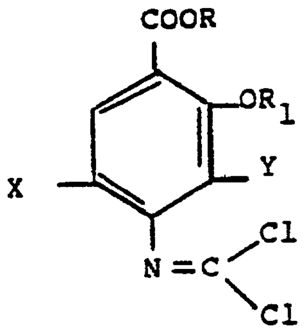

(VIII)

dieses in ein 4-[(1-H-4,5-dihydro-2-imidazolyl)amino]-benzoat der Formel (VI), in der Z eine Gruppe NH bedeutet, überführt und schließlich die Verbindung der Formel (VI) für die Herstellung der entsprechenden Verbindung der Formel (I) amidiert oder die Verbindung der Formel (VI) für die Herstellung einer Verbindung der Formel (I), in der $R_2$ ein Wasserstoffatom ist, amidiert und diese in eine Verbindung der Formel (I) überführt, in der $R_2$ eine Alkyl- oder Alkenylgruppe ist.

20. Verfahren zur Herstellung der Verbindung der Formel (I), in dem man eine Verbindung der Formel (VI) in ein Derivat der Formel (VI'), in der Z eine NH-Gruppe und $R_2$ eine Alkyl- oder Alkenylgruppe ist, überführt und diese zur Herstellung der Verbindung der Formel (I) amidiert.

21. Verfahren zur Herstellung einer pharmazeutischen Zusammensetzung, dadurch gekennzeichnet, daß man mindestens eine der Verbindungen, erhalten gemäß einem jeden der Ansprüche 1 bis 20, einem pharmazeutisch verträglichen Exzipienten zusetzt.

ACTIVITE MOTRICE (EN % PAR RAPPORT AU TEMOIN)
EN FONCTION DE LA DOSE

☆ = faiblement activant
A = activation
Q = sédation

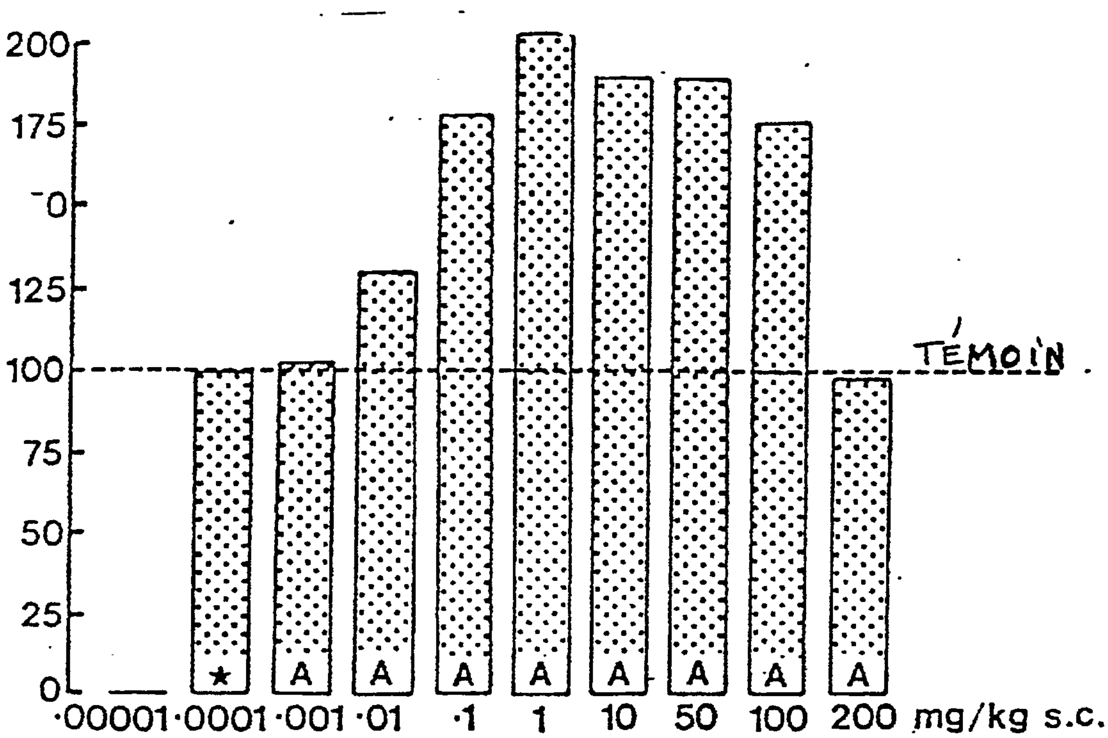


FIG. 1

COMPOSE

Exemple I

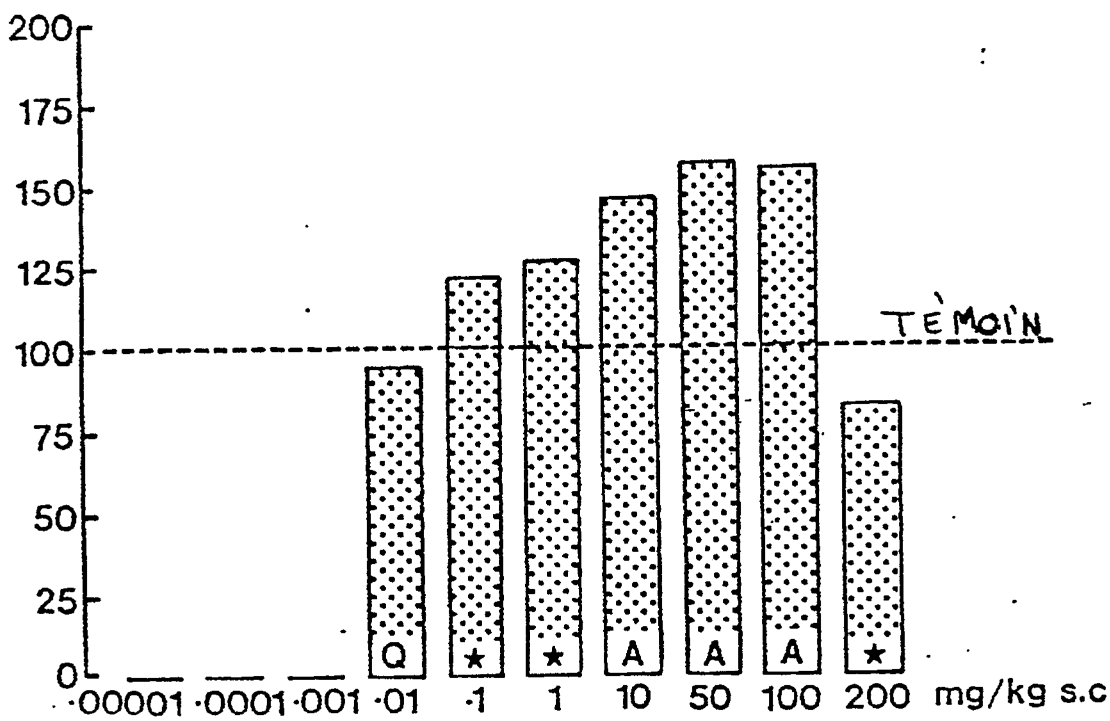


FIG. 2

COMPOSE

Exemple I'

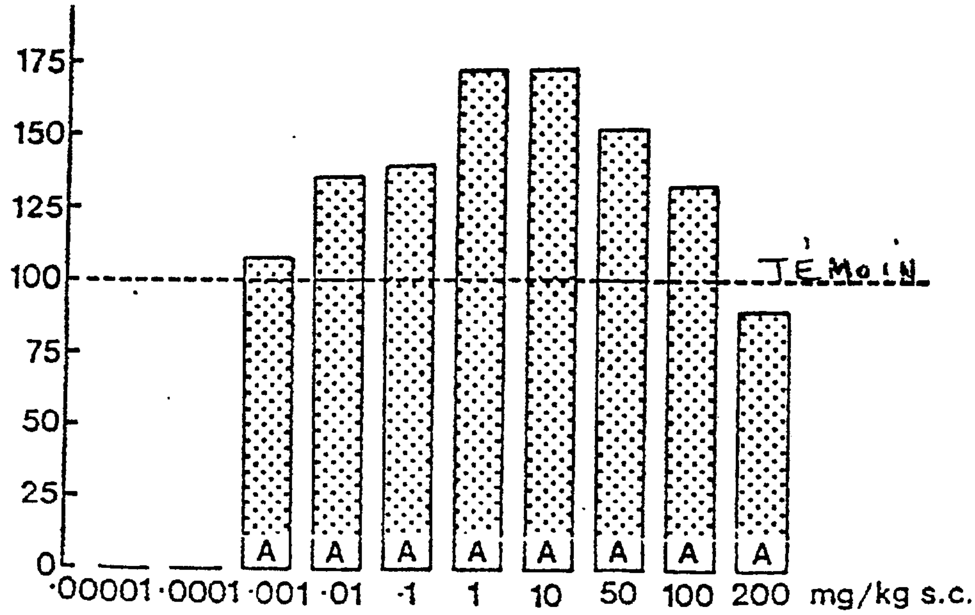


FIG. 3

COMPOSE

Exemple V

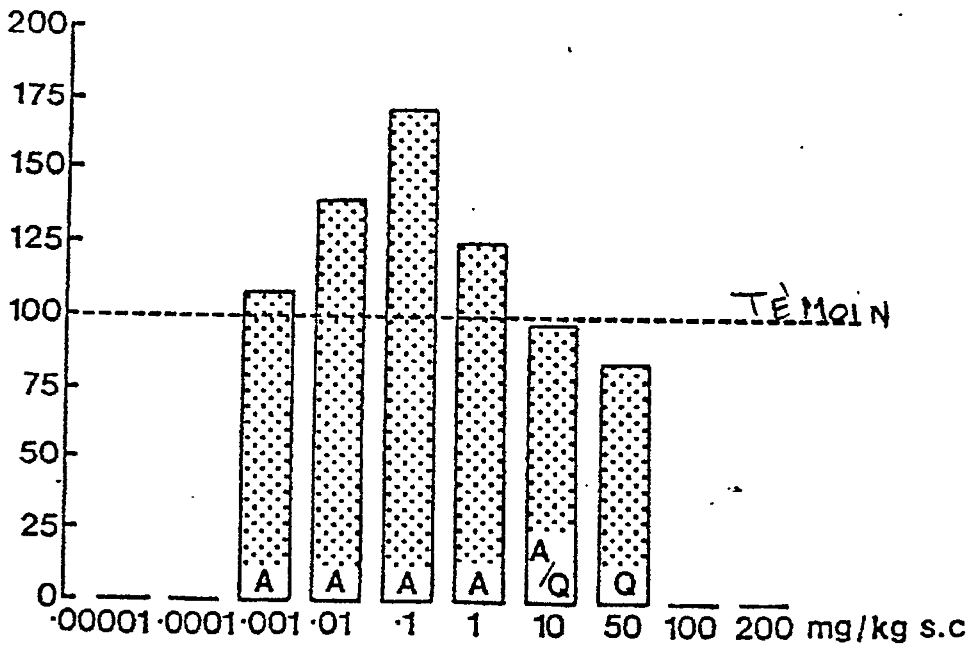


FIG. 4

COMPOSE

Exemple II

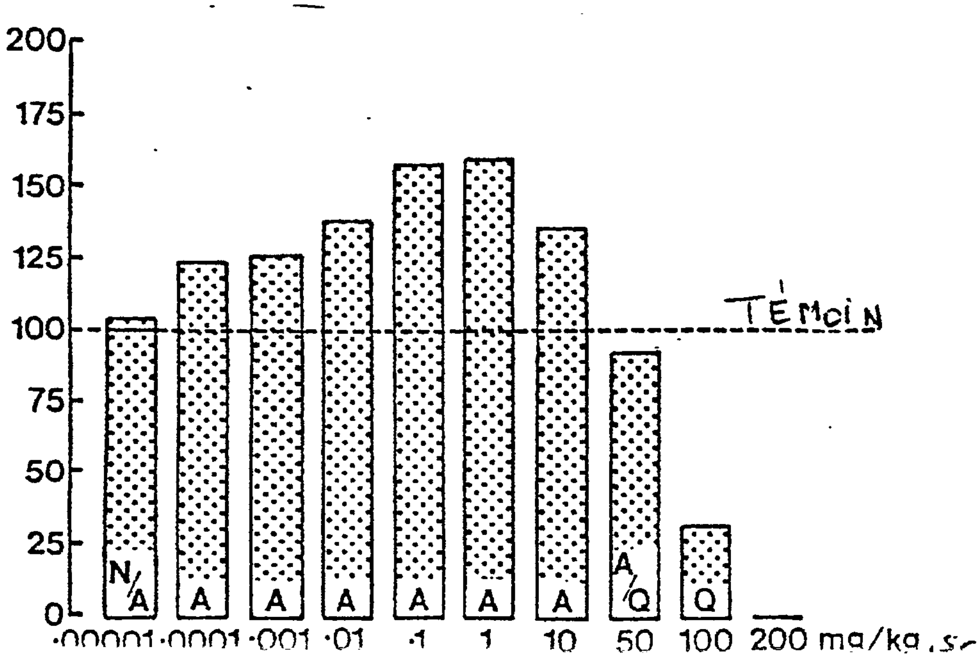


FIG. 5

COMPOSE

Exemple IX

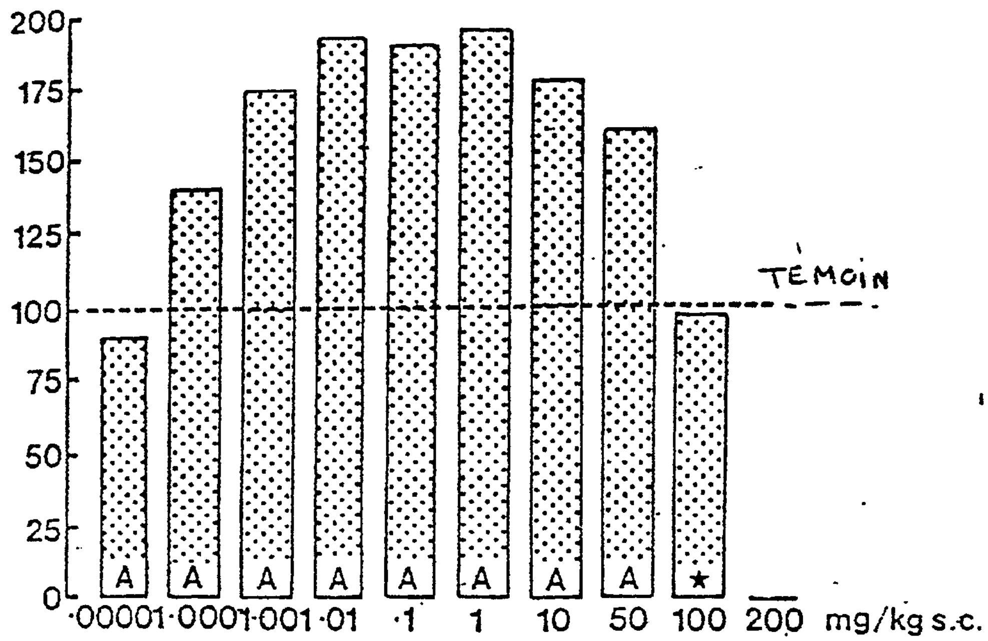


FIG. 6

COMPOSE

Exemple XVI

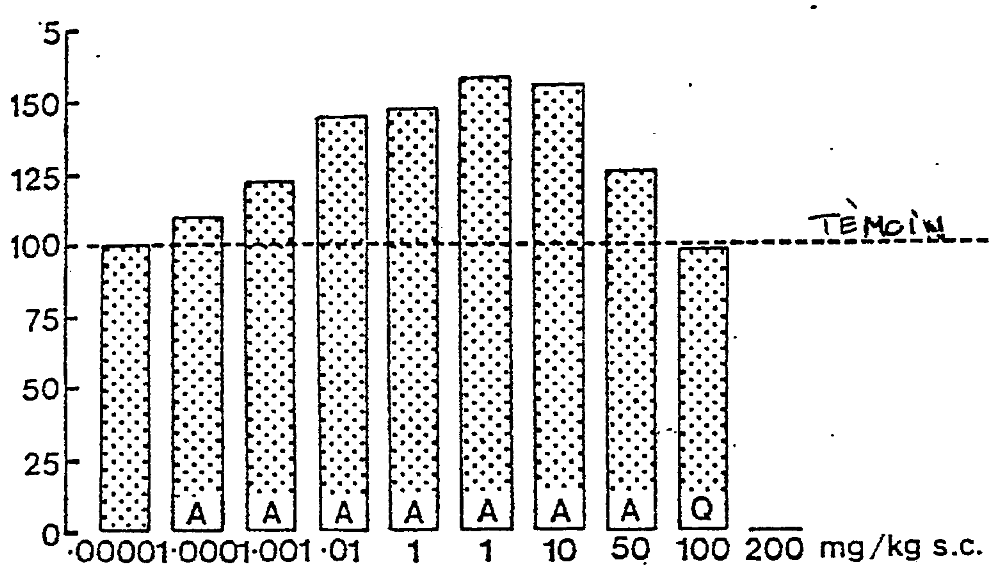


FIG. 7

COMPOSE

Exemple XV

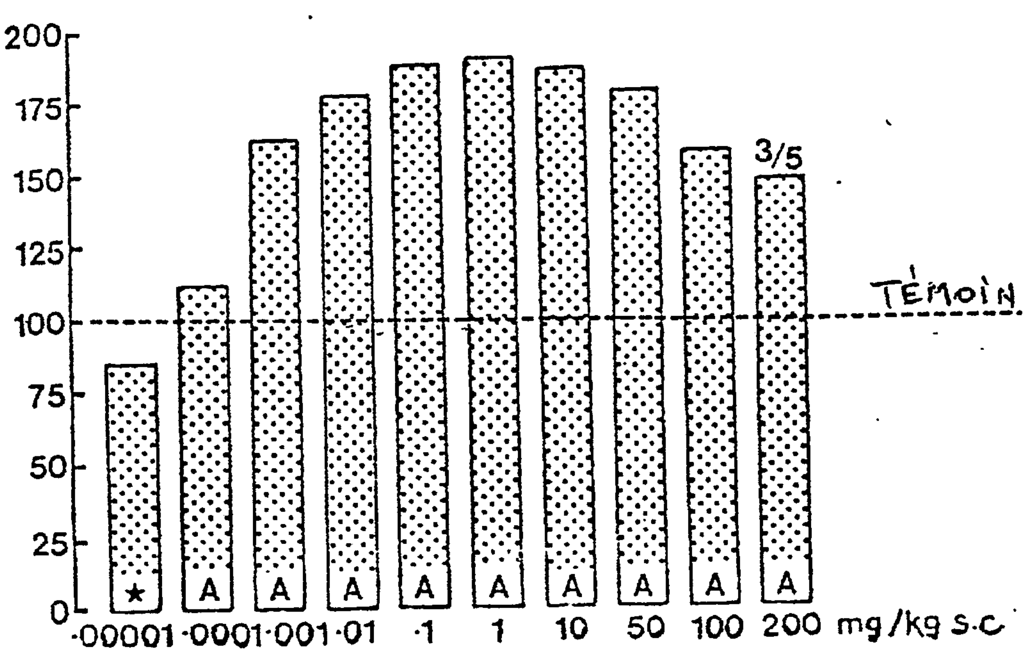


FIG. 8

COMPOSE

Exemple XIV

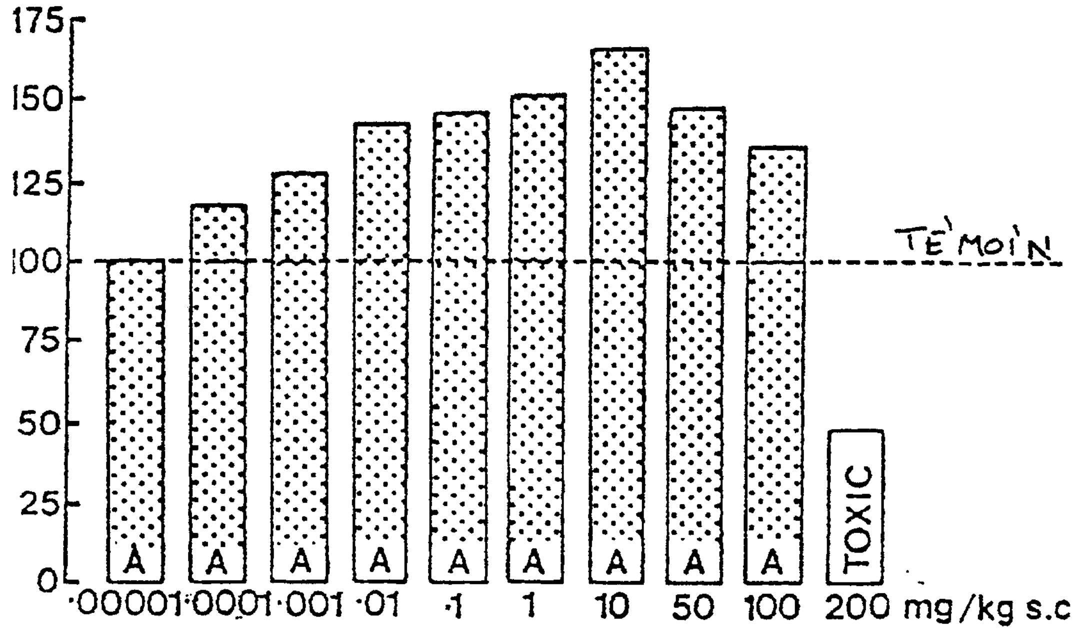


FIG. 9

COMPOSE

Exemple XVIII

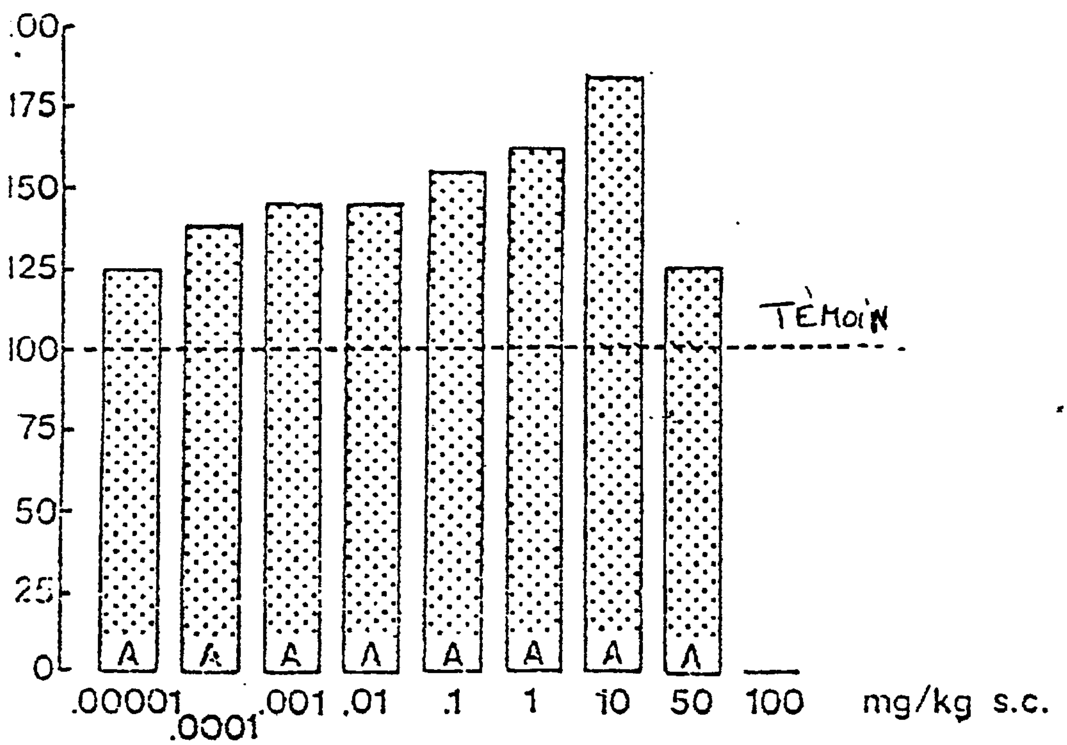


FIG. 10

COMPOSE

Exemple XXVII

ACTION ACTIVANTE (ADMINISTRATION INTRA ACCUMBENS)

A = activation

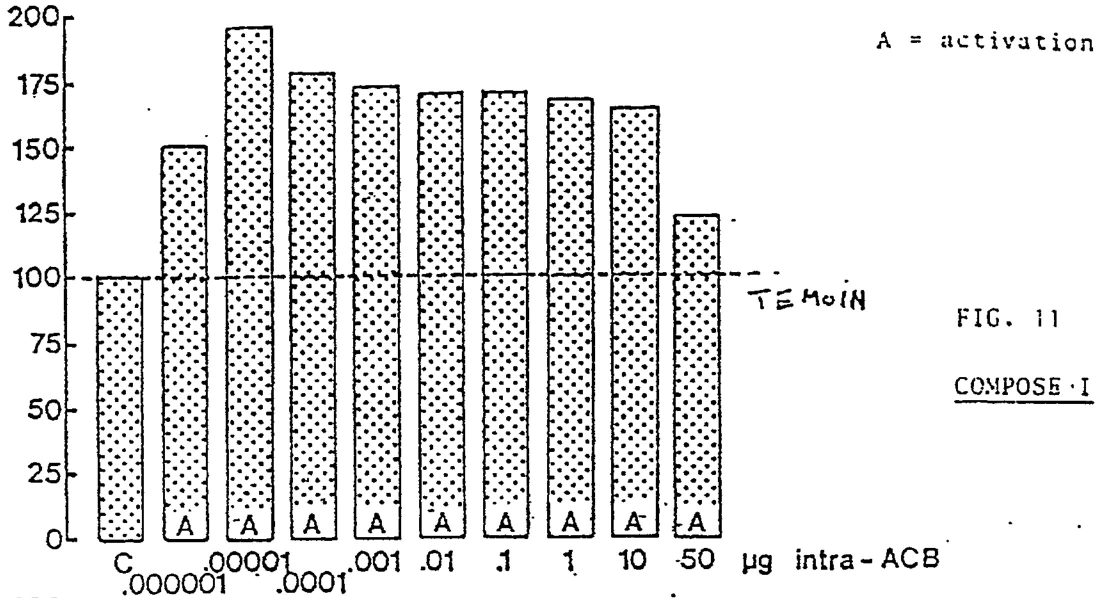


FIG. 11

COMPOSE I

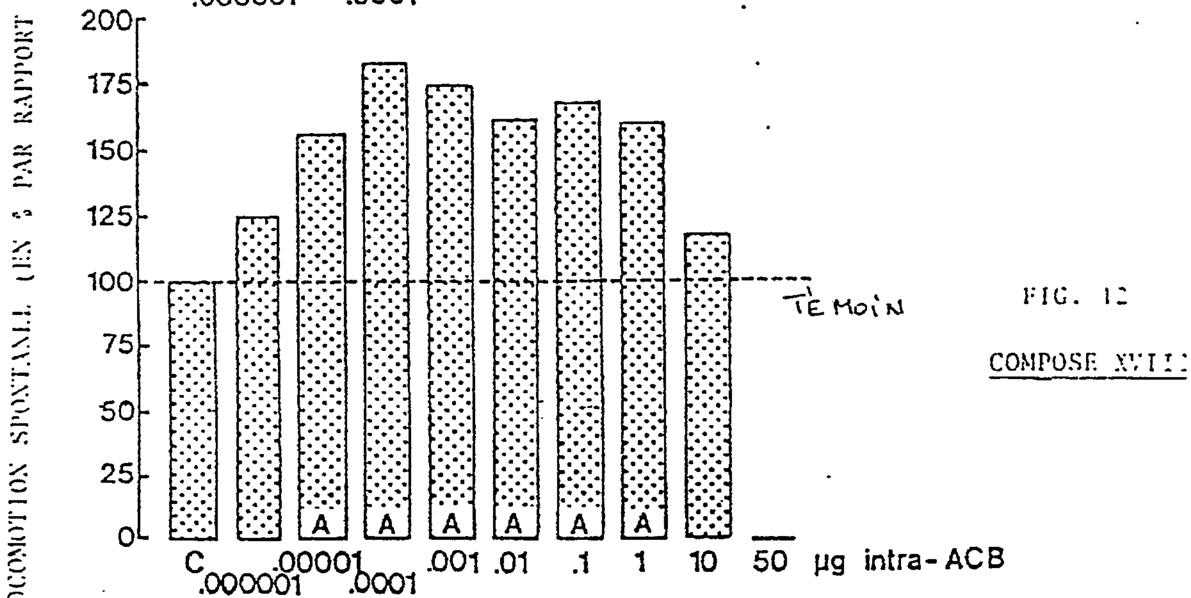


FIG. 12

COMPOSE XVIII

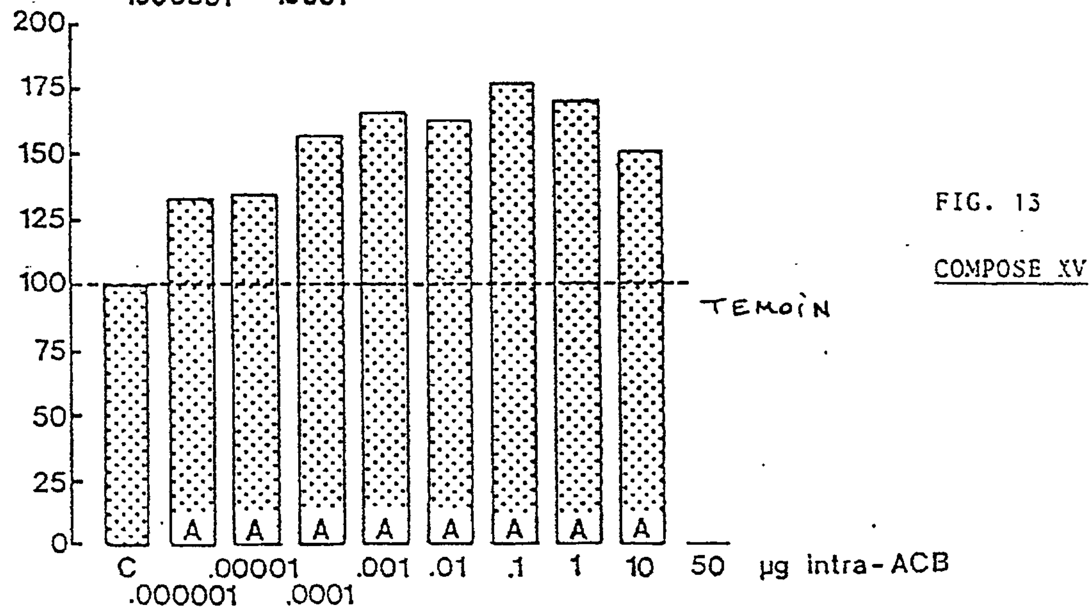


FIG. 13

COMPOSE XV

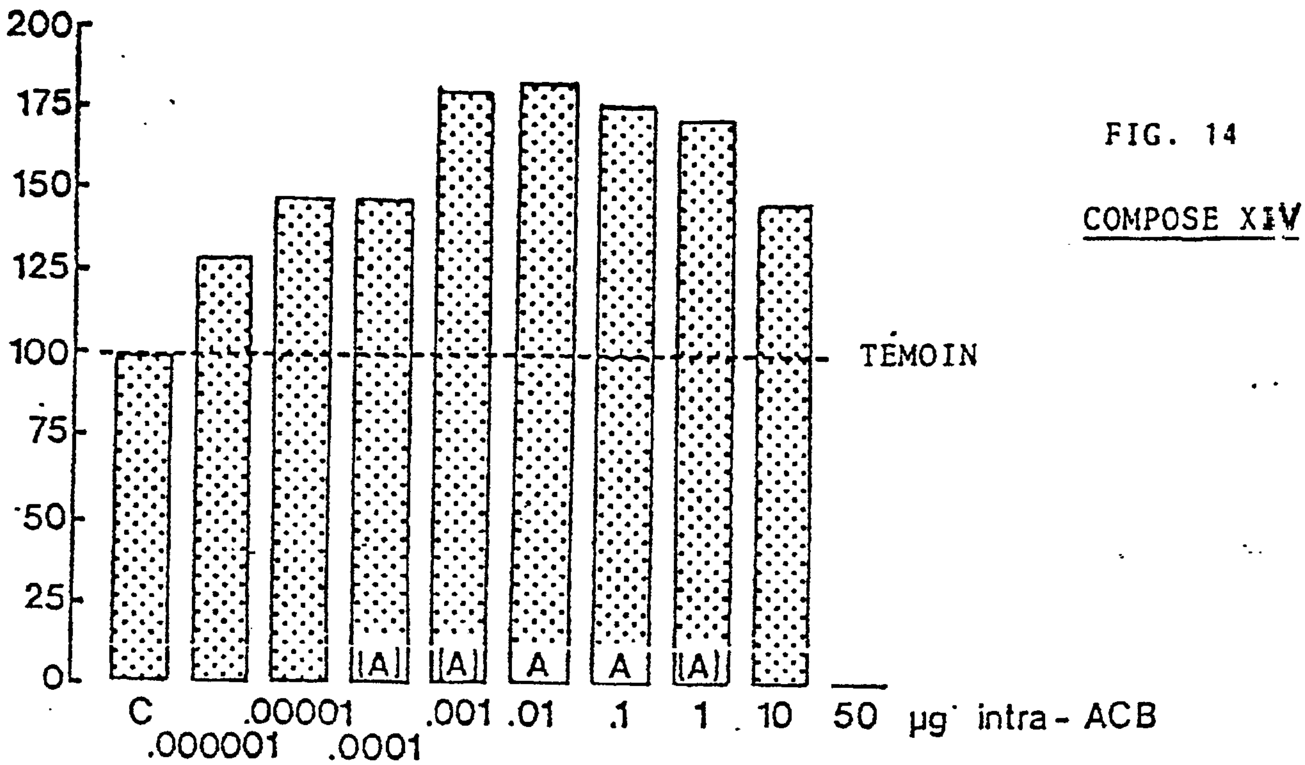


FIG. 14

COMPOSE XIV

FIG. 15

AUGMENTATION DE L'ACTIVITE
MOTRICE CHEZ LE MARMOUSET.

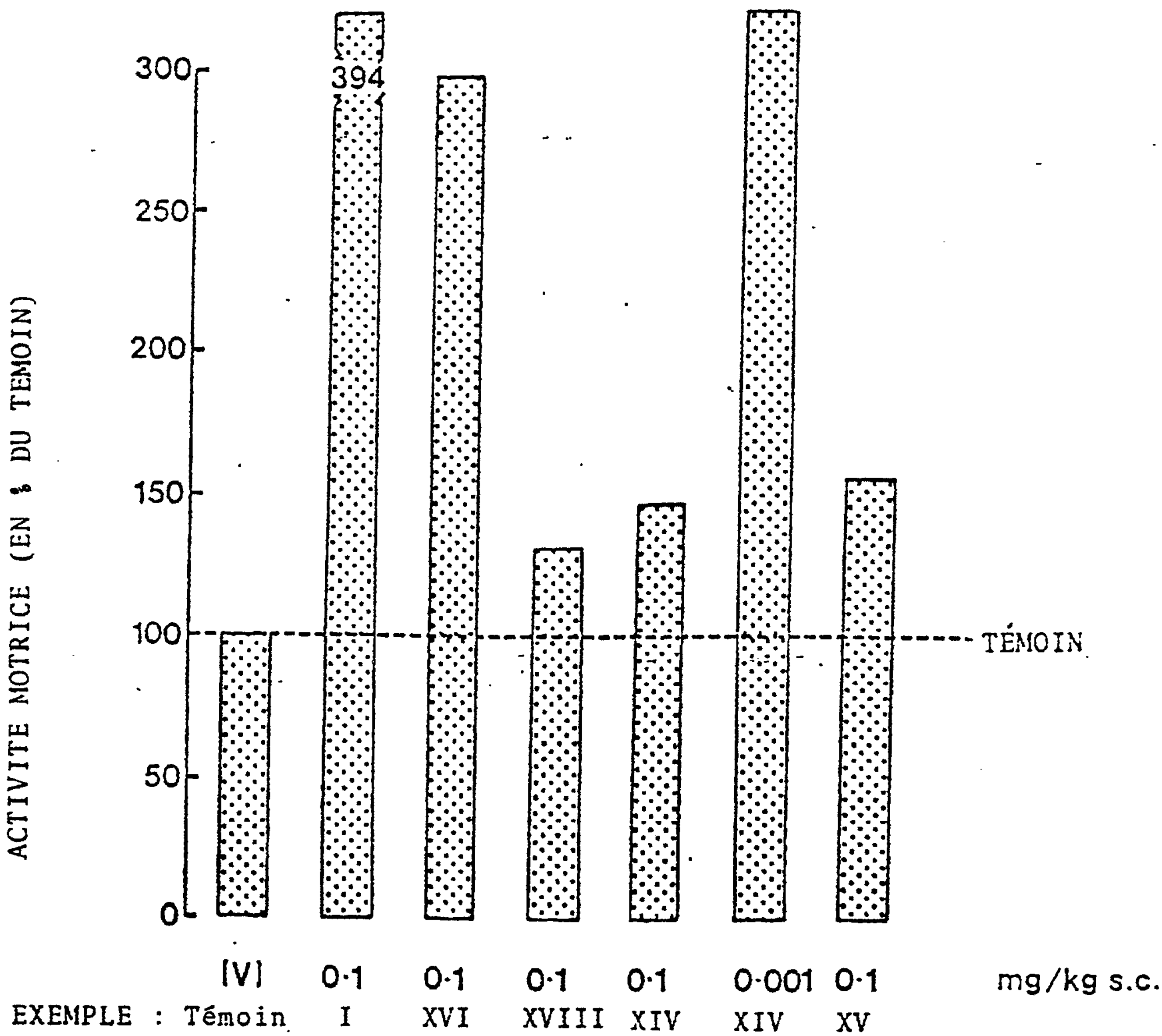

FIG. 16

EFFET ACTIVANT EN TRAITEMENT
CHRONIQUE (48 jours) CHEZ LE RAT